# EUROPEAN PATENT APPLICATION

(11) **EP 2 447 265 A1**
(43) Date of publication of application: **02.05.2012**
(21) Application number: 10792217.1
(22) Date of filing: 26.06.2010
(51) Int. Cl.: C07D 417/14, A61K 31/427, A61K 31/433, A61P 3/10, A61P 9/10, A61P 13/12, A61P 25/00, A61P 27/02, A61P 43/00, C07D 333/40, C07D 409/04

(54) **NOVEL THIOPHENE CARBOXAMIDE DERIVATIVE AND MEDICINAL USE OF SAME**

(30) Priority: 26.06.2009 JP 2009152741
(71) Applicant: Sanwa Kagaku Kenkyusho Co., Ltd, Nagoya-shi Aichi 461-8631 (JP)
(72) Inventor: YAMASHITA, Tokuyuki, Nagoya-shi Aichi 461-8631 (JP); CHIKAMATSU, Kaori, Aichi 441-0204 (JP); TAKAHASHI, Naoki, Nagoya-shi Aichi 461-8631 (JP); IWAI, Hisakazu, Nagoya-shi Aichi 461-8631 (JP); KOGAMI, Masakazu, Nagoya-shi Aichi 461-8631 (JP); SAKAIRI, Masao, Nagoya-shi Aichi 461-8631 (JP); WATANABE, Nobuhide, Nagoya-shi Aichi 461-8631 (JP); FUJIEDA, Hiroki, Nagoya-shi Aichi 461-8631 (JP); KATAOKA, Daisuke, Nagoya-shi Aichi 461-8631 (JP); MAKINO, Mitsuhiro, Nagoya-shi Aichi 461-8631 (JP); KATO, Noriyasu, Nagoya-shi Aichi 461-8631 (JP); MIYAZAWA, Toshiyuki, Nagoya-shi Aichi 461-8631 (JP); NAGAI, Kazushige, Nagoya-shi Aichi 461-8631 (JP); KASUGAI, Nobuyoshi, Nagoya-shi Aichi 461-8631 (JP); KATAOKA, Hiroyo, Nagoya-shi Aichi 461-8631 (JP); HIRAMATSU, Naoki, Nagoya-shi Aichi 461-8631 (JP); TSURUTA, Nobuaki, Nagoya-shi Aichi 461-8631 (JP); YAMADA, Yurie, Osaka-shi Osaka 532-0031 (JP); MIYOSHI, Mika, Nagoya-shi Aichi 461-8631 (JP); MURAKAMI, Sei, Nagoya-shi Aichi 461-8631 (JP); GOTO, Izumi, Nagoya-shi Aichi 461-8631 (JP); IZUCHI, Tohru, Nagoya-shi Aichi 461-8631 (JP); SUZUKI, Kimie, Nagoya-shi Aichi 461-8631 (JP); HARADA, Satoko, Nagoya-shi Aichi 461-8631 (JP); LANG, Martin, 82166 Graefelfing (DE); SEIFERT, Hans-Juergen Markus, 81377 Munich (DE); WOLF, Kaarina Kristina, 82288 Kottgeisering (DE)
(74) Representative: Grünecker, Kinkeldey, Stockmair & Schwanhäusser
(86) International application number: PCT/JP2010/060916
(87) International publication number: WO 2010/150899

(57) **Abstract**

The object of the present invention is to provide a compound having a glucokinase-activating effect.

A pharmaceutical composition comprising a compound represented by the general formula (I) or a pharmaceutically acceptable salt thereof as an active ingredient: 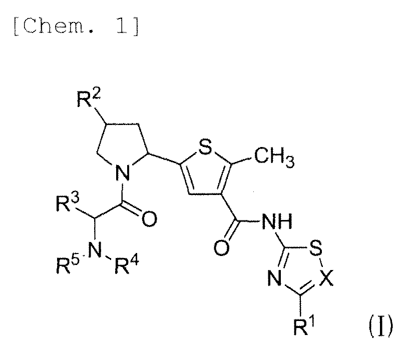 wherein
X means a nitrogen atom or CR⁶ wherein R⁶ means a hydrogen atom or a halogen atom;
R¹ means a hydrogen atom, a C1-C6 alkyl group, a C1-C6 alkoxy group or a C1-C6 alkylthio group;
R² means a hydrogen atom or a fluorine atom;
R³ means a hydrogen atom or a C1-C6 alkyl group; and
one of R⁴ and R⁵ means a hydrogen atom or a C1-C6 alkyl group, and the other means a C1-C6 alkylenecarboxylic acid, a C1-C6 alkylsulfonyl group, a C1-C6 alkylcarbonyl group, or CONH₂.

## Description

### Technical Field

The present invention relates to a novel thiophenecarboxamide derivative and the pharmaceutical use thereof. Said compound has various pharmaceutical uses as a glucokinase activator.

### Background Art

Glucokinase (ATP: D-glucose 6-phosphotransferae, EC2.7.1.1.) is one of four kinds of hexokinases that are present in mammals (hexokinase IV). Hexokinases are enzymes that catalyze the first step of glucose metabolism, and they convert glucose to glucose-6 phosphate. Glucokinase is expressed in mainly liver and pancreatic β cells, acts as a rate-controlling enzyme for glucose metabolism, and plays an important role in systemic glucose homeostasis.

Glucokinase has low affinity for glucose, and has a Km value (8-15 mM) that is close to a physiological blood glucose level. Furthermore, glucokinase is not inhibited by glucose-6 phosphate of a physiological concentration. Therefore, when a normal blood glucose level (5 mM) is raised to from 10 to 15 mM by diet, glucose metabolism through glucokinase is increased. In view of these facts, it was considered that glucokinase acts as a glucose sensor in liver and pancreatic β cells.

The role of glucokinase in animals was confirmed by studies using genetically modified animals. It was reported that a mouse that did not express glucokinase died of severe diabetes immediately after birth, whereas glucose tolerance is improved in a mouse in which glucokinase was overexpressed. It was confirmed by these studies that glucokinase actually has an important role in systemic glucose homeostasis.

Maturity onset diabetes of the young (MODY-2) is caused by spontaneous mutation of function loss of glucokinase gene, and decrease in glucokinase activity causes increase in blood glucose. Furthermore, descents having spontaneous mutation that increases the activity of glucokinase were also found, and in these descents, a state of fasting hypoglycemia is shown by increase in blood plasma insulin level.

As mentioned above, glucokinase acts as a glucose sensor and plays an important role in adjustment of blood glucose, and thus it is considered that blood glucose control utilizing a glucose sensor system will be a useful treatment for many patients with Type II diabetes. It is considered that a substance that activates glucokinase is useful as an agent for the prevention or treatment of Type II diabetes since it enhances the action of a glucose sensor, whereby an effect of promoting secretion of insulin in pancreatic P cells and an effect of promoting intaking of glucose and suppressing release of glucose in liver cells can be expected.

Many compounds having a glucokinase-activating effect have been reported until now (Non-patent Literature 1) . As compounds in which two five-membered heteroaromatic ring are amide-bonded, a compound having a pyrrole ring was reported in Patent Literature 1, and a compound having an indole ring was reported in Patent Literature 2, but these compounds are different from the thiophenecarboxamide derivative of the present invention in structure.

Meanwhile, as a thiophenecarboxamide derivative, 2, 5-dimethylthiophene-3-carboxylic acid thiazol-2-ylamide is sold as a reagent by Enamine, Ltd. (Ukraine); however, there is no report about the bioactivity and the like thereof, and the compound is different in structure from the compound of the present invention that has a pyrrolidine ring with a thiophene ring.

### Prior Art Documents

### Patent Literatures

Patent Literature 1: International Publication No.2008/149382
Patent Literature 2: International Publication No.2004/031179

### Non-patent Literatures

Non-patent Literature 1: Expert Open. Ther. Patents 18, 759-768, 2008

### Summary of the invention

### Problem to be solved by the invention

The present invention aims at providing a compound having a glucokinase-activating effect, and providing an agent for the prevention or treatment of diabetes or diabetic retinopathy, diabetic nephropathy, diabetic neuropathy, ischemic heart diseases, or chronic complications of diabetes such as arteriosclerosis based on the glucokinase-activating effect.

### Means of solving the problem

In view of the above-mentioned points, the present inventors considered that a compound having a novel basic structure is effective as a means for solving the above-mentioned problems, and did intensive studies in an effort to create a novel glucokinase activator. As a result, they found that the compound represented by the following general formula (I) and a salt thereof have an exceptional glucokinase-activating effect, further have excellent properties in physical properties as a medicament such as solubility, and become a safe and useful medicament that is excellent in divergence of various side effects (effects against hERG and CYP) and medicinal benefits, which led to the completion of the present invention.

Namely, the present invention provides compounds represented by the general formula (I): 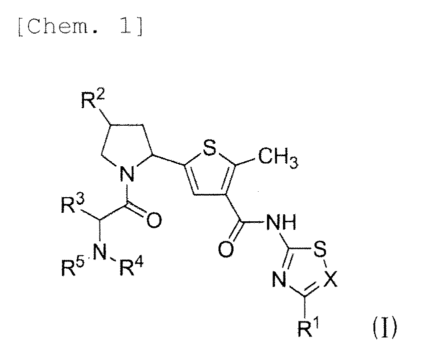 wherein
X means a nitrogen atom or CR⁶, wherein R⁶ means a hydrogen atom or a halogen atom;
R¹ means a hydrogen atom, a C1-C6 alkyl group, a C1-C6 alkoxy group, or a C1-C6 alkylthio group;
R² means a hydrogen atom or a fluorine atom;
R³ means a hydrogen atom or a C1-C6 alkyl group; and
one of R⁴ and R⁵ means a hydrogen atom or a C1-C6 alkyl group, and the other means a C1-C6 alkylenecarboxylic acid, a C1-C6 alkylsulfonyl group, a C1-C6 alkylcarbonyl group, or CONH₂, or pharmaceutically acceptable salts thereof, and these compounds are hereinafter referred to as "compounds of the present invention". Hereinafter various exemplary embodiments of the compounds of the present invention are listed.

An exemplary embodiment of the present invention is a compound of the above-mentioned general formula (I), wherein X is a nitrogen atom, C-H, C-F, or C-C1, R¹ is a hydrogen atom, a C1-C3 alkyl group, a C1-C3 alkoxy group, or a C1-C3 alkylthio group.

Another exemplary embodiment of the present invention is a compound of the above-mentioned general formula (I), wherein X is C-H, C-F, or C-C1, and R¹ is a hydrogen atom.

A still another exemplary embodiment of the present invention is a compound of the above-mentioned general formula (I), wherein X is a nitrogen atom, and R¹ is a hydrogen atom or a C1-C3 alkyl group.

A still another exemplary embodiment of the present invention is a compound of the above-mentioned general formula (I), wherein R³ is a hydrogen atom or a C1-C3 alkyl group, and one off R⁴ and R⁵ is a hydrogen atom or a C1-C6 alkyl group, and the other is a C1-C3 alkylenecarboxylic acid, a C1-C3 alkylsulfonyl group, a C1-C3 alkylcarbonyl group, or CONH₂.

A still another exemplary embodiment of the present invention is a compound of the above-mentioned general formula (I), wherein one of R⁴ and R⁵ is a hydrogen atom or a C1-C6 alkyl group, and the other is a C1-C3 alkylenecarboxylic acid or a C1-C3 alkylsulfonyl group.

A still another exemplary embodiment of the present invention is a compound of the above-mentioned general formula (I), wherein X is C-F, R¹ is a hydrogen atom, R² is a hydrogen atom or a fluorine atom, R³ is a hydrogen atom or a C1-C3 alkyl group, and one of R⁴ and R⁵ is a hydrogen atom or a C1-C6 alkyl group, and the other is a C1-C3 alkylenecarboxylic acid.

A still another exemplary embodiment of the present invention is a compound of the above-mentioned general formula (I), wherein X is a nitrogen atom or C-F, R¹ is a hydrogen atom or a C1-C3 alkyl group, R² is a hydrogen atom or a fluorine atom, R³ is a hydrogen atom or a C1-C3 alkyl group, and one of R⁴ and R⁵ is a hydrogen atom, and the other is a C1-C3 alkylsulfonyl group.

The present invention also provides a compound represented by the following general formula (II), a compound represented by the following general formula (III) and a compound represented by the following general formula (IV), which are intermediates of the compound of the present invention represented by the above-mentioned general formula (I): 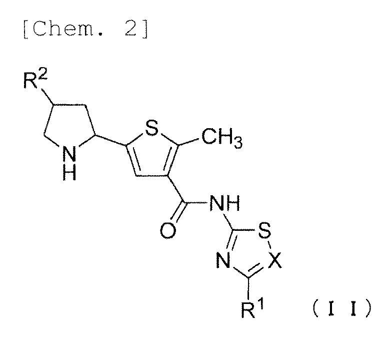 wherein
X means a nitrogen atom or CR⁶, wherein R⁶ s a hydrogen atom or a halogen atom;
R¹ means a hydrogen atom, a C1-C6 alkyl group, a C1-C6 alkoxy group, or a C1-C6 alkylthio group; and
R² means a hydrogen atom or a fluorine atom, 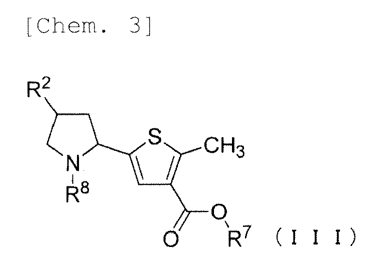
wherein
R² means a hydrogen atom or a fluorine atom;
R⁷ means a hydrogen atom, or a protective group for a carboxyl group; and
R⁸ means a hydrogen atom, or a protective group for an amino group, and 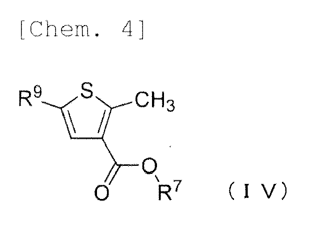
wherein
R⁷ means a hydrogen atom, or a protective group for a carboxyl group; and
R⁹ means a bromine atom or an iodine atom.

The present invention further provides a pharmaceutical composition comprising the above-mentioned compound of the present invention as an active ingredient.
Namely, the pharmaceutical composition of the present invention is used for the prevention or treatment of diabetes.

### Effect of the invention

The compound of the present invention has an excellent glucokinase-activating effect, and thus is useful as an agent for the prevention or treatment of diabetes or diabetic retinopathy, diabetic nephropathy, diabetic neuropathy, ischemic heart diseases or chronic complications of diabetes such as arteriosclerosis. Furthermore, the present invention provides a safe and useful medicament that is excellent in divergence of various side effects (effects against hERG and CYP) and medicinal benefits.

### Embodiments for carrying out the invention

Hereinafter the compound of the present invention is explained.

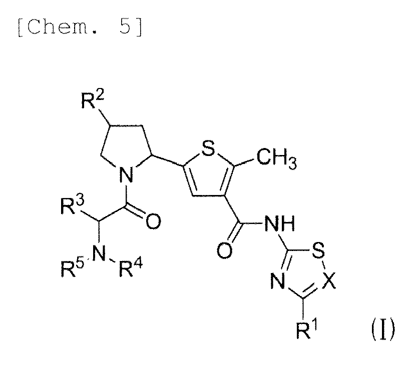

In the compound of the present invention represented by the above-mentioned general formula (I), X is a nitrogen atom or CR⁶, wherein R⁶ means a hydrogen atom or a halogen atom, and R¹ is a hydrogen atom, a C1-C6 alkyl group, a C1-C6 alkoxy group, or a C1-C6 alkylthio group. Among these, X is preferably a nitrogen atom, C-F, or C-Cl, and R¹ is preferably a hydrogen atom, a C1-C3 alkyl group, a C1-C3 alkoxy group, or a C1-C3 alkylthio group. Of these, when X is C-F or C-C1, R¹ is preferably a hydrogen atom, or when X is a nitrogen atom, R¹ is preferably a hydrogen atom or a C1-C3 alkyl group.

R² is a hydrogen atom or a fluorine atom. R³ is a hydrogen atom or a C1-C6 alkyl group, preferably a hydrogen atom or a C1-C3 alkyl group.

One of R⁴ and R⁵ is a hydrogen atom or a C1-C6 alkyl group, and the other is a C1-C6 alkylenecarboxylic acid, a C1-C6 alkylsulfonyl group, a C1-C6 alkylcarbonyl group, or CONH₂. Among these, one of R⁴ and R⁵ is preferably a hydrogen atom or a C1-C6 alkyl group, and the other is preferably a C1-C3 alkylenecarboxylic acid, a C1-C3 alkylsulfonyl group, a C1-C3 alkylcarbonyl group, or CONH₂, of which the other is optimally a C1-C3 alkylenecarboxylic acid or a C1-C3 alkylsulfonyl group.

A specifically preferable compound of the present invention is the compound of the general formula (I), wherein X is C-F, R¹ is a hydrogen atom, R² is a hydrogen atom or a fluorine atom, R³ is a hydrogen atom or a C1-C3 alkyl group, and one of R⁴ and R⁵ is a hydrogen atom or a C1-C6 alkyl group, and the other is a C1-C3 alkylenecarboxylic acid.

Another specifically preferable compound of the present invention is the compound of the general formula (I), wherein X is a nitrogen atom or C-F, R¹ is a hydrogen atom or a C1-C3 alkyl group, R² is a hydrogen atom or a fluorine atom, R³ is a hydrogen atom or a C1-C3 alkyl group, and one of R⁴ and R⁵ is a hydrogen atom, and the other is a C1-C3 alkylsulfonyl group.

Meanwhile, in the compound of the present invention, the "halogen atom" means a fluorine atom, a chlorine atom, a bromine atom or an iodine atom.

The "C1-C6 alkyl group" means a straight chain or branched chain alkyl group composed of 1 to 6 carbon atom(s), and examples thereof may include a methyl group, an ethyl group, a *n*-propyl group, an *i*-propyl group, a *n*-butyl group, an *i*-butyl group, a s-butyl group, a *t*-butyl group, a *n*-pentyl group, an *i*-pentyl group, a *neo*-pentyl group, a *t*-pentyl group, a *n*-hexyi group, an *i*-hexyl group, a 1-methylbutyl group, a 2-methylbutyl group, a 1,2-dimethylpropyl group and the like.

The "C1-C6 alkoxy group" means a -O-(C1-C6 alkyl) group, and examples thereof may include a methoxy group, an ethoxy group, a *n*-propoxy group, an *i*-propoxy group, a *n*-butoxy group, an *i*-butoxy group, a s-butoxy group, a *t*-butoxy group, a *n*-pentoxy group, an *i*-pentoxy group, a *neo*-pentoxy group, a *t*-pentoxy group, a 1-methylbutoxy group, a 2-methylbutoxy group, a 1,2-dimethylpropoxy group, a *n*-hexyloxy group and the like.

The "C1-C6 alkylthio group" means a -S-(C1-C6 alkyl) group. Furthermore, the "C1-C6 alkylenecarboxylic acid" means -(C1-C6 alkylene)-COOH, and the "C1-C6 alkylene" means a linear alkylene composed of 1 to 6 carbon atom(s), and examples thereof may include methylene, ethylene, *n*-propylene, *n*-butylene and the like.

The "C1-C6 alkylsulfonyl group" means a -SO₂-(C1-C6 alkyl) group. Furthermore, the "C1-C6 alkylcarbonyl group" means a -CO-(C1-C6 alkyl) group.

Next, the compounds that are intermediates for the compound of the present invention are explained. 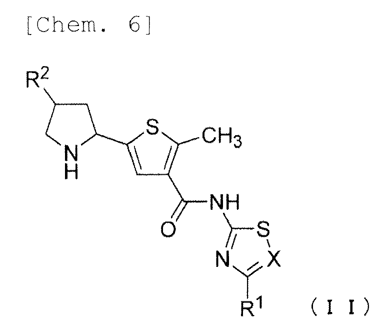 In the intermediate represented by the above-mentioned general formula (II), X is a nitrogen atom or CR⁶, wherein R⁶ is a hydrogen atom or a halogen atom. R¹ ls a hydrogen atom, a C1-C6 alkyl group, a C1-C6 alkoxy group, or a C1-C6 alkylthio group. Among theses is preferably a nitrogen atom, C-F, or C-C1, and R¹ is preferably a hydrogen atom, a C1-C3 alkyl group, a C1-C3 alkoxy group, or a C1-C3 alkylthio group. Among these, when X is C-F or C-C1, R¹ is preferably a hydrogen atom, or when X is a nitrogen atom, R¹ is preferably a hydrogen atom or a C1-C3 alkyl group. R² is a hydrogen atom or a fluorine atom.

The compound that is a still another intermediate for the compound of the present invention is explained. 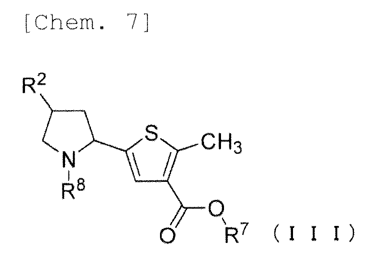 In the intermediates represented by the above-mentioned general formula (II), R² is a hydrogen atom or a fluorine atom, R⁷ is a hydrogen atom, or a protective group for a carboxyl group, and R⁸ is a hydrogen atom, or a protective group for an amino group.

The compound that is a still another intermediate for the compound of the present invention is explained. 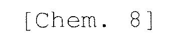 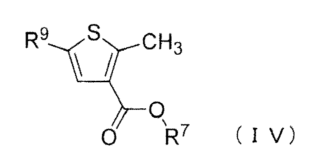 In the intermediate represented by the above-mentioned general formula (III), R⁷ is a hydrogen atom, or a protective group for a carboxyl group, and R⁹ is a bromine atom or an iodine atom.

The "protective group for a carboxyl group" generally means a group that is known as a protective group for a carboxyl group in organic synthesis, and examples may include (1) linear or branched chain lower alkyl groups having 1 to 4 carbon atom(s) such as a methyl group, an ethyl group, an *i*-propyl group and a *t*-butyl group, (2) halogeno-lower alkyl groups such as a 2-iodoethyl group and a 2,2,2-trichloroethyl group, (3) lower alkoxymethyl groups such as a methoxymethyl group, an ethoxymethyl group and an *i*-butoxymethyl group, (4) lower aliphatic acyloxymethyl groups such as a butyryloxymethyl group and a pivaloyloxymethyl group, (5) 1-lower alkoxycarbonyloxyethyl groups such as a 1-methoxycarbonyloxyethyl group and a 1-ethoxycarbonyloxyethyl group, (6) aralkyl groups such as benzyl, a *p*-methoxybenzyl group, an *o*-nitrobenzyl group and a *p*-nitrobenzyl group, (7) a benzhydryl group, (8) a phthalidyl group, and the like.

The "protective group for an amino group" generally means a group that is known as a protective group for an amino group in organic synthesis, and examples may include (1) substituted or unsubstituted lower alkanoyl groups such as a formyl group, an acetyl group, a chloroacetyl group, a dichloroacetyl group, a propionyl group, a phenylacetyl group, a phenoxyacetyl group and a thienylacetyl group, (2) substituted or unsubstituted lower alkoxycarbonyl groups such as a benzyloxycarbonyl group, a *t*-butoxyca group, a *p*-nitrobenzyloxycarbonyl group and a 9-fluorenylmethyloxycarbonyl group, (3) substituted lower alkyl groups such as a methyl group, a *t*-butyl group, a 2,2,2-trichloroethyl group, a trityl group, a *p*-methoxybenzyl group, a *p*-nitrobenzyl group, a diphenylmethyl group and a pivaloyloxymethyl group, (4) substituted silyl groups such as a trimethylsilyl group and a *t*-butyldimethylsilyl group, (5) substituted or unsubstituted benzylidene groups such as a benzylidene group, a salicylidene group, a *p*-nitrobenzylidene group, a *m*-chlorobenzylidene group, a 3,5-di(*t*-butyl)-4-hydroxybenzylidene group and a 3,5-di(*t*-butyl)benzylidene group.

The "pharmaceutically acceptable salt" means a salt that retains the bioavailability and properties of the compound represented by the general formula (I) and causes no inconvenience in biological or other aspects. Such pharmaceutically acceptable salt falls within the scope of the present invention. Examples of the pharmaceutically acceptable salt may include inorganic acid addition salts (for example, salts with hydrochloric acid, hydrobromic acid, sulfuric acid, nitric acid, phosphoric acid and the like), organic acid addition salts (for example, salts with methane sulfonic acid, p-toluenesulfonic acid, acetic acid, oxalic acid, citric acid, fumaric acid, maleic acid, tartaric acid, succinic acid and malic acid), salts with amino acids (for example, salts with lysine, arginine and the like), alkali metal addition salts (for example, salts with sodium, potassium and the like), alkaline earth metal addition salts (for example, salts with calcium, magnesium and the like), organic amine addition salts (for example, salts with diethylamine, diethanolamine, piperazine and the like) and the like. The reactions for forming these addition salts can be conducted according to general methods.

The compound of the present invention also encompasses a prodrug that means a compound that is converted to the above-mentioned general formula (I) by a reaction by an enzyme, gastric acid or the like under a physiological condition in vivo, and various prodrugs are already known in the art. For example, examples of the prodrug wherein the compound represented by the general formula (I) has a carboxylic acid group may include compounds in which said carboxylic acid group has been esterified or amidated (for example, ethyl esterified, carboxymethyl esterified, pivaloyloxymethylated, or methylamidated compounds) and the like. Examples of the prodrugs when the compound represented by the general formula (I) has an amino group may include compounds in which said amino group has been acylated, alkylated or phospharylated (for example,
eicasanoylated, alanylated, pentylaminocarbanylated, tetrahydrofuranylated, pyrrolidylmethylated, acetoxymethylated or *t*-butylated compounds) and the like.

Furthermore, when one or more asymmetric carbon(s) is/are present in the compound of the present invention, the present invention encompasses both isomers based on the asymmetric carbon(s) and compounds of any combination of the isomers, and when geometric isomerism or tautomerism is present, the present invention encompasses both of those geometric isomers and tautomers. In addition, the compound of the present invention also encompasses solvates with solvents that are accepted as a medicament such as water and ethanol.

The compound represented by the general formula (I), which is the compound of the present invention, can be produced by combining the methods shown in the following Reaction step formulas I to VI, the methods described in Examples, or known methods.

### [Reaction step formula 1]

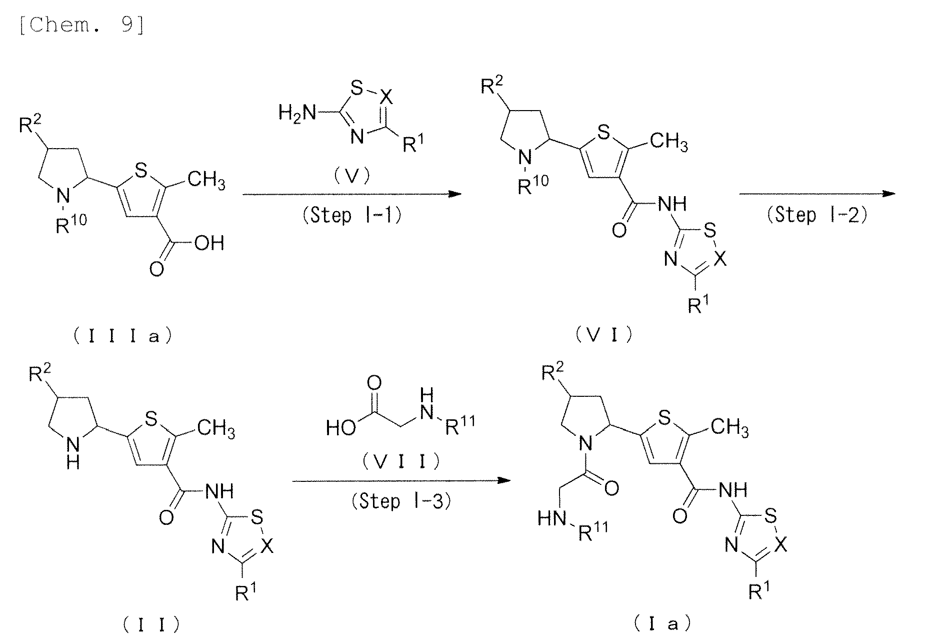

In the formula, R¹⁰ is a protective group for an amino group, R¹¹ is a C1-C6 alkylsulfonyl group, a C1-C6 alkylcarbonyl group, or CONH₂, and other symbols are defined as above.

### [Step I-1]

The compound represented by the general formula (VI) can be obtained by converting the compound represented by the general formula (IIIa) to an acid chloride using a chlorinating agent (for example, thionyl chloride, oxalyl chloride) in a suitable solvent (for example, toluene, methylene chloride and the like), and reacting the acid chloride with the compound represented by the general formula (V) using a base (for example, triethylamine, *N,N*-diethylaniline and the like) in a suitable solvent (for example, toluene, methylene chloride, tetrahydrofuran, *N,N*-dimethylformamide and the like). The reaction temperature is from -20°C to the boiling point of the solvent, and the reaction time is from 30 minutes to 48 hours. Furthermore, the compound represented by the general formula (VI) can also be obtained by reacting the compound represented by the general formula (IIIa) and the compound represented by the general formula (V) using a condensing agent (for example, 1-ethyl-3-(3-dimethylaminopropyl)carbodiimide, dicyclohexylcarbodiimide and the like) in a suitable solvent (for example, *N,N*-dimethylformamide, methylene chloride, tetrahydrofuran and the like) in the presence or absence of an additive (for example, diisopropylethylamine, 4-dimethylaminopyridine, 1-hydroxy-1H-benzotriazole and the like). The reaction temperature is from -20°C to the boiling point of the solvent, and the reaction time is from 30 minutes to 48 hours. Meanwhile, the compound represented by the general formula (V) is available as a commercial product, or can be produced by using a known method.

### [Step 1-2]

The compound represented by the general formula (II) can be obtained by removing the protective group R¹⁰ of the compound represented by the general formula (VI) with referring to the methods described in "Protecting Groups in Organic Synthesis, 3rd Edition, Wiley (1999)".

### [Step I-3],

The compound represented by the general formula (Ia) can be obtained by reacting the compound represented by the general formula (II) and the compound represented by the general formula (VII) by using a condensing agent (for example, 1-ethyl-3-(3-dimethylaminopropyl)carbodiimide, dicyclohexylcarbodiimide and the like) in a suitable solvent (for example, *N,N*-dimethylformamide, methylene chloride, tetrahydrofuran and the like) in the presence or absence of an additive (for example, diisopropylethylamine, 4-dimethylaminopyridine, 1-hydroxy-1H-benzotriazole and the like). The reaction temperature is from -20°C to the boiling point of the solvent, and the reaction time is from 30 minutes to 48 hours. Meanwhile, the compound represented by the general formula (VII) is available as a commercial product, or can be produced by using a known method.

### [Reaction step formula II]

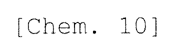

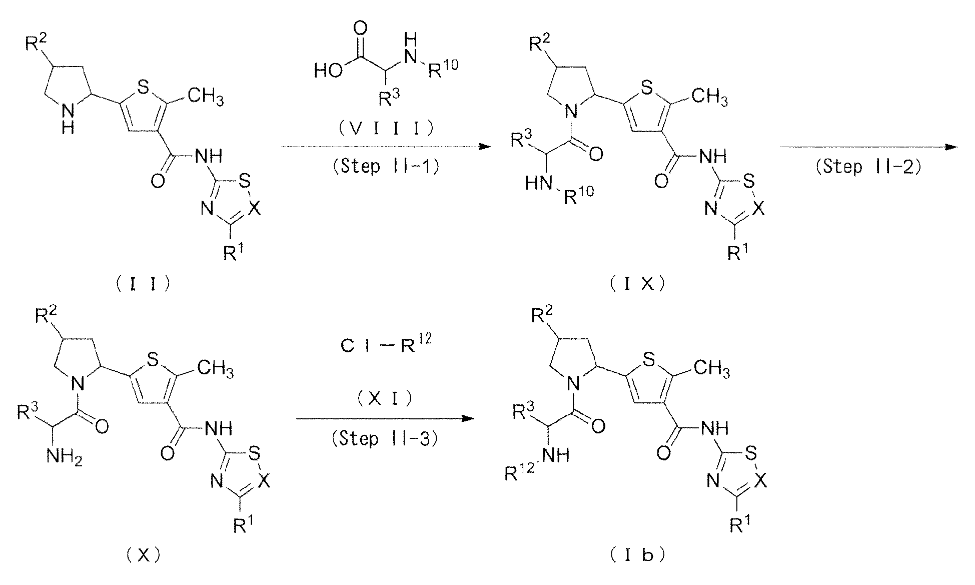

In the formula, R¹² is a C1-C6 alkylsulfonyl group or a C1-C6 alkylcarbonyl group, and other symbols are as defined in the above-mentioned general formulas and the above-mentioned Reaction step formulas.

### [Step II-I]

The compound represented by the general formula (IX) can be obtained by reacting the compound represented by the general formula (II) and the compound represented by the general formula (VIII) using a condensing agent (for example, 1-ethyl-3-(3-dimethylaminopropyl)carbodiimide, dicyclohexylcarbodiimide and the like) in a suitable solvent (for example, *N,N*-dimethylformamide, methylene chloride, tetrahydrofuran and the like) in the presence or absence of an additive (for example, diisopropylethylamine, 4-dimethylaminopyridine, 1-hydroxy-1H-benzotriazole and the like). The reaction temperature is from -20°C to the boiling point of the solvent, and the reaction time is from 30 minutes to 48 hours. Meanwhile, the compound represented by the general formula (VIII) is available as a commercial product, or can be produced by using a known method.

### [Step II-2]

The compound represented by the general formula (X) can be obtained by removing the protective group R¹⁰ of the compound represented by the general formula (IX) with referring to the method described in the above-mentioned "Protecting Groups in Organic Synthesis".

### [Step II-3]

The compound represented by the general formula (Ib) can be obtained by reacting the compound represented by the general formula (X) and the compound represented by the general formula (XI) using a base (for example, pyridine, triethylamine, N,N-diethylaniline and the like) in a suitable solvent (for example, methylene chloride, toluene, tetrahydrofuran, N, N-dimethylformamide and the like). The reaction temperature is from -20°C to the boiling point of the solvent, and the reaction time is from 30 minutes to 48 hours. Meanwhile, the compound represented by the general formula (XI) is available as a commercial product, or can be produced by using a known method.

### [Reaction step formula III]

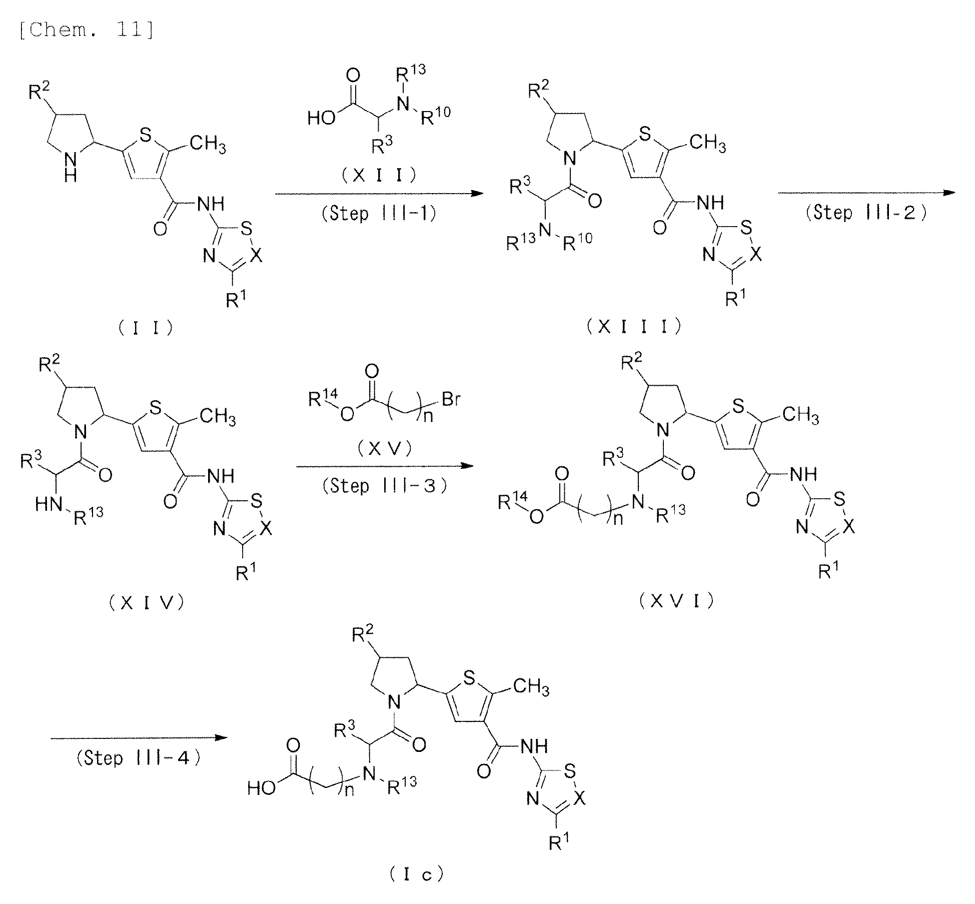

In the formula, R¹³ is a hydrogen atom or a C1-C6 alkyl group, R¹⁴ is a protective group for a carboxyl group, n is an integer of from 1 to 6, and other symbols are as defined in the above-mentioned general formulas and the above-mentioned Reaction step formulas.

### [Step III-I]

The compound represented by the general formula (XIII) can be obtained by reacting the compound represented by the general formula (II) and the compound represented by the general formula (XII) by using a condensing agent (for example, 1-ethyl-3-(3-dimethylaminopropyl)earbodiimide, dicyclohexylcarbodiimide and the like) in a suitable solvent (for example, *N,N*-dimethformamide, methylene chloride, tetrahydrofuran and the like) in the presence or absence of an additive (for example, diisopropylethylamine, 4-dimethylaminopyridine, 1-hydroxy-1H-benzotriazole and the like). The reaction temperature is from -20°C to the boiling point of the solvent, and the reaction time is from 30 minutes to 48 hours. Meanwhile, the compound represented by the general formula (XII) is available as a commercial product, or can be produced by using a known method.

### [Step III-2]

The compound represented by the general formula (XIV) can be obtained by removing the protective group R¹⁰ of the compound represented by the general formula (XIII) with referring to the method described in the above-mentioned "Protecting Groups in Organic Syntheses".

### [Step III-3]

The compound represented by the general formula (XVI) can be obtained by reacting the compound represented by the general formula (XIV) and the compound represented by the general formula (XV) by using a base (for example, triethylamine, diisopropylethylamine, pyridine and the like) in a suitable solvent (for example, *N,N*-dimethylformamide, methylene chloride, tetrahydrofuran and the like).
The reaction temperature is from -20°C to the boiling point of the solvent, and the reaction time is from 30 minutes to 48 hours. Meanwhile, the compound represented by the general formula (XV) is available as a commercial product, or can be produced by using a known method.

### [Step III-4]

The compound represented by the general formula (Ic) can be obtained by removing the protective group R¹⁴ of the compound represented by the general formula (XVI) with referring to the method described in the above-mentioned "Protecting Groups in Organic Synthesis".

### [Reaction step formula IV]

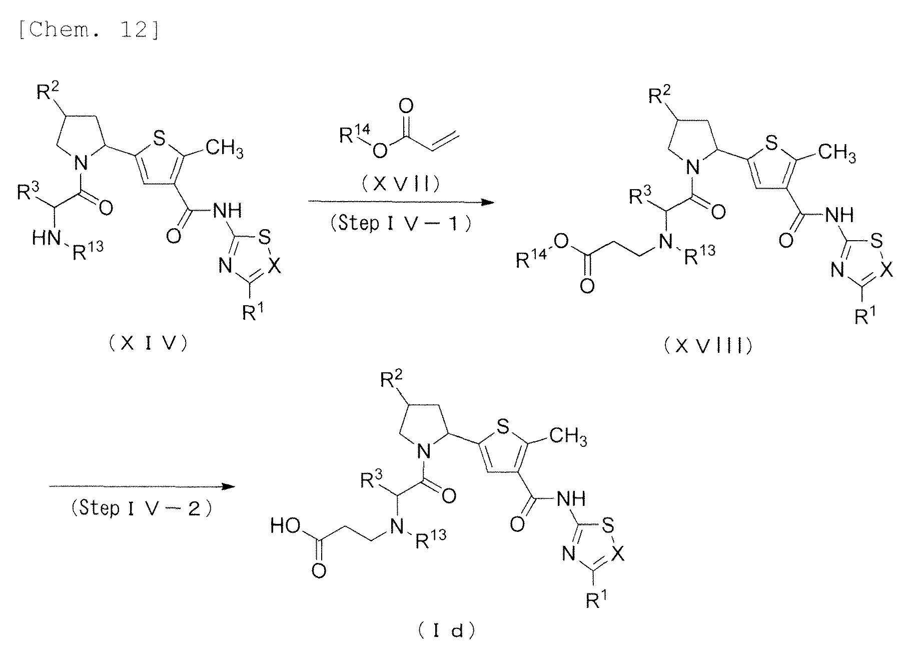

In the formula, all of the symbols are as defined in the above-mentioned general formulas and the above-mentioned Reaction step formulas.

### [Step IV-1]

The compound represented by the general formula (XVIII) can be obtained by reacting the compound represented by the general formula (XIV) and the compound represented by the general formula (XVII) by using a base (for example, triethylamine, Triton B, sodium hydroxide and the like) in a suitable solvent (for example, ethanol, 1,4-dioxane, *N,N*-dimethylformamide and the like). The reaction temperature is from -20°C to the boiling point of the solvent, and the reaction time is from 30 minutes to 48 hours. Meanwhile, the compound represented by the general formula (XVII) is available as a commercial product, or can be produced by using a known method.

### [Step IV-2]

The compound represented by the general formula (Id) can be obtained by removing the protective group R¹⁴ of the compound represented by the general formula (XVIII) with referring to the method described in the above-mentioned "Protecting Groups in Organic Synthesis".

### [Reaction step formula V]

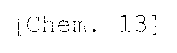

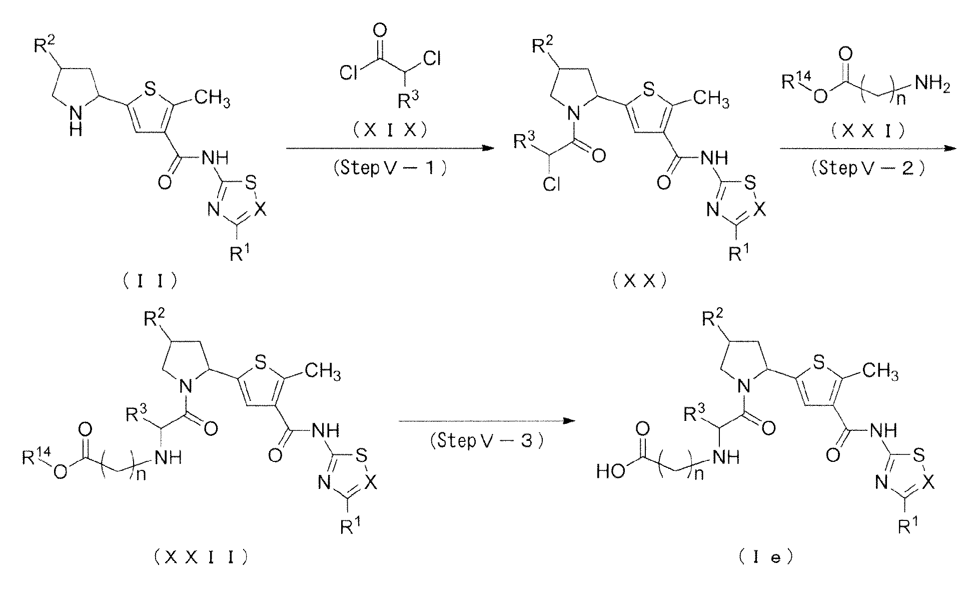

In the formula, all of the symbols are as defined in the above-mentioned general formulas and the above-mentioned Reaction step formulas.

### [Step V-1]

The compound represented by the general formula (XX) can be obtained by reacting the compound represented by the general formula (II) and the compound represented by the general formula (XIX) by using a base (for example, triethylamine, pyridine, di-*t*-butylpyridine and the like) in a suitable solvent (for example, toluene, methylene chloride, tetrahydrofuran, *N,N*-dimethylformamide and the like). The reaction temperature is from -20°C to the boiling point of the solvent, and the reaction time is from 30 minutes to 48 hours. Meanwhile, the compound represented by the general formula (XIX) is available as a commercial product, or can be produced by using a known method.

### [Step V-2]

The compound represented by the general formula (XXII) can be obtained by reacting the compound represented by the general formula (XX) and the compound represented by the general formula (XXI) by using a base (for example, triethylamine, diisopropylethylamine, pyridine and the like) in a suitable solvent (for example, *N,N*-dimethylformamide, methylene chloride, tetrahydrofuran and the like).
The reaction temperature is from -20°C to the boiling point of the solvent, and the reaction time is from 30 minutes to 48 hours. Meanwhile, the compound represented by the general formula (XXI) is available as a commercial product, or can be produced by using a known method.

### [Step V-3]

The compound represented by the general formula (Ie) can be obtained by removing the protective group R¹⁴ of the compound represented by the general formula (XXII) with referring to the method described in the above-mentioned "Protecting Groups in Organic Synthesis".

### [Reaction step formula VI]

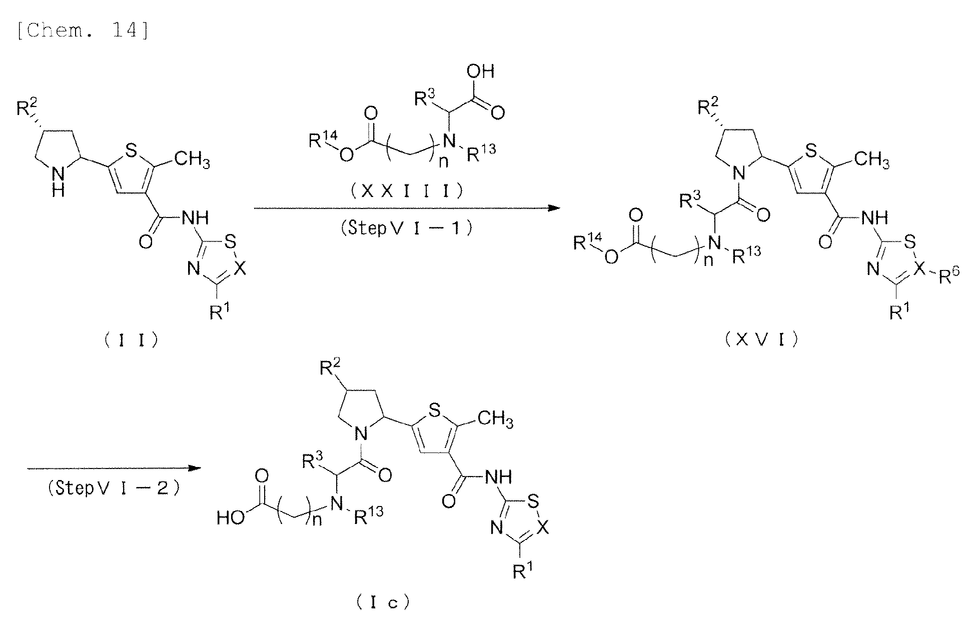

In the formula, all of the symbols are as defined in the above-mentioned general formulas and the above-mentioned Reaction step formulas.

### [Step VI-1]

The compound represented by the general formula (XVI) can be obtained by reacting the compound represented by the general formula (II) and the compound represented by the general formula (XXIII) using a condensing agent (for example, 1-ethyl-3-(3-dimethylaminopropyl)carbodiimide, dicyclohexylcarbodiimide and the like) in a suitable solvent (for example, *N,N*-dimethylformamide, methylene chloride, tetrahydrofuran and the like) in the presence or absence of an additive (for example, diisopropylethylamine, 4-dimethylaminopyridine, 1-hydraxy-1H-benzotriazole and the like). The reaction temperature is from -20°C to the boiling point of the solvent, and the reaction time is from 30 minutes to 48 hours. Meanwhile, the compound represented by the general formula (XXIII) can be produced by using a known method, or the methods described in Reference Examples 66 to 81.

### [Step VI-2]

The compound represented by the general formula (Ic) can be obtained by removing the protective group R¹⁴ of the compound represented by the general formula (XVI) with referring to the method described in the above-mentioned "Protecting Groups in Organic Syntheses".

The compounds represented by the general formulas (III) and (IV), which are intermediates for the compound of the present invention represented by the above-mentioned general formula (I), can be produced by combining the methods shown in the following Reaction step formulas VII to IX, the methods described in Examples 1 to 9, or known methods.

### [Reaction step formula VII]

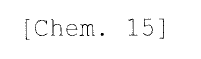

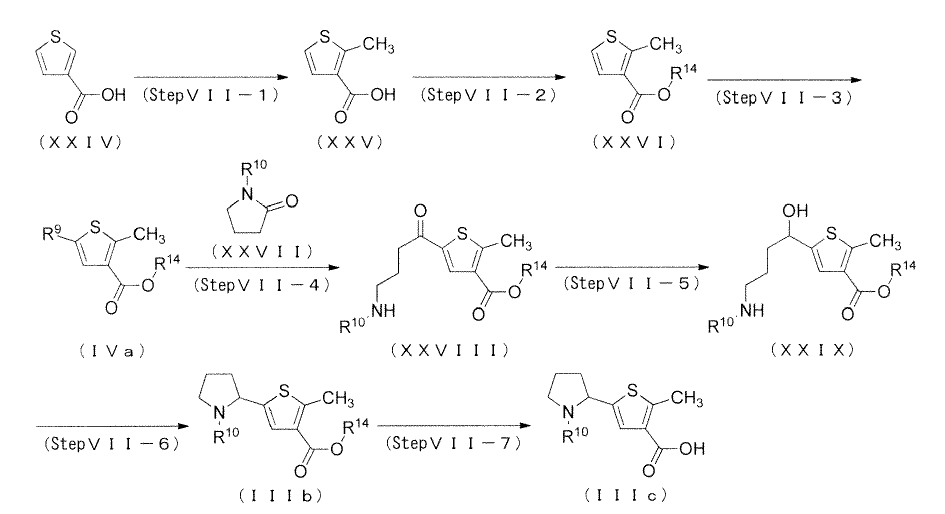

In the formula, all of the symbols are as defined in the above-mentioned general formulas and the above-mentioned Reaction step formulas.

### [step VII-1]

The compound represented by the general formula (XXV) can be obtained by reacting the compound represented by the general formula (XXIV) and a halogenated methyl by using a base (for example, *n*-butyllithium and the like) in a suitable solvent (for example, tetrahydrofuran, diethyl ether and the like) in the presence of an amine reagent (for example, diisopropylamine, hexamethylphosphoramide, *N,N,N,N*-tetramethylethylenediamine and the like). The reaction temperature is from -78°C to room temperature, and the reaction time is from 10 minutes to 24 hours.

### [Step VII-2]

The compounds represented by the general formula (XXVI) can be obtained by removing the protective group R¹⁴ of the compound represented by the general formula (XXV) with referring to the method described in the above-mentioned "Protecting Groups in Organic Synthesis".

### [Step VII-3]

The compound represented by the general formula (IVa) can be obtained by reacting the compound represented by the general formula (XXVI) by using a brominating agent or an iodinating agent (for example, *N*-bromosuccinic acid imide, bromine, *N*-iodosuccinic acid imide, iodine and the like) in a suitable solvent (for example, *N,N-*dimethylformamide, methylene chloride, diethyl ether and the like).
The reaction temperature is from -50°C to the boiling point of the solvent, and the reaction time is from 10 minutes to 48 hours.

### [Step VII-4]

The compound represented by the general formula (XXVIII) can be obtained by reacting the compound represented by the general formula (IVa) with the compound represented by the general formula (XXVII) in a suitable solvent (for example, tetrahydrofuran, diethyl ether and the like) in the presence of an organic lithium or an organic magnesium reagent (for example, isopropyl magnesium bromide, *n*-butyllithium and the like). The reaction temperature is from -78°C to the boiling point of the solvent, and the reaction time is from 10 minutes to 48 hours. Meanwhile, the compound represented by the general formula (XXVII) can be obtained by introducing a protective group in a commercially available 2-pyrrolidone with referring to the method described in the above-mentioned "Protecting Groups in Organic Synthesis".

### [Step VII-5]

The compound represented by the general formula (XXIX) can be obtained by reacting the compound represented by the general formula (XXVIII) by using a reducing agent (for examples, sodium borohydrate and the like) in a suitable solvent (for example, methanol, ethanol, tetrahydrofuran, diethyl ether and the like). The reaction temperature is from -20°C to the boiling point of the solvent, and the reaction time is from 10 minutes to 48 hours.

### [Step VII-6]

The compound represented by the general formula (IIIb) can be obtained by cyclizing the compound represented by the general formula (XXIX) by using methanesulfonyl chloride in the presence of a base (for example, triethylamine, pyridine and the like) in a suitable solvent (for example, methylene chloride, tetrahydrofuran and the like). The reaction temperature is from -78°C to the boiling point of the solvent, and the reaction time is from 10 minutes to 48 hours.

### [Step VII-7]

The compound represented by the general formula (IIIc) can be obtained by removing the protective group R¹⁴ of the compound represented by the general formula (IIIb) with referring to the method described in the above-mentioned "Protecting Groups in Organic Syntheses".

### [Reaction step formula VIII]

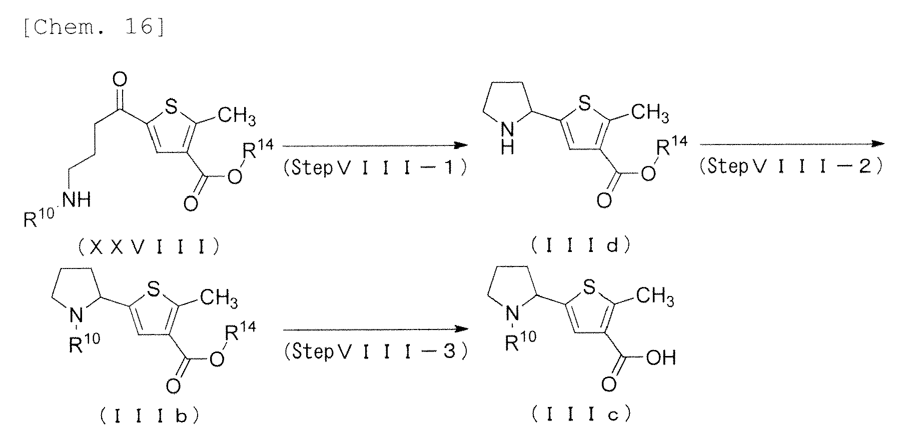

In the formula, all of the symbols are as defined in the above-mentioned general formulas and the above-mentioned Reaction step formulas.

### [Step VIII-1]

The compound represented by the general formula (IIId) can be obtained by removing the protective group R¹⁰ of the compound represented by the general formula (XXVIII) with referring to the method described in the above-mentioned "Protecting Groups in Organic Syntheses", and reacting the product by using a reducing agent (for example, sodium cyanoborohydride and the like) in a suitable solvent (for example, *i*-propanol, ethanol and the like) in the presence of an acid (for example, hydrochloric acid and the like). The reaction temperature is from -20°C to the boiling point of the solvent, and the reaction time is from 10 minutes to 48 hours.

### [Step VIII-2]

The compound represented by the general formula (IIIb) can be obtained by removing the protective group R¹⁰ of the compound represented by the general formula (IIId) with referring to the method described in the above-mentioned "Protecting Groups in Organic Synthesis".

### [Step VIII-3]

The compound represented by the general formula (IIIc) can be obtained by removing the protective group R¹⁴ of the compound represented by the general formula (IIIb) with referring to the method described in the above-mentioned "Protecting Groups in Organic Synthesis".

### [Reaction step formula IX]

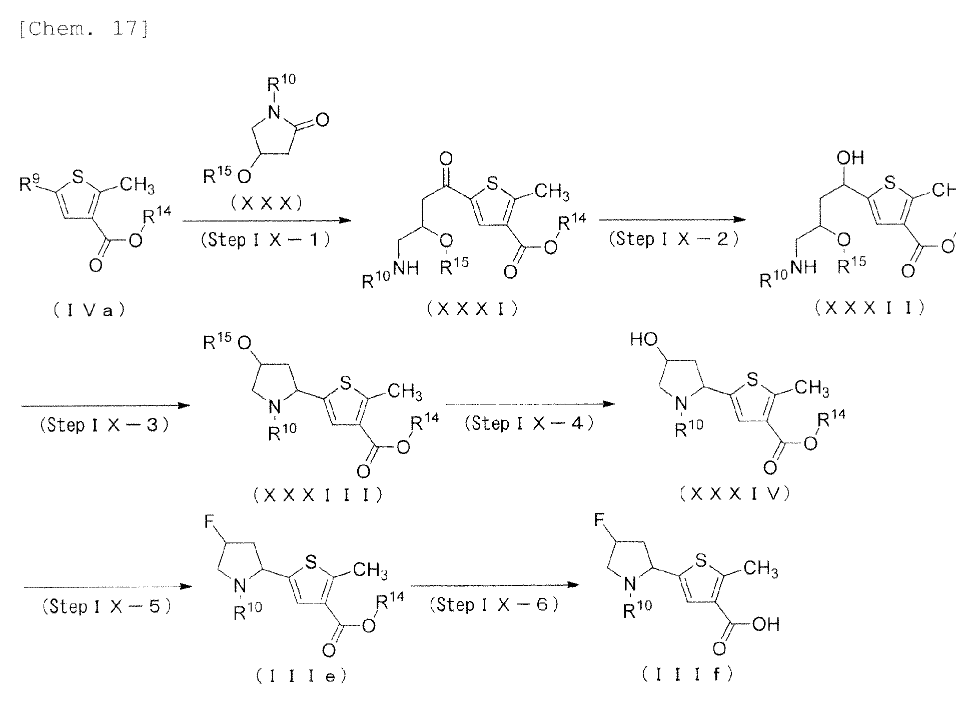

In the formula, R¹⁵ is a protective group for a hydroxy group, and other symbols are as defined in the above-mentioned general formulas and the above-mentioned Reaction step formulas.

### [Step IX-1]

The compound represented by the general formula (XXXI) can be obtained by reacting the compound represented by the general formula (IVa) with the compound represented by the general formula (XXX) in a suitable solvent (for example, tetrahydrofuran, diethyl ether and the like) in the presence of an organic lithium or an organic magnesium reagent (for example, *i*-propylmagnesium bromide, *n*-butyllithium and the like). The reaction temperature is from -78°C to the boiling point of the solvent, and the reaction time is from 10 minutes to 48 hours. Meanwhile, the compound represented by the general formula (XXX) can be obtained by introducing a protective group in a commercially available 4-hydroxypyrrolidin-2-one with referring to the method described in the above-mentioned "Protecting Groups in Organic Synthesis".

### [Step IX-2]

The compound represented by the general formula (XXXII) can be obtained by reacting the compound represented by the general formula (XXXI) by using a reducing agent (for example, sodium borohydrate and the like) in a suitable solvent (for example, methanol, ethanol, tetrahydrofuran, diethyl ether and the like). The reaction temperature is from -20°C to the boiling point of the solvent, and the reaction time is from 10 minutes to 48 hours.

### [Step IX-3]

The compound represented by the general formula (XXXIII) can be obtained by cyclizing the compound represented by the general formula (XXXII) by using methanesulfonyl chloride in a suitable solvent (for example, methylene chloride, tetrahydrofuran and the like) in the presence of a base (for example, triethylamine, pyridine and the like). The reaction temperature is from -78°C to the boiling point of the solvent, and the reaction time is from 10 minutes to 48 hours.

### [Step IX-4]

The compound represented by the general formula (XXXIV) can be obtained by removing the protective group R¹⁵ of the compound represented by the general formula (XXXIII) with referring to the method described in the above-mentioned "Protecting Groups in Organic Synthesis".

### [Step IX-5]

The compound represented by the general formula (IIIe) can be obtained by reacting the compound represented by the general formula (XXXIV) with a fluorinating reagent (for example, dimethylaminosulfur trifluoride, *N*-fiuoro-*N*'-chloromethyltriethylenediamine bis (tetrafluoroborate) and the like) in a suitable solvent (for example, methylene chloride, tetrahydrofuran and the like). The reaction temperature is from -20°C to the boiling point of the solvent, and the react ion time is from 10 minutes to 48 hours.

### [Step IX-6]

The compound represented by the general formula (IIIf) can be obtained by removing the protective group R¹⁴ of the compound represented by the general formula (IIIe) with referring to the method described in the above-mentioned "Protecting Groups in Organic Synthesis".

Other compounds used as starting raw materials, intermediates, or reagents, which are necessary for producing the compounds of the present invention, are available as commercial products, or can be produced with referring to known methods.

It is sometimes effective for the production of a compound to introduce a suitable protective group into a substituent (for example, a hydroxy group, an amino group, a carboxylic acid group and the like) that is included in the compound of the present invention and a compound that is used for the production of said compound in the stage of a raw material or an intermediate, and where necessary, the protective group described in the above-mentioned "Protecting Groups in Organic Synthesis" may be suitably selected and used.

A generally-used method can be used for isolating and purifying the compound of the present invention and the compounds that are used for producing said compound from the reaction liquid. For example, solvent extraction, an ion exchange resin, column chromatography, preparation by high performance liquid chromatography (HPLC), thin layer chromatography using silica gel, alumina or the like as a support, a scavenger resin, recrystallization and the like can be used, and these isolation and purification methods can be conducted solely or in combination. The isolation and purification may be conducted by each reaction, or may be conducted after completion of several reactions.

When the compound in the present specification has asymmetric carbons and optical isomers are present, these optical isomers can be resolved by a general optical resolution method for racemic compounds, for example, conventional methods such as fractionation crystallization comprising recrystallizing as a general diastereomer salt with a general optically active compound, or chromatography. Furthermore, the respective optical isomers can also be isolated by preparation by high performance liquid chromatography using a column for separating optically active substances.

Since the compound of the present invention produced as above acts as a glucokinase activator, it can be used as a pharmaceutical composition. Said pharmaceutical composition is useful as an agent for the prevention or treatment of diabetes or diabetic retinopathy, diabetic nephropathy, diabetic neuropathy, ischemic heart diseases, or chronic complications of diabetes such as arteriosclerosis.

As a form of administration when the compound of the present invention is used as a medicament, the various forms of administration described in the general rules for preparations in the "Japanese Pharmacopoeia" can be selected according to the purpose. For example, in forming into a tablet, an ingredient that can be taken orally that is generally used in the art may be selected. For example, excipients such as lactose, crystal cellulose, sucrose and potassium phosphate correspond to the ingredient.
Furthermore, if desired, various additives that are generally used in the field of formulation such as a binder, a disintegrating agent, a lubricant, an aggregation inhibitor and the like may be incorporated.

The amount of the compound of the present invention that is incorporated as an active ingredient in the formulation of the present invention is not specifically limited and suitably selected from a wide range. The amount to be administered of the active ingredient compound is suitably determined according to the dose regimen thereof, the age, sex and other conditions of a patient, and the degree of a disease, and in the case of oral administration, the compound of the present invention can be suitably administered in the range from about 1 µg to 100 mg per 1 kg body weight a day in one to four portion (s) a day. However, since the amount to be administered and number of administration are determined in view of the relating situations including the degree of the condition to be treated, the selection of the compound to be administered and the selected administration route, the range of the amount to be administered and the number of administration as mentioned above do not limit the scope of the present invention.

### Examples

Hereinafter the contents of the present invention are explained in more detail with referring to Examples, Reference Examples and Pharmacological Test Examples; however, the technical scope of the present invention is not construed to be limited by the contents of the description thereof.
For the nuclear magnetic resonance (¹H-NMR) spectra in the following Examples and Reference Examples, the chemical shift values were described by σ values (ppm) using tetramethylsilane as a standard substance. For the splitting patterns, singlets were represented by "s", doublets were represented by "d", triplets were represented by "t", quartet were represented by represented by "q", multiplets were represented by "m" and broad lines were represented by "br". The mass spectroscopy was conducted by an electrospray ionization method (ESI). In the tables, methyl groups were represented by "Me", ethyl groups were represented by "Et", *i*-propyl groups were represented by "*i*-Pr", *n*-propyl groups were represented by "*n*-Pr", *i*-butyl groups were represented by "*i*-Bu", *t*-butyl groups were represented by "*t*-Bu", *n*-pentyl groups were represented by "*r*-Pen", ethanesulfonyl groups were represented by "Es", methanesulfonyl groups were represented by "Ms", acetyl groups were represented by "Ac", and propionyl groups were represented by "EtCO".

### Reference Example 1

### (S)-4-(t-Butyldimethylsilanyloxy)pyrrolidin-2-one

(*S*)-4-Hydroxypyrrolidin-2-one (101 g) was dissolved in *N*,*N*-dimethylformamide (400 mL), imidazole (102 g) and *t*-butyldimethylchlorosilane (158 g) were added thereto at 0°C, and stirring was conducted at room temperature for 21 hours. The reaction liquid was added to water, and the precipitated white solid was collected by filtration and washed with water to give the title compound (210 g) as a white solid. ¹H-NMR(CDCl₃) δ(ppm): 0.02(6H,s), 0.81(9H,s), 2.16-2.22(1H,m), 2 .42-2.50(1H,m), 3.14-3.18(1H,m), 3.49-3.54(1H,m), 4.46-4.52(1H ,m), 5.76(1H,br.s).
ESI/MS(m/z): 216(M+H)⁺.

### Reference Example 2

### (S)-4-(t-Butyldimethylsilanyloxy)-2-oxopyrrolidine-1-carbox ylic acid t-butyl ester

(*S*)-4-(t-Butyldimethylsilanyloxy)pyrrolidin-2-one (210 g) and 4-dimethylaminopyridine (5.90 g) were dissolved in acetonitrile (500 mL), di-*t*-butyl dicarbonate (234 mL) was added thereto at 0°C, and stirring was conducted at room temperature for 22 hours. Ethyl acetate was added to the reaction liquid, and the reaction liquid was washed sequentially with a saturated aqueous sodium hydrogen carbonate solution, a 4% aqueous citric acid solution and a 1 N aqueous sodium hydroxide solution and dried over anhydrous sodium sulfate. Filtration was conducted, the filtrate was then concentrated under a reduced pressure, and the obtained residue was washed with hexane to give the title compound (271 g) as a white solid.
¹H-NMR (CDCl₃) δ (ppm): 0.05(6H,s), 0.81(9H,s), 1.47(9H,s) 2. 36-2 .42(1H,m), 2.58-2.67(1H,m), 3.56-3.58(1H,m), 3.76-3.81(1H,m), 4 .28-4.34(1H,m).

### Reference Example 3

### 2-Oxopyrrolidine-1-carboxylic acid t-butyl ester

2-Pyrrolidone (25.0 g) was dissolved in acetonitrile (300 mL), di-*t*-butyl dicarbonate (70.5 mL) and 4-dimethylaminopyridine (1.79 g) were added thereto, and stirring was conducted at room temperature for 5 hours. The reaction liquid was concentrated under a reduced pressure, a saturated aqueous sodium hydrogen carbonate solution was added to the obtained residue, and extraction was conducted by using ethyl acetate. The organic phase was washed with saturated brine and dried over anhydrous sodium sulfate. Filtration was conducted, the filtrate was then concentrated under a reduced pressure, and the obtained residue was purified by silica gel chromatography (ethyl acetate) to give the title compound (51.7 g) as a yellow oily substance.
¹H-NMR (CDCl₃) δ (ppm): 1.53(9H,s) 1.96-2.05(2H,m) 2.51(2H,t,*J*= 5.5Hz), 3.75(2H,t, *J*=7.1Hz).

### Reference Example 4

### 2-Methylthiophene-3-carboxylic acid

Diisopropyiamine (233 g) was dissolved in tetrahydrofuran (2.3 L), a solution of *n*-butyllithium in hexane (1.50 L) was added dropwise thereto at 0°C, and stirring was conducted for 40 minutes. The reaction liquid was cooled to from -68 to -60°C, a solution of thiophene-3-carboxylic acid (223 g) in tetrahydrofuran (500 mL) was added dropwise thereto, and the reaction liquid was stirred for 1 hour. Methyl iodide (254 g) was then added thereto, the temperature was raised to room temperature, and the reaction liquid was further stirred for 1 hour. The reaction liquid was concentrated under a reduced pressure, 6 N hydrochloric acid was added to the obtained residue to adjust the pH to 1, and extraction was conducted by using ethyl acetate. The organic phase was washed with saturated brine and dried over anhydrous sodium sulfate. Filtration was conducted, the filtrate was then concentrated under a reduced pressure, and the obtained residue was recrystallized with water/acetic acid to give the title compound (209 g).
¹H-NMR (CDCl₃) δ (ppm) :2.78(3H,s) 7.01(1H,d, *J*=5.5Hz) 7.45(1H,d , *J*=5.5Hz).
ESI/MS(m/z): 141(M-H)⁻.

### Reference Example 5

### 2-Methylthiophene-3-carboxylic acid methyl ester

2-Methylthiophene-3-carboxylic acid (100 g) was dissolved in methanol (500 mL), thionyl chloride (200 mL) was slowly added dropwise thereto, and reflux was conducted for 3 hours. The reaction liquid was concentrated under a reduced pressure, water was added to the obtained residue, and extraction was conducted by using methylene chloride. The organic phase was washed sequentially with a saturated aqueous sodium hydrogen carbonate solution and saturated brine and dried over anhydrous sodium sulfate. Filtration was conducted, and the filtrate was then concentrated under a reduced pressure to give the title compound (105 g).
¹H-NMR (CDCl₃) δ (ppm) :2.74(3H,s), 3.85(3H,s) , 6.98 (1H,d, *J*=5.1Hz ), 7.38 (1H,d, *J*=5.5Hz).

### Example 1

### 5-Bromo-2-methylthiophene-3-carboxylic acid methyl ester

2-Methylthiophene-3-carboxylic acid methyl ester (156 g) was dissolved in *N*,*N*-dimethylformamide (750 mL), *N*-bromosuccinimide (178 g) was added thereto, and stirring was conducted overnight at room temperature. Water was added to the reaction liquid, and extraction was conducted by using hexane. The organic phase was washed sequentially with an aqueous sodium hydrogen sulfate solution and saturated brine and dried over anhydrous sodium sulfate. Filtration was conducted, and the filtrate was then concentrated under a reduced pressure to give the title compound (226 g). ¹H-NMR (CDCl₃) δ (ppm): 2.67(3H,s) 3.84(3H,s) 7.33(1H,s).

### Reference Example 6

### (S)-4-Hydroxy-2-(4-methoxycarbonyl-5-methylthiophen-2-yl)py rrolidine-1-carboxylic acid t-butyl ester

Under an argon atmosphere, 5-bromo-2-methylthiophene-3-carboxylic acid methyl ester (227 g) was dissolved in tetrahydrofuran (350 mL) and cooled to 0°C. Isopropylmagnesium bromide (1500 mL) was added dropwise thereto at 10°C or less, and stirring was conducted at 0°C for 2 hours. A solution of (*S*)-4-(t-butyldimethylsilanyloxy)-2-oxopyrrolidine-1-carbox ylic acid *t*-butyl ester (276 g) in tetrahydrofuran (550 mL) was then added dropwise thereto at 10°C or less, and stirring was conducted at room temperature for 1.5 hours. The reaction liquid was cooled to 0°C, a 30% aqueous citric acid solution was added dropwise thereto at 10°C or less to adjust the pH 3, and stirring was conducted at room temperature for 1.5 hours. Ethyl acetate was added to the reaction liquid, and the organic phase was washed sequentially by a 5% aqueous sodium hydrogen carbonate solution and saturated brine and dried over anhydrous sodium sulfate. Filtration was conducted, and the filtrate was then concentrated under a reduced pressure to give a crude product (418 g).
Under an argon atmosphere, (*R*)-5,5-diphenyl-2-methyl-3,4-propano-1,3,2-oxaborolidine (49.1 g) was dissolved in tetrahydrofuran (86 mL), a boran-dimethylsulfide complex (886 mL) was added dropwise thereto at room temperature, and stirring was conducted for 20 minutes. A solution of the above-mentioned crude product (418 g) in tetrahydrofuran (450 mL) was then added dropwise thereto at room temperature over 45 minutes, and stirring was conducted for 30 minutes. The reaction liquid was cooled to 0°C, methanol/saturated brine was added dropwise thereto, ethyl acetate was then added thereto, and the organic phase was washed with saturated brine and dried over anhydrous sodium sulfate. Filtration was conducted, the obtained filtrate was then concentrated under a reduced pressure, and the obtained residue was passed through a short column of a silica gel (ethyl acetate/hexane=1/2) to give a crude product (337 g).
Under an argon atmosphere, the above-mentioned crude product (302 g) and triethylamine (265 mL) were dissolved in methylene chloride (800 mL), a solution of methanesulfonyl chloride (73.8 mL) in methylene chloride (295 mL) was added dropwise thereto at 0°C, and stirring was conducted for 30 minutes. Water was added to the reaction liquid, extraction was conducted by using methylene chloride, and the organic phase was dried over anhydrous sodium sulfate. Filtration was conducted, and the filtrate was then concentrated under a reduced pressure to give a crude product (304 g).
The above-mentioned crude product (265 g) was dissolved in tetrahydrofuran (265 mL)) , tetrabutylammonium fluoride (565 mL) was added dropwise thereto at room temperature, and stirring was conducted continuously for 1.5 hours. The reaction liquid was concentrated under a reduced pressure, extracted with ethyl acetate, washed sequentially with water and saturated brine and dried over anhydrous sodium sulfate. Filtration was conducted, the filtrate was then concentrated under a reduced pressure, and the obtained residue was purified by silica gel column chromatography (ethyl acetate/hexane=1/5-2/1) to give the title compound (128 g) as a yellow white solid.
¹H-NMR (CDCl₃) δ (ppm):1.24-1.45(9H,m), 1.66(1H,t, *J*=4.9Hz), 2.18 (1H, d, *J*=14.3Hz), 2.35-2.51(1H,m), 2.71(3H,s), 3.51(1H,d, *J*=11.7 Hz), 3.66(1H, t, *J*=7.7Hz), 3.83(3H,s), 4.48-4.58(1H,m), 5.09(1H,b r.s) , 7.16-7.21(1H,m).
ESI/MS(m/z) : 342(M+H) ⁺,340(M-H)⁻.

### Example 2

### (2S,4R)-4-Fluoro-2-(4-methoxycarbonyl-5-methylthiophen-2-yl )pyrrolidine-1-carboxylic acid t-butyl ester and Example 3 (2R,4R)-4-Fluoro-2-(4-methoxycarbonyl-5-methylthiophen-2-yl )pyrrolidine-1-carboxylic acid t-butyl ester

(*S*)-4-hydroxy-2-(4-methoxycarbonyl-5-methylthiophen-2-yl)py rrolidine-1-carboxylic acid *t*-butyl ester (113 g) was dissolved in methylene chloride (1130 mL) and cooled to 0°C. Diethylaminosulfur trifluoride (130 mL) was added dropwise thereto at 5°C or less, and stirring was conducted for 8 hours at room temperature. The reaction liquid was cooled to 0°C, a saturated aqueous sodium hydrogen carbonate solution was added dropwise thereto, and the reaction liquid was extracted with methylene chloride and dried over anhydrous sodium sulfate. Filtration was conducted, the filtrate was then concentrated under a reduced pressure, and the obtained residue was purified by silica gel column chromatography (ethyl acetate/hexane-1/20-1/1) to give the title compound (2*S*,4*R*) (55. 5 g) as a yellow solid and the title compound (2*R*,4*R*) (21.5 g) as a yellow solid.
Physical property data of the (2*S*,4*R*) form
¹H-NMR(CDCl₃)δ(ppm): 1.32-1.47(9H,m), 2.05-2.23(1H,m), 2.65-2. 75(4H,m), 3.54-3.70(1H,m), 3.82(3H,s), 3.90-4.05(1H,m), 5.05-5. 30(3H,m),7.18(1H,s).
ESI/MS(m/z) : 344(M+H)⁺, 342(M-H)⁻.
Physical property data of the (2R,4R) form
¹H-NMR(CDCl₃)δ(ppm): 1.45(9H, b.r.d, *J*=42.1Hz), 2.43-2.55(2H,m), 2.67(3H,s), 3.75-3.85(4H,m), 5.12-5.35(2H,m), 7.20(1H,s). ESI/MS(m/z): 344(M+H)⁺,342(M-H)⁻.

### Reference Example 7

### 5-(4-t-Butoxycarbanylamino-1-hydroxybutyl)-2-methylthiopnene-3-carboxylic acid methyl ester

Under an argon atmosphere, 5-bromo-2-methylthiophene-3-carboxylic acid methyl ester (2.00 g) was dissolved in tetrahydrofuran (9.47 mL), isopropylmagnesium bromide (12.9mL) was added dropwise thereto at -40°C, and stirring was conducted for 1.5 hours. A solution of 2-oxopyrrolidine-1-carboxylic acid *t*-butyl ester (1.89 g) in tetrahydrofuran (11.2 mL) was added dropwise at -40°C, and stirred at room temperature for 24 hours. The reaction liquid was cooled to -10°C, methanol (17.2 mL) was added thereto, sodium borohydrate (486 mg) was then added thereto in portions, and stirring was conducted for 15 minutes. A saturated aqueous ammonium chloride solution was added to the reaction liquid, and the reaction liquid was extracted with chloroform and dried over anhydrous sodium sulfate. Filtration was conducted, the filtrate was then concentrated under a reduced pressure, and the obtained residue was purified by silica gel chromatography (hexane/ethyl acetate=1/1) to give the title compound (2.13 g) as a yellow oily substance.
¹H-NMR(CDCl₃)δ(ppm): 1.44(9H,s), 1.51-1.69(4H,m) 1.79-1.84(1H ,m), 2.70(3H,s), 3.15-3.19(1H,m), 3.83(3H,s), 4.57-4.87(1H,m), 7 .26(1H,s) .

### Example 4

### 2-(4-Methoxycarbonyl-5-methylthiophen-2-yl)pyrrolidine-1-ca rboxylic acid t-butyl ester

5-(4-*t*-Butoxycarbonylamino-1-hydroxybutyl)-2-methylthiophe ne-3-carboxylic acid methyl ester (2.13 g) was dissolved in methylene chloride (58 mL), and the mixrure was cooled to -6 0°C. Triethylamine (2.58 mL) and methanesulfonyl chloride (530 µL) were added dropwise, and stirring was conducted at -60°C for 2 hours. Water was added to the reaction liquid, a nd the reaction liquid was extracted with chloroform and dri ed over anhydrous sodium sulfate. Filtration was conducted, the filtrate was then concentrated under a reduced pressure, and the obtained residue was purified by silica gel chromat ography (hexane/ethyl acetate=1/0-4/1) to give the title com pound (1.52 g) as a yellow oily substance.
¹H-NMR(CDCl₃)δ(ppm): 1.36-1.46(9H,m), 1.95-1.99(3H,m), 2.19-2. 28(1H,m), 2.67(3H,s), 3.40-3.59(2H,m), 3.82(3H,s), 4.95-5.07(1H ,m), 7.12(1H,s).
ESI/MS(m/z): 326(M+H)⁺, 324(M-H)⁻.

### Example 5

### 2-Methyl-5-pyrrolidin-2-ylthiophene-3-carboxylic acid methyl ester

2-(4-Methoxyearbonyl-5-methylthiophen-2-yl) pyrrolidine-1-carboxylic acid *t*-butyl ester (132 g) was dissolved in metha nol (500 mL), methane sulfonate (34.4 mL) was added dropwise thereto, and stirring was conducted at room temperature for 1 hour, and further stirred at 50°C for 7 hours. The reacti on liquid was concentrated under a reduced pressure, methano 1 (360 mL) was added to the obtained residue, and the residu e was cooled to 0°C. A 1 N aqueous sodium hydroxide solution was added to adjust the pH 8, and extraction was conducted b y using methylene chloride. The organic phase was washed wi th saturated brine and dried over anhydrous sodium sulfate. Filtration was conducted, and the filtrate was then concent rated under a reduced pressure to give the title compound (9 1.4 g) as a pale red oily substance.
¹H-NMR(CDCl₃)δ(ppm): 1.94-1.74(3H,m), 2.14-2.18(1H,m), 2.68 (3H ,s), 2.98-3.00(1H,m), 3.10-3.13(1H,m), 3.82(3H,s), 4.30(1H,t,*J*= 7.0Hz), 7.17(1H,s).
ESI/MS(m/z) :226(M+H)⁺.

### Example 6

### (-)-2-Methyl-5-pyrrolidin-2-ylthiophene-3-carboxylic acid methyl ester (-)-dibenzoyl-L-tartrate

(-)-Dibenzoyl-L-tartaric acid (35.3 g) was dissolved in methanol (1.00 L), a solution of 2-methyl-5-pyrrolidin-2-ylthiophene-3-carboxylic acid methyl ester (21.1 g) in methanol (400 mL) was added thereto, and the solution was stood still at room temperature for 9 hours. The precipitated crystal was collected by filtration and washed with methanol to give the title compound (11.8 g) as a white crystal.
¹H-NMR(CDCl₃)δ(ppm): 1.84-1.99(3H,m), 2.28-2.21(1H,m), 2.62(3H ,s), 3.13-3.18(2H,m), 3.77(3H,s), 4.66 (1H,t ,*J*=7.7Hz), 5.68(2H,s ), 7.39(1H,d, *J*=8.4Hz), 7.50(4H,t, *J*=7.7Hz), 7.62-7.4(2H,m), 7.9 5(4H,t, *J*=4.2Hz).
ESI/MS(m/z): 226(M+H)⁺.

### Example 7

### (-)-2-(4-Carboxy-5-methylthiophen-2-yl)pyrrolidine-1-carbox ylic acid t-butyl ester

(-)-2-Methyl-5-pyrrolidin-2-ylthiophene-3-carboxylic acid methyl ester (-)-dibenzoyl-L-tartrate (23.7 g) was suspended in chloroform (100 mL), an aqueous 1 N sodium hydroxide solution (82.0 mL) was added thereto, and stirring was conducted at room temperature for 1 hour. The reaction liquid was extracted with chloroform, washed with saturated brine and dried over anhydrous sodium sulfate. The reaction liquid was concentrated under a reduced pressure to give a colorless oily substance (10.6 g), and the substance was then dissolved in methylene chloride (90 mL), di-*t*-butyl dicarbonate (11.2 mL) and triethylamine (6.80 mL) were added dropwise thereto, and stirring was conducted at room temperature for 2 hours. The reaction liquid was concentrated under a reduced pressure to give a pale orange oily substance (17.2 g) and the substance was then dissolved in methanol (100 mL), water (10 mL) and lithium hydroxide monohydrate (5.10 g) were added therto, and stirring was conducted at 50°C for 6 hours. The reaction liquid was cooled to 0°C, water was added thereto, the reaction liquid was neutralized with 1 N hydrochloric acid, and the precipitated crystal was collected by filtration to give the title compound (12.6 g) as a white solid.
¹H-NMR(CDCl₃)δ(ppm): 1.39(9H,br.d, *J*=51.6Hz), 1.92-2.02(3H,m), 2.28(1H,br.s), 2.66(3H,s), 3.39-3.52(2H,m), 4.97(1H,br.d, *J*=19H z), 7.12(1H,s).
ESI/MS(m/z):310(M-H)⁻.

### Example 8

### (2S,4R)-2-(4-Carboxy-5-methylthiophen-2-yl)-4-fluoropyrroli dine-1-carboxylic acid t-butyl ester

(2*S*,4*R*)-4-Fluoro-2-(4-methoxycarbonyl-5-methylthiophen-2-yl )pyrrolidine-1-carboxylic acid *t*-butyl ester (12.1 g) and lithium hydroxide monohydrate (4.40 g) were dissolved in a mixed solvent of methanol (90 mL) and water (30 mL)) , and stirring was conducted at 50°C for 4 hours. The reaction liquid was cooled to 0°C, water was added thereto, the pH was adjusted to 6 with 1 N hydrochloric acid, and the precipitated crystal was collected by filtration. The crystal collected by filtration was dissolved in ethyl acetate and dried over anhydrous sodium sulfate. Filtration was conducted, and the filtrate was then concentrated under a reduced pressure to give the title compound (11.2 g) as a white solid.
¹H-NMR(CDCl₃)δ(ppm): 1.34-1.44(9H,m), 1.95-2.05(3H,m), 2.21-2. 32(1H,m), 2.66(3H,s), 3.42-3.51(2H,m), 5.05-5.15(1H,m), 7.12(1H ,s).
ESI/MS(m/z): 328(M-H)⁻.

### Example 9

### 2-(4-Carboxy-5-methylthiophen-2-yl)pyrrolidine-1-carboxylic acid t-butyl ester

2-(4-Methoxycarbonyl-5-methylthiophen-2-yl)pyrrolidine-1-c arboxylic acid *t*-butyl ester (1. 52 g) was dissolved in methanol (14.0 mL), water (2.33 mL) and lithium hydroxide monohydrate (588 mg) were added thereto at 0°C, and stirring was conducted at 50°C for 17 hours. The reaction liquid was cooled to 0°C_{,} water was added thereto, the reaction liquid was neutralized with 1 N hydrochloric acid, and the precipitated crystal was collected by filtration to give the title compound (1.26 g) as a white solid.
¹H-NMR(CDCl₃)δ(ppm):1.34-1.44(9H,m), 1.95-2.05(3H,m), 2.21-2. 32(1H,m), 2.66(3H,s), 3.42-3.51(2H,m), 5.05-5.15(1H,m), 7.12(1H ,s).
ESI/MS(m/z): 310(M-H)⁻.

### Reference Example 8

### (-)-2-"4-(5-Fluorothiazol-2-ylcarbamoyl)-5-methylthiophen-2 -yl"pyrrolidine-1-carboxylic acid t-butyl ester

(-)-2-(4-Carboxy-5-methylthiophen-2-yl)pyrrolidine-1-carbo xylic acid *t*-butyl ester (1.00 g) was dissolved in a mixed solvent of toluene (10 mL) and methylene chloride (10 mL), oxalyl chloride (2.74 mL) was added dropwise thereto, and stirring was conducted at room temperature for 1 hour. The reaction liquid was concentrated under a reduced pressure, the obtained residue was dissolved in a mixed solvent of toluene (10 mL) and methylene chloride (10 mL), 2-amino-5-fluorothiazole hydrochloride (745 mg) and *N*, *N*-diethylaniline (1,98 mL) were added thereto, and stirring was conducted overnight at room temperature. Water was added to the reaction liquid, extraction was conducted by using ethyl acetate, and the organic phase was washed sequentially with a saturated aqueous sodium hydrogen carbonate solution and saturated brine and dried over anhydrous sodium sulfate. Filtration was conducted, the filtrate was then concentrated under a reduced pressure, and the obtained residue was purified by silica gel chromatography (hexane/ethyl acetate=4/1-2/1) to give the title compound (1.01 g) as a white powder.
¹H-NMR(CDCl₃)δ(ppm): 1.35-1.46(9H,m), 1.70-1.75(1H,m), 1.88-1. 97(2H,m), 2.9-2.45(1H,m), 2.73(3H,s), 3.38-3.55(2H,m), 4.98(1H ,br.t, *J*=35.3Hz), 6.84(1H,br.d, *J*=36.6Hz), 7.05(1H,br.t, *J*=15.7H z), 10.27(1H,br.s).
ESI/MS(m/z): 412(M+H)⁺ 410(M-H)⁻.

### Reference Example 9

### (-)-2-[4-(5-Chlorothiazol-2-ylcarbamoyl)-5-methylthiophen-2 -yl]pyrrolidine-1-carboxylic acid t-butyl ester

The title compound was obtained by conducting a reaction according to a similar method to that of Reference Example 8 by using 2-amino-5-chlorothiazole hydrochloride instead of 2-amino-5-fluorothiazole hydrochloride.
¹H-NMR(COCl₃)δ(ppm): 1.16-1.54(9H,m), 1.89-2.12(3H,m), 2.23-2. 37(1H,m), 2.68(3H,s), 3.39-3.57(2H,m), 5.03(1H,br.s), 7.24(1H,b r.s), 7.33(1H,br.s).
ESI/MS(m/z): 428(M+H)⁺,426(M-H)⁻.

### Reference Example 10

### (-)-2-[5-Methyl-4-([1,2,4]thiadiazol-5-ylcarbamoyl)thiophen -2-yl]pyrrolidine-1-carboxylic acid t-butyl ester

The title compound was obtained by conducting a reaction according to a similar method to that of Reference Example 8 by using 5-amino-1,2,4-thiathzazole instead of 2-amino-5-fluorothiazole hydrochloride.
¹H-NMR(CDCl₃)δ(ppm): 1.32-1.46(9H,m), 1.95-2.01(3H,m), 2.21-2. 34(1H,m), 2.78(3H,s), 3.38-3.59(2H,m), 4.95-5,15(1H,m), 7.16-7. 20 (1H,m),8.28 (1H,s).
ESI/MS(m/z): 395(M+H)⁺, 393(M-H)⁻.

### Reference Example 11

### (-)-2-[4-(3-Ethyl-[1,2,4]thiadiazol-5-ylcarbamoyl)-5-methyl thiophen-2-yl]pyrrolidine-1-carboxylic acid t-butyl ester

The title compound was obtained by conducting a reaction according to a similar method to that of Reference Example 8 by using 5-amino-3-ethyl-[1,2,4]-thiathiazole instead of 2-amino-5-fluorothiazole hydrochloride.
ESI/MS(m/z): 423(M+H)⁺,421(M-H)⁻.

### Reference Example 12

### (2S,4R)-4-Fluoro-2-[4-(5-fluorothiazol-2-ylcarbamoyl)-5-met hylthiophen-2-yl]pyrrolidine-1-carboxylic acid t-butyl ester

(2*S*,4*R*)-2-(4-Carboxy-5-methylthiophen-2-yl)-4-fluoropyrrol idine-1-carbaxylic acid *t*-butyl ester (11.0 g) was dissolved in toluene (180 mL), oxalyl chloride (28.6 mL) was added dropwise thereto at 0°C, and stirring was conducted at room temperature for 2 hours. The reaction liquid was concentrated under a reduced pressure, the obtained residue was dissolved in toluene (150 mL), *N*,*N*-diethylaniline (21.3 mL) and 2-amina-5-fluorothiazole hydrochloride (7.75 g) were added thereto at 0°C, and stirring was conducted at room temperature for 13 hours. Water was added to the reaction liquid, extraction was conducted by using ethyl acetate, and the organic phase was washed with a saturated aqueous sodium hydrogen carbonate solution and dried over anhydrous sodium sulfate. Filtration was conducted, the filtrate was then concentrated under a reduced pressure, and the obtained residue was purified by silica gel chromatography (ethyl acetate/hexane=1/20-1/5) to give the title compound (11.8 g) as a pale yellow white powder. ¹H-NMR(CDCl₃)δ(ppm): 1.26-1.45(9H,m), 2.04-2.20(1H,m), 2.70-2. 75(4H,m), 3.57-3.68(1H,m), 2.73(3H,s), 3.38-3.55(2H,m), 4.00 (1H ,br.s), 5.15-5.20(1H,m), 6.91(1H,s), 7.10(1H,s), 10.07(1H,br.s)
ESI/MS(m/z): 430(M+H)⁺, 428(M-H)⁻ .

### Reference Example 13

### (2S,4R)-4-Fluoro-2-[4-(5-chlorothiazol-2-ylcarbamoyl)-5-met hylthiophen-2-yl],pyrrolidine-1-carboxylic acid t-butyl ester

The title compound was obtained by conducting a reaction according to a similar method to that of Reference Example 12 by using 2-amino-5-chlorothiazole instead of 2-amino-5-fluorothiazole hydrochloride.
¹H-NMR(CD₃OD)δ(ppm): 1.37-1.46(9H,m), 2.15-2.28(1H,m), 2.68-2. 78(4H,m), 3.58-4.24(2H,m), 5.15-5.33(1H,m), 5.68-6.02(1H,m), 7. 34-7.37 (2H,m) .
ESI/MS(m/z): 446(M+H)⁺, 444(M-H)⁻.

### Reference Example 14

### (2S,4R)-2-[4-(3-Ethyl-[1,2,4]thiadiazol-5-ylcarbamoyl)-5-me thylthiophen-2-yl)-4-fluoropyrrolidine-1-carboxylic acid t-butyl ester

The title compound was obtained by conducting a reaction according to a similar method to that of Reference Example 12 by using 3-ethyl-5-amino-[1,2,4]thiadiazole instead of 2-amino-5-fluorothiazole hydrochloride.
ESI/MS(m/z): 441(M+H)⁺, 439(M-H)⁻.

### Reference Example 15

### (2S,4R)-4-Fluoro-2-[5-methyl-4-([1,2,4]thiadiazol-5-ylcarba moyl) thiophen-2-yl]pyrrolidine-1-carboxylic acid t-butyl ester

The title compound was obtained by conducting a reaction according to a similar method to that of Reference Example 12 by using 5-amino-[1,2,4]-thiathiazole instead of 2-amino-5-fluorothiazole hydrochloride.
¹HNMR(CDCl₃)δ(ppm): 1.3-1.5(9H,m), 2,14(1H,ddt, *J*=38,3.6,9.6Hz ),2.70 (1H,m),2.79 (3H,s),3.63 (1H,ddd,*J*=38,8.0,3.6Hz),4.00 (1H ,m),5.0-5.5 (2H,m),7.25 (1H,br.s),8.31 (1H,s),10.7 (1H,br.s).

### Reference Example 16

### 2-[4-(5-Fluorothiazol-2-ylcarbamoyl)-5-methylthiophen-2-yl] pyrrolidine-1-carboxylic acid t-butyl ester

2-(4-Carboxy-5-methylthiophen-2-yl)pyrrolidine-1-carboxyli c acid *t*-butyl ester (1.56 g) was suspended in toluene (1 6 mL)), oxalyl chloride (4.30 mL) was added dropwise thereto at 0°C, and stirring was conducted at room temperature for 1 hour. The reaction liquid was concentrated under a reduced pressure, the obtained residue was dissolved in toluene (16 mL), 2-amino-5-fluorothiazole hydrochloride (1.16 g) and *N*,*N*-diethylaniline (3.20 mL) were added thereto at 0°C, and stirring was conducted overnight at room temperature. Water was added to the reaction liquid, extraction was conducted by using ethyl acetate, and the organic phase was washed sequentially with a saturated aqueous sodium hydrogen carbonate solution and saturated brine and dried over anhydrous sodium sulfate. Filtration was conducted, the filtrate was then concentrated under a reduced pressure, and the obtained residue was purified by silica gel chromatography (hexane/ethyl acetate=3/2) to give the title compound (1.62 g) as a white powder.
¹H-NMR(CDCl₃)δ(ppm): 1.36-1.46(9H,m), 1.91-1.99(3H,m), 2.21-2. 28(1H,m), 2.74((3H,s), 3.41-3.56(2H,m), 4.99-5.09(1H,m), 6.98(1H ,s), 7.06(1H,br.s).
ESI/MS (m/z) : 412(M+H)⁺, 410(M-H)⁻.

### Reference Example 17

### 2-[4-(5-Chlorothiazol-2-ylcarbamoyl)-5-methylthiophen-2-yl] pyrrolidine-1-carboxylic acid---butyl ester

The title compound was obtained by conducting a reaction according to a similar method to that of Reference Example 16 by using 2-amino-5-chlorothiazole instead of 2-amino-5-fluorothiazole hydrochloride.
¹H-NMR(CDCl₃)δ(ppm): 1.24-1.46(9H,m), 1.93-1.97(3H,m), 2.23-2. 32 (1H,m),2.74 (3H,s),3.40-3.56 (2H,m),4.95-5.11 (1H,m),6.87-7. 12(1H,m), 7.18(1H,br.s), 9.89(1H,br.s).
ESI/MS(m/z) : 428(M+H)⁺, 426(M-H)⁻ .

### Reference Example 18

### 2-[5-Methyl-4-(thiazol-2-ylcarbamoyl) thiophen-2-yl] -pyrroli dine-1-carboxylic acid t-butyl ester

The title compound was obtained by conducting a reaction according to a similar method to that of Reference Example 16 by using 2-aminothiazole instead of 2-amino-5-fluorothiazole hydrochloride.
¹H-NMR(CDCl₃)δ(ppm): 1.24-1.46(9H,m), 1.93-1.97(3H,m), 2.23-2. 32(1H,m), 2.74(3H,s), 3.40-3.56(2H,m), 4.95-5.11(1H,m), 6.87-7. 12(1H,m), 7.18(1H,br.s), 9.89(1H,br.s).
ESI/MS(m/z): 428(M+H)⁺, 426(M-H)⁻.

### Reference Example 19

### 2-[5-Methyl-4-([1,2,4]thiadiazol-5-ylcarbamoyl)thiophen-2-y 1]pyrrolidine-1-carboxylic acid t-butyl ester

The title compound was obtained by conducting a reaction according to a similar method to that of Reference Example 16 by using 5-amino-[1,2,4]thiadiazole instead of 2-amino-5-fluorothiazole hydrochloride.
ESI/MS(m/z): 395(M+H)⁺, 393(M-H)⁻.

### Reference Example 20

### 2-[4-(3-Ethyl-[1,2,4] thiadiazol-5-ylcarbamoyl)-5-methylthio phen-2-yl]pyrrolidine-1-carboxylic acid t-butyl ester

The title compound was obtained by conducting a reaction according to a similar method to that of Reference Example 16 by using 5-amino-3-ethyl-[1,2,4]thiadiazole instead of 2-amino-5-fluorothiazole hydrochloride.
ESI/MS(m/z): 423(M+H)⁺, 421(M-H)⁻.

### Reference Example 21

### 2-[5-Methyl-4-[3-methylsulfanyl-[1,2,4]thiadiazol-5-ylcarba moyl]thiophen-2-yl]pyrrolidine-1-carboxylic acid t-butyl ester

The title compound was obtained by conducting a reaction according to a similar method to that of Reference Example 16 by using 5-amino-3-methylsulfanyl-[1,2,4] thiadiazole instead of 2-amino-5-fluorothiazole hydrochloride.
ESI/MS(m/z) : 441(M+H)⁺, 439(M-H)⁻ .

### Reference Example 22

### 2-[4-[3-Methoxy-[1,2,4] thiadiazol-5-ylcarbamoyl]-5-methyith iophen-2-yl]pyrrolidine-1-carboxylic acid t-butyl ester

The title compound was obtained by conducting a reaction according to a similar method to that of Reference Example 16 by using 5-amino-3-methoxy-[1,2,4]thiadiazole instead of 2-amino-5-fluorothiazole hydrochloride.
ESI/MS(m/z) : 425(M+H)⁺, 423(M-H)⁻.

### Reference Example 23

### (-)-2-Methyl-5-pyrrolidin-2-ylthiophene-3-carboxylic acid (5-fluorothiazol-2-yl)amide

(-)-2-"4-(5-Fluorothiazol-2-ylcarbamoyl)-5-methylthiophen-2-yl"pyrrolidine-1-carboxylic acid *t*-butyl ester (970 mg) was dissolved in methylene chloride (10 mL), trifluoroacetic acid (2.72 mL) was added thereto, and stirring was conducted at room temperature for 2 hours. The reaction liquid was concentrated under a reduced pressure, a saturated aqueous sodium hydrogen carbonate solution was added to the obtained residue, and extraction was conducted by using methylene chloride. The organic phase was washed with saturated brine and dried over anhydrous sodium sulfate. Filtration was conducted, and the filtrate was then concentrated under a reduced pressure to give the title compound (578 mg) as a white powder.
¹H-NMR(CDCl₃)δ(ppm): 1.70-1.98(3H,m), 2.14-2.22(1H,m), 2.73 (3H ,s), 2.99-3.05(1H,m), 3.11-3.15(1H,m), 4.34(1H,t, *J*=7.1Hz), 6.80 (1H,d, *J*=2.6Hz), 7.08 (1H,s).
ESI/MS(m/z): 312(M+H)⁺, 310(M-H)⁻.

Compounds were synthesized according to the following reaction formula with referring to the method of Reference Example 23. The synthesized compounds are shown in Table 1, and the data are shown in Table 2.

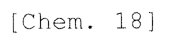

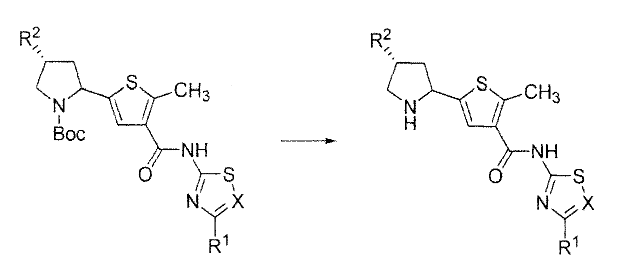

**[Table 1]**

| Reference Example | Starting raw material | X | R¹ | R² |
|---|---|---|---|---|
| Reference Example 24 | Reference Example 9 | C-Cl | H | H |
| Reference Example 25 | Reference Example 10 | N | H | H |
| Reference Example 26 | Reference Example 11 I | N | Et | H |
| Reference Example 27 | Reference Example 12 | C-F | H | F |
| Reference. Example 28 | Reference Example 13 | C-Cl | H | F |
| Reference Example 29 | Reference Example 14 | N | Et | F |
| Reference Example 30 | Reference Example 16 | C-F | H | H |
| Reference Example 31 | Reference Example 17 | C-Cl | H | H |
| Reference Example 32 | Reference Example 18 | C-H | H | H |
| Reference Example 33 | Reference Example 19 | N | H | H |
| Reference Example 34 | Reference Example 20 | N | Et | H |
| Reference Example 35 | Reference Example 21 | N | SMe | H |
| Reference Example 36 | Reference Example 22 | N | OMe | H |

**[Table 2]**

| Reference Example | ¹H-NMR(CDCl₃)δ(ppm) | ESI/MS(m/z): |
|---|---|---|
| Reference Example 24 | 1.77-2.03(3H,m),2.18-2.32(1H,m),2.70(3H,s),2.91-3.01(1H,m),3.08-3.17(1H,m),4.27(1H,t,*J*=7.1Hz),7.30(1H,s),7.31(1H,s). | 328(M+H)⁺ 326(M-H)⁻ |
| Reference Example 25 | 1.70-1.96(3H,m),2.16-2.26(1H,m),2.78(3H,s),3.00-3.07(1H,m),3.13-3.16(1H,m),4.37(1H,t,*J*=7.0Hz),7.13(1H,s),8.28(1H,s). | 295(M+H)⁺ 293(M-H)⁻ |
| Reference Example 26 | 1.35(3H,m), 1.72-1.98(3H,m),2,05-2.25(1H,m),2.77(3H,s),2.83-2.89( 2H,m),3.00-3.08(1H,m),3.11-3.17(1H,m),4.35(1H,t,*J*=7.1Hz),7.07(1 H,s). | 323(M+H)⁺ 321(M-H)⁻ |
| Reference Example 27 | 1.83-2.00(1H,m),2.47-2.57(3H,m),2.72(314,s),3.20-3.37(2H,m),4.67( 1H,t,*J*=7.9Hz),5.22-5.36(1H,m),6,93(1H,s),7.05(1H,s). | 330(M+H)⁺ 328(M-H)⁻ |
| Reference Example 28 | 1.83-1.98(1H,m),2.46-2.57(1H,m),2.74(3H,s),3.20-3.37(2H,m),4.64-4.68(1H,m),5.22-5.36(1N,m),5.84-6.05(1H,m),7.09(1H,t,*J*=3.5Hz),7. 10(1H,s). | 346(M+H)⁺ 344(M-H)⁻ |
| Reference Example 29 | 1.36(31-1,t,*J*=7.7Hz),1.83-2.00(1H,m),2.48-2.59(1H,m),2.79(3H,s),2. 84-2.90(2H,m),3.18-3.34(2H,m),4.60-4.71(1H,m),5.23-5.38(1H,m),7 .07(1H,s). | 341 (M+H)⁺ 339 (M-H)⁻ |
| Reference Example 30 | 1.74-1.96(3H,m),2.16-2.19(1H,m),2.73(3H,s),2.99-3.05(1H,m),3.10-3.16(1H.m),4.33(1H,t,,*J*=7.0Hz),6.87(1H,d,*J*=2.6Hz),7.06(1H,s). | 312(M+H)⁺ 310(M-H)⁻ |
| Reference Example 31 | 1.52-1.61(1H,m),1.73-1.93(3H,m),2.14-2.25(1H,m),2.75(3H,s),2.98-3.15(1H,m),4.34(1H,t,*J*=7.1Hz),7.03(1H,s),7.2 (1H,s). | 328 (M+H)⁺ 326 (M-H)⁻ |
| Reference Example 32 | 81.71-1.95(5H,m),2.14-2.21(1H,m),2.76(3H,s),2.98-3.04(1H,m),3.11-3.17(1H,m),4.34(1H,d,*J*=7.3Hz),6.96(1H,d,J=3.7Hz),7.10(1H,s),7.3 1(1H,d,*J*=3.7Hz). | 294 (M+H)⁺ 292 (M-H)⁻ |
| Reference Example 33 | 1.70-1.96(3H,m),2.16-2.26(1H,m),2,78(3H,s),3.00-3.07(1H,m),3,13-3.16(1H,m),4.37(1H,t,*J*=7.0Hz),7.13(1H,s),8.28(1H,s). | 295 (M+H)⁺ 293 (M-H)⁻ |
| Reference Example 34 | 1.35(3H,t,*J*=7.5Hz),1.72-1.98(3H,m),2.05-2.25(1H,m),2.77(3H,s),2. 83-2.89(2H,m),3.00-3.08(1H,m),3.11-3.17(1H,m),4.35(1H,t,*J*=7.1Hz ),7.06(1H,s). | 323 (M+H)⁺ 321 (M-H)⁻ |
| Reference Example 35 | 1.72-1.98(4H,m),2.16-2.23(1H,m),2.66(3H,s),2.76(3H,s),3.00-3.08(1 H,m),3.11-3.18(1H,m),4.35(1H,t,*J*=7.0Hz),7.07(1H,s). | 341 (M+H)⁺ 339 (M-H)⁻ |
| Reference Example 36 | 1.72-2.27(5H,m),2.76(3H,s),3.01-3.06(1H,m),3.11-3.18(1H,m),4.04( 3H,s),4.37(1H,t,*J*=7.1Hz),7.08(1H,s). | 325 (M+H)⁺ 323 (M-H)⁻ |

### Reference Example 37

### 5-[(2S,4R)-4-Fluoropyrrolidin-2-yl]-2-methylthiophene-3-car boxylic acid [1,2,4]thiazole-5-ylamide

The title compound was obtained by conducting a reaction according to a similar method to that of Reference Example 23 by using (2*S*,4*R*)-4-fluoro-2-[5-methyl1-4-([1,2,4]thiadiazol-5-ylcarb amoyl)-thiophen-2-yl]-pyrrolidine-1-carboxylic acid *t*-butyl ester instead of (-)-2-"4-(5-fluorothiazol-2-ylcarbamoyl)-5-methylthiophen-2 -yl"pyrrolidine-1-carboxylic acid *t*-butyl ester.
ESI/MS(m/z): 313(M+H)⁺, 311(M-H)⁻.

### Reference Example 38

### Ethylaminoacetic acid benzyl ester hydrochloride

*t*-Butoxycarbonylethylaminoacetic acid (152 g) was dissolved in acetone (750 mL), potassium carbonate (156 g), benzyl chloride (94.7 mL) and sodium iodide (124 g) were added thereto, and stirring was conducted at 60°C for 4.5 hours. The reaction liquid was diluted with ethyl acetate, insoluble substances were filtered off, and the filtrate was concentrated under a reduced pressure. The obtained residue was diluted with ethyl acetate and washed twice with saturated brine. The organic layer was dried over anhydrous sodium sulfate and concentrated under a reduced pressure. The obtained residue (234 g) was dissolved in a 4 N hydrochloric acid/ethyl acetate solution (1.2 L), and stirring was conducted at room temperature for 1.5 hours. Diethyl ether was added to the reaction liquid, and the precipitated solid was collected by filtration and washed with diethyl ether to give the title compound (157 g).
¹H-NMR(CDCl₃)δ(ppm): 1.45-1.49(3H,m), 3.18(2H,br.s), 3.86(2H,b r.s), 5.21(2H,s), 7.32-7.36(5H,m), 9.92(2H,br.s).

### Reference Example 39

### (S)-2-Aminobutanoic acid benzyl ester

(S)-2-t-Butoxycarbonylaminobutanoic acid (2.0 g) was dissolved in N,N-dimethylformamide (20 mL), potassium carbonate (2.72 g) was added thereto, and benzyl bromide (1.29 mL) was added dropwise thereto. Stirring was conducted at room temperature for 5 hours, the reaction liquid was extracted with ethyl acetate and a saturated aqueous sodium hydrogen carbonate solution, and the organic layer was dried over anhydrous sodium sulfate. Concentration was conducted under a reduced pressure, and the obtained residue was purified by silica gel chromatography (hexane/ethyl acetate=4/1) to give (*S*)-2-*t*-butoxycarbonylaminobutanoic acid benzyl ester (2.80 g). A 4 N hydrochloric acid/ethyl acetate solution (5.1 mL) was added to the obtained (*S*)-2-*t*-butoxycarbonylaminobutanoic acid benzyl ester (1.0 g), and stirring was conducted at room temperature for 3 hours. Concentration was conducted under a reduced pressure, the concentrate was made basic with a 1 N aqueous sodium hydroxide solution and extracted with diethyl ether, and the organic layer was dried over anhydrous sodium sulfate. Concentration was conducted under a reduced pressure to give the title compound (637 mg) as a colorless oil.
¹H-NMR(CDCl₃)δ(ppm): 0.94(3H,t,*J*=7.6Hz), 1.56(2H,br.s), 1.59-1 .69(1H,m), 1.73-1.84(1H,m), 3.45(1H,t,*J*=6.4Hz), 5.16(2H,s), 7.3 1-7.39 (5H,m) .
ESI/MS(m/z) :194(M+H)⁺.

### Reference Example 40

### (R)-2-Aminobutanoic acid benzyl ester

The title compound was obtained by conducting a reaction according to a similar method to that of Reference Example 39 by using (*R*)-2-*t*-butoxycarbonylaminobutanoic acid instead of (*S*)-2-*t*-butoxycarbonylamznobutanoic acid.
¹H-NMR(CDCl₃)δ(ppm) :
0.94(3H,t,*J*=7.6Hz), 1.56(2H,br.s), 1.59-1.69(1H,m), 1.73-1.84 ( 1H,m), 3.45(1H,t,*J*=6.4Hz), 5.16 (2H,s), 7.31-7.39(5H,m). ESI/MS(m/z): 194(M+H)⁺.

### Reference Example 41

### (R)-2-aminopropanoic acid benzyl ester

The title compound was obtained by conducting a reaction according to a similar method to that of Reference Example 39 by using (*R*)-2-*t*-butoxycarbonylaminopropanoic acid instead of (*S*)-2-*t*-butoxycarbonylaminobutanoic acid.
¹H-NMR(CDCl₃)δ(ppm): 1.44(3H,d,*J*=77.0Hz), 4.10-4.21(1H,m), 5.25 (2H,s), 7.32-7.49(5H,m), 8.55(1H,br.s).

### Reference Example 42

### (S)-2-Ethylaminobutanoic acid benzyl ester

(*S*)-2-Aminobutanoic acid benzyl ester (520 mg) and acetaldehyde (166 µL) were dissolved in methanol (5 mL) , sodium cyanoborohydride (169 mg) was added thereto, and stirring was conducted at room temperature for 2 hours. A 1 N aqueous sodium hydroxide solution was added thereto, extraction was conducted by using diethyl ether, and the organic layer was dried over anhydrous sodium hydrogen sulfate. Concentration was conducted under a reduced pressure, and the obtained residue was purified with NH-silica gel chromatography (hexane/ethyl acetate=2/1) to give the title compound (200 mg) as a colorless oil.
¹H-NMR(CDCl₃)δ(ppm): 0. 90(3H,t,*J*=7.6Hz), 1.08(3H,t,*J*=6.8Hz), 1 .46(1H,br.s), 1.64-1.72(2H,m), 2.47-2.55(1H,m), 2.59-2.67(1H,m ), 3.25(1H,t,*J*=6.4Hz), 5.17(2H,s), 7.30-7.39(5H,m). ESI/MS(m/z) : 222(M+H)⁺.

### Reference Example 43

### (R)-2-Ethylaminobutanoic acid benzyl ester

The title compound was obtained by conducting a reaction according to a similar method to that of Reference Example 41 by using (*R*)-2-aminobutanoic acid benzyl ester instead of (*S*)-2-aminobutanoic acid benzyl ester.
¹H-NMR(CDCl₃)δ(ppm): 0.90(3H,t,*J*=7.6Hz), 1.08(3H,t,*J=*7.2Hz), 1 .64-1.72(2H,m), 2.47-2.55(1H,m), 2.59-2.671(1H,m), 3.25(1H,t,*J*= 6,4Hz), 5.17(2H,s), 7.31-7.39(5H,m). ESI/MS(m/z): 222(M+H)⁺.

### Reference Example 44

### (S)-2-Ethylaminopropanoic acid benzyl ester hydrochloride

(S) -2-Aminopropanoic acid benzyl ester hydrochloride (1. 00 g) was dissolved in trimethyl orthoformate (20.0 mL), *N*,*N*-diisopropylethylamine (798 µL) was added thereto, and stirring was conducted at room temperature for 5 minutes. Acetaldehyde (2.64 mL) was then added thereto, and stirring was conducted at room temperature for 5 minutes. Thereafter the solution was cooled under ice-cooling, sodium borohydride (704 mg) was added thereto, and stirring was conducted at room temperature for 1 hour. Water was added to the reaction liquid, extraction was then conducted by using dichloromethane, and the organic layer was dried over anhydrous sodium sulfate and concentrated under a reduced pressure. A 4 N hydrochloric acid/ethyl acetate solution was added to the the obtained residue under ice-cooling, concentration was conducted under a reduced pressure, and diethyl ether was added to the residue to solidify the residue. The precipitated product was collected by filtration, washed with diethyl ether and dried under a reduced pressure to give the title compound (1.04 g) . ¹H-NMR(CDCl₃)δ(ppm): 1.22(3H,t,*J*=7.0Hz), 1.48(3H,d,*J*=7.0Hz), 2 .88-3.09(2H,br.s), 4.18-4.29(1H,m), 5.28(2H,s), 7.36-7.51(5H,m ), 9.09-9.23(1H,br.s), 9.33-9.49(1H,br.s).

### Reference Example 45

### (R)-2-Ethylaminopropanoic acid benzyl ester hydrochloride.

The title compound was obtained by conducting a reaction according to a similar method to that of Reference Example 44 by using (*R*)-2-aminopropanoic acid benzyl ester hydrochloride instead of (*S*)-2-aminopropanoic acid benzyl ester hydrochloride.
¹H-NMR(CDCl₃)δ(ppm): 1.22(3H,t,*J*=7.1Hz), 1.48(3H,d,*J*=7.3Hz), 2 .89-3.08(2H,m), 4.18-4.29(1H,m), 5.28(2H,s), 7.34-7.49(5H,m), 9 .02-9.40(2H,m).

### Reference Example 46

### (S)-2-Ethylaminopentanoic acid benzyl ester hydrochloride

Methanol (22 mL) was added to (*S*) -2-ethylaminopentanoic acid benzyl ester hydrochloride (1.12 g) . Acetaldehyde (906 µL) was added thereto while cooling under ice-cooling, and stirring was conducted at the same temperature for 30 minutes. Sodium borohydride (245 mg) was added thereto under ice-cooling, and stirring was conducted at the same temperature for 45 minutes. Water was added to the reaction liquid, and extraction was conducted by using chloroform. The organic phase was dried over anhydrous sodium sulfate and concentrated under a reduced pressure. A 4 N hydrochloric acid/ethyl acetate solution (60 mL) was added to the obtained residue under ice-cooling, and stirring was conducted at the same temperature for 1 hour. Concentration was conducted under a reduced pressure, and diethyl ether was added to the residue to solidify the residue. The precipitated product was collected by filtration, washed with diethyl ether and dried under a reduced pressure to give the title compound (1.33 g).
¹H-NMR(CDCl₃)δ(ppm): 0.90(3H,t,*J*=7.3Hz), 1.25-1.55(2H,m), 1. 47 (3H,t,*J*=7.2Hz), 2.05-2.25(2H,m), 3.04(2H,br.d), 3.80(1H,br.s), 5.20-5.35(2H,m), 7.3-7.45(5H,m).

### Reference Example 47

### (R)-2-Ethylaminopentanoic acid benzyl ester hydrochloride

The title compound was obtained by conducting a reaction according to a similar method to that of Reference Example 46 by using (*R*)-2-ethylaminopentanoic acid benzyl ester hydrochloride instead of (*S*)-2-ethylaminopentanoic acid benzyl ester hydrochloride.
¹H-NMR(CDCl₃) δ(ppm): 0.90(3H,t,*J*=7.3Hz), 1.25-1.55(2H,m), 1.47 (3H,t,*J*=7.2Hz), 2.05-2.25(2H,m), 3.04(2H,br.d), 3.80(1H,br.s), 5.20-5.35(2H,m), 7.3-7.45(5H,m).

### Reference Example 48

### (S)-2-Ethylamino-3-methylbutanoic acid benzyl ester hydrochloride

(*S*)-2-Amino-3-methylbutanoic acid benzyl ester hydrochloride (1.00 g) and diisopropylethylamine (715 uL) were dissolved in methanol (30 mL) and cooled with ice. Acetaldehyde (2.3 mL) was added thereto, stirring was conducted for 15 minutes under ice-cooling, sodium borohydrate (620 mg) was then added thereto, and stirring was continued for 1 hour. Water was added to the reaction liquid, extraction was conducted by using diethyl ether, and the organic layer was washed with water and saturated brine and dried over anhydrous sodium sulfate. Concentration was conducted under a reduced pressure, the obtained residue was dissolved in ethyl acetate (10 mL), a 4 N hydrochloric acid/ethyl acetate solution was added thereto, and concentration was conducted again under a reduced pressure. The obtained solid was suspended in diethyl ether, and this was collected by filtration and dried under a reduced pressure to give the title compound (1.01 g) as a white powder.
¹H-NMR(DMSO-d₆) δ(ppm): 0.89(3H,d,*J*=7.3Hz), 1.01(3H,d,*J*=7.0Hz) ,1.23(3H,t,*J*=7.0Hz), 2.32-2.40(1H,m), 2.98(2H,br.s), 4.04(1H,b r.s), 5.25(1H,d,*J*=12.1Hz), 5.32(1H,d,*J*=12.1Hz), 7.35-7.46(5H,m ), 9.10(1H,br.s), 9.37(1H,br.s).
ESI/MS (m/z): 236(M+H)⁺(free form).

### Reference Example 49

### (R)-2-Ethylamino-3-methylbutanoic acid benzyl ester hydrochloride

The title compound was obtained by conducting a reaction according to a similar method to that of Reference Example 47 by using (*R*)-2-amino-3-methylbutanoic acid benzyl ester hydrochloride instead of (*S*)-2-amino-3-methylbutanoic acid benzyl ester hydrochloride.
¹H-NMR(DMSO-d₆) δ(ppm): 0.89(3H,d,*J*=7.0Hz), 1.01(3H,d,*J*=7.0Hz) ,1.23(3H,t,*J*=7.3Hz), 2.34-2.40(1H,m), 2.97(2H,br.s), 4.03(1H,b r.s), 5.24(1H,d,*J*=12.1Hz), 5.32(1H,d,*J*=12.1Hz), 7.36-7.45(5H,m ), 9.12(1H,br.s), 9.47(1H,br.s).
ESI/MS (m/z): 236(M+H)⁺(free form).

### Reference Example 50

### (S)-2-(2-t-Butoxycarbanylethylamino)propanoic acid benzyl ester

(*S*)-2-Aminopropanoic acid benzyl ester hydrochloride (1.00 g) was dissolved in tetrahydrofuran (20 mL), potassium carbonate (3.2 g) was added thereto, and stirring was conducted for 1 hour. The reaction liquid was filtered and concentrated under a reduced pressure, *t*-butyl acrylate (20 mL) was added thereto, and stirring was conducted at 60°C for 62 hours. The reaction liquid was concentrated under a reduced pressure, and the obtained residue was purified by silica gel chromatography (hexane/ethyl acetate=1/1) to give the title compound (832 mg) as a colorless oily substance.
¹H-NMR(CDCl₃) δ(ppm): 1.31(3H,d,*J*=7.0Hz), 1.32-1.51(9H,m), 2.40 (2H,t,*J*=6.6Hz), 2.68-2.75(1H,m), 2.82-2.91(1H,m), 3.36-3.44(1H ,m), 5.17(2H,d,*J*=2.2Hz), 7.28-7.41(5H,m).
ESI/MS (m/z): 308(M+H)⁺.

### Reference Example 51

### (R)-2-(2-t-Butoxycarbonylethylamino)propanoic acid benzyl ester

The title compound was obtained by conducting a reaction according to a similar method to that of Reference Example 50 by using (*R*)-2-aminopropanoic acid benzyl ester hydrochloride instead of (*S*)-2-aminopropanoic acid benzyl ester hydrochloride.
¹H-NMR(CDCl₃) δ(ppm): 1.31(3H,d,*J*=7.0Hz), 1.32-1.50(9H,m), 2.40 (2H,t,*J*=6.8Hz), 2.66-2.75(1H,m), 2.80-2.90(1H,m), 3.35-3.42(1H ,m), 5.17(2H,d,*J*=2.2Hz), 7.28-7.41(5H,m).
ESI/MS (m/z): 308(M+H)⁺.

### Reference Example 52

### (3-Benzyloxycarbonylmethylethylamino) propanoic acid t-butyl ester

*t*-Butyl acrylate (1.46 mL) was dissolved in tetrahydrofuran (10 mL), a 2 N ethylamine/tetrahydrofuran solution (25 mL) was added thereto at room temperature, and stirring was conducted overnight under reflux. The reaction liquid was cooled to room temperature, concentrated under a reduced pressure, the residue (346.5 mg) was dissolved in tetrahydrofuran (2 mL) and triethylamine (557 µL), bromoacetic acid benzyl ester (377 µL) was added thereto at room temperature, and stirring was conducted for 12 hours. The reactant was diluted with water and extracted with diethyl ether. The extract was washed three times with an aqueous saturated ammonium chloride solution and dried over anhydrous sodium sulfate. Concentration was conducted under a reduced pressure, and the residue was purified by thin layer silica gel chromatography (ethyl acetate/hexane=1/5) to give the title compound (559 mg) as a colorless oil.
¹H-NMR(CDCl₃) δ(ppm): 1.03(3H,q,*J*=7.8Hz) 1.43(9H,s), 2.38(2H,t ,*J*=7.3Hz), 2.65-2.73(2H,m), 2.92(2H,t,*J*=7.3Hz), 3.41(2H,s), 5.1 4(2H,s), 7.32-7.37(5H,m).

### Reference Example 53

### (3-Benzyloxycarbonyl-z-prapylamino)propanoic acid t-butyl ester

The title compound was obtained by conducting a reaction according to a similar method to that of Reference Example 52 by using isopropylamine instead of the 2 N ethylamine/tetrahydrofuran solution.
¹H-NMR(CDCl₃) δ(ppm): 0.98-1.02(6H,m), 1.43(9H,s), 2.37-2.41(2H ,m), 2.87(2H,t,*J*=7.3Hz), 2.99-3.03(1H,m), 3.35(2H,s), 5.14(2H,s ),7.30-7.38(5H,m).

### Reference Example 54

### (t-Butoxycarbonylmethylethylamino)acetic acid benzyl ester

Ethylaminoacetic acid benzyl ester hydrochloride (157 g) was suspended in tetrahydrofuran (1.0 L), triethylamine (286 mL) was added thereto at room temperature, and stirring was conducted for 5 minutes. Bromoacetic acid *t*-butyl ester (120 mL) was further added thereto, and stirring was conducted at room temperature for 22 hours. Additional bromoacetic acid *t*-butyl ester (18.4 mL) was added thereto, and stirring was conducted at room temperature for 42 hours. The reaction liquid was diluted with ethyl acetate, the insoluble substances were filtered off, and the filtrate was concentrated under a reduced pressure. The obtained residue was diluted with ethyl acetate and washed with saturated brine. The organic layer was dried over anhydrous sodium sulfate and concentrated under a reduced pressure. The obtained residue was purified by silica gel column chromatography (hexane/ethyl acetate=6/1-5/1) to give the title compound (180 g).
¹H-NMR(CDCl₃) δ(ppm): 1.05-1.11(3H,m), 1.45(9H,s), 2.74-2.81(2H ,m), 3.45(2H,s), 3.61(2H,s), 5.15(2H,s), 7.33-7.36(5H,m).

### Reference Example 55

### (S)-2-(t-Butoxycarbonylmethylethylamino) butanoic acid benzyl ester

(*S*)-2-Ethylaminobutanoic acid benzyl ester (200 mg), bromoacetic acid *t*-butyl ester (352 mg), potassium carbonate (250 mg) and sodium iodide (136 mg) were dissolved in *N,N*-dimethylformamide (4 mL). Stirring was conducted at room temperature for 15 hours and extracted with water and diethyl ether, and the organic layer was dried over anhydrous sodium sulfate. Concentration was conducted under a reduced pressure, and the obtained residue was purified by silica gel chromatography (hexane/ethyl acetate=5/1) to give the title compound (250 mg) as a colorless oil.
¹H-NMR(CDCl₃) δ(ppm): 0.95(3H,t,*J*=7.6Hz), 1.04(3H,t,*J*=7.2Hz), 1 .44(9H,s), 1.61-1.82(2H,m), 2.61-2.79(2H,m), 3.22(1H,d,*J*=17.6H z), 3.40(1H,t,*J*=7.2Hz), 3.45(1H,d,*J*=17.6Hz), 5.13(2H,s), 7.30-7 .40(5H,m).
ESI/MS (m/z): 336(M+H)⁺.

### Reference Example 56

### (R)-2-(t-Butoxycarbonylmethylethylamino) butanoic acid benzyl ester

The title compound was obtained by conducting a reaction according to a similar method to that of Reference Example 55 by using (*R*)-2-ethylaminobutanoic acid benzyl ester instead of (*S*)-2-ethylaminobutanoic acid benzyl ester.
¹H-NMR(CDCl₃) δ(ppm): 0.95(3H,t,*J*=7.6Hz), 1.04(3H,t,*J*=7.2Hz), 1 .44(9H,s), 1.61-1.82(2H,m), 2.61-2.79(2H,m), 3.22(2H,d,*J*=17.2H z), 3.39(1H,t,*J*=7.6Hz), 3.45(1H,d,*J*=17.2Hz), 5.13(2H,s), 7.30-7 .39(5H,m).
ESI/MS (m/z): 336(M+H)⁺.

### Reference Example 57

### (S)-2-(t-Butoxycarbonylmethylethylamino)-3-methylbutanoic acid benzyl ester

The title compound was obtained by conducting a reaction according to a similar method to that of Reference Example 55 by using (*S*)-2-ethylamino-3-methylbutanoic acid benzyl ester hydrochloride instead of (*S*)-2-ethylaminobutanoic acid benzyl ester.
¹H-NMR(CDCl₃) δ(ppm): 0.84(3H,d,*J*=6.6Hz), 1.01(3H,d,*J*=6.6Hz), 1 .03((3H,t,*J*=7.3Hz), 1.43(9H,s), 1.98(1H,dq,*J*=10.6,6.6Hz), 2.59( 1H,dt,*J*=12.8,7.0Hz), 2.75(1H,dt,*J*=12.8,7.3Hz), 2.96(1H,d,*J*=10 .6Hz), 3.12(1H,d,*J*=17.2Hz), 3.46(1H,d,*J*=17.2Hz), 5.13(2H,s), 7. 32-7.37(5H,m).
ESI/MS (m/z): 350(M+H)⁺.

### Reference Example 58

### (R)-2-(t-Butoxycarbonylmethylethylamino)-3-methylbutanoic acid benzyl ester

The title compound was obtained by conducting a reaction according to a similar method to that of Reference Example 55 by using (*R*)-2-ethylamino-3-methylbutanoic acid benzyl ester hydrochloride instead of (*S*)-2-ethylaminobutanoic acid benzyl ester.
¹H-NMR(CDCl₃) δ(ppm): 0.84(3H,d,*J*=6.2Hz), 1.01(3H,d,*J*=7.0Hz), 1 .03(3H,t,*J*=7.3Hz), 1.43(9H,s), 1.99(1H,dq,*J*=10.6,6.6Hz), 2.60( 1H,dt,*J*=13.2,7.0Hz), 2.75(1H,dt,*J*=13.2,7.3Hz), 2.96(1H,d,*J*=10 .6Hz), 3.12(1H,d,*J*=17.2Hz), 3.46(1H,d,J=17.2Hz), 5.13(2H,s), 7. 31-7.37(5H,m).
ESI/MS (m/z): 350(M+H)⁺.

### Reference Example 59

### (S)-2-(t-Butoxycarbonylmethylethylamino)propanoic acid benzyl ester

*N,N*-Dimethylformamide (4 mL), *t*-butyl bromoacetate (481 µL) and triethylamine (571 µL) were added to (*S*)-2-ethylaminopropanoic acid benzyl ester hydrochloride (400 mg), and stirring was conducted at room temperature for 22 hours. Water was added to the reaction liquid, extraction was conducted by using ethyl acetate, and the organic layer was washed with a saturated aqueous sodium hydrogen carbonate solution, dried over anhydrous sodium sulfate and concentrated under a reduced pressure. The obtained residue was purified by silica gel chromatography (hexane/ethylacetate=7/1) to give the title compound (169 g).
¹H-NMR(CDCl₃) δ(ppm): 1.06(3H,t,*J*=7.1Hz), 1.33(3H,d,*J*=7.3Hz), 1 .44(9H,s), 2.6-2.8(2H,m), 3.28-3.48(2H,m), 3.69-3.74(1H,m), 5.1 3(2H,s), 7.25-7.4(5H,m).

### Reference Example 60

### (R)-2-(t-Butoxycarbonylmethylethylamino)propanoic acid benzyl ester

The title compound was obtained by conducting a reaction according to a similar method to that of Reference Example 59 by using (*R*)-2-ethylaminopropanoic acid benzyl ester hydrochloride instead of (*S*)-2-ethylaminopropanoic acid benzyl ester hydrochloride.
¹H-NMR(CDCl₃) δ(ppm): 1.06(3H,t,*J*=7.3Hz), 1.33(3H,d,*J*=7.3Hz), 1 .44(9H,s), 2.6-2.8(2H,m), 3.27-3.48(2H,m), 3.69-3.74(1H,m), 5.1 3(2H,s), 7.25-7.4(5H,m).

### Reference Example 61

### (S)-2-(t-Butoxycarbonylmethylethylamino)pentanoic acid benzyl ester

The title compound was obtained by conducting a reaction according to a similar method to that of Reference Example 59 by using (*S*)-2-ethylaminopentanoic acid benzyl ester hydrochloride instead of (*S*)-2-ethylaminopropanoic acid benzyl ester hydrochloride.
¹H-NMR(CDCl₃) δ(ppm): 0.90(3H,t,*J*=7.5Hz), 1.04(3H,t,*J*=7.1Hz), 1 .25-1.55(2H,m), 1.44(9H,s), 1.55-1.75(2H,m), 2.6-2.8(2H,m), 3.2 0-3.48(2H,m), 3.48(1H,t,*J*=7.3Hz), 5.13(2H,s), 7.3-7.4(5H,m).

### Reference Example 62

### (R)-2-(t-Butoxycarbonylmethylethylamino)pentanoic acid benzyl ester

The title compound was obtained by conducting a reaction according to a similar method to that of Reference Example 59 by using (*R*)-2-ethylaminopentanoic acid benzyl ester hydrochloride instead of (*S*)-2-ethylaminopropanoic acid benzyl ester hydrochloride.
¹H-NMR(CDCl₃) δ(ppm): 0.90(3H,t,*J*=7*.*3Hz), 1.04(3H,t,*J*=7.1Hz), .25-1.5(2H,m), 1.44(9H,s), 1.55-1.75(2H,m), 2.6-2.8(2H,m), 3.20 -3.48(2H,m), 3.48(1H,t,*J*=7.5Hz), 5.13(2H,s), 7.25-7.4(5H,m).

### Reference Example 63

### (S)-2-(t-Butoxycarbonylethylethylamino)propanoic acid benzyl ester

(*S*)-2-(2-*t*-Butoxycarbonylethylamino)propanoic acid benzyl ester (782 mg) was dissolved in trimethyl orthoformate (15.6 mL), acetaldehyde (1.43 mL) and triacetoxysodium borohydride (2.67 g) were added thereto, and stirring was conducted at room temperature for 20 hours. A saturated aqueous sodium hydrogen carbonate solution was added to the reaction liquid, extraction was conducted by using methylene chloride, and the organic layer was dried over anhydrous sodium sulfate. Concentration was conducted under a reduced pressure, and the obtained residue was purified by thin layer silica gel chromatography (hexane/ethyl acetate=4/1) to give the title compound (284 mg) as a colorless oily substance.
¹H-NMR(CDCl₃) δ(ppm): 1.02(3H,t,*J*=7.1Hz), 1.30(3H,d,*J*=7.3Hz), .44(9H,s), 2.34(2H,t,*J*=7.1Hz), 2.49-2.58(1H,m), 2.62-2.73(1H,m ), 2.76-2.85(1H,m), 2.92-3.02(1H,m), 3.58(1H,q,*J*=7.2Hz), 5.14(2 H,s), 7.27-7.41(5H,m).
ESI/MS (m/z): 336(M+H)⁺.

### Reference Example 64

### (R)-2-(t-Butoxycarbonylethylethylamino)propanoic acid benzyl ester

The title compound was obtained by conducting a reaction according to a similar method to that of Reference Example 63 by using (*R*)-2-(2-*t*-butoxycarbonylethylamino)propanoic acid benzyl ester instead of (*S*)-2-(2-*t*-butoxycarbonylethylamino)propanoic acid benzyl ester.
¹H-NMR(CDCl₃) δ(ppm): 1.01(3H,t,*J*=7.1Hz), 1.29(3H,d,*J*=7.0Hz), 1 .43(9H,s), 2.33(2H,t,*J*=7.1Hz), 2.47-2.58(1H,m), 2.61-2.72(1H,m ), 2.75-2.85(1H,m), 2.90-3.02(1H,m), 3.57(1H,q,*J*=7.0Hz), 5.12(2 H,s),7.21-7.39(5H,m).
ESI/MS (m/z): 336(M+H)⁺.

### Reference Example 65

### (Benzyloxycarbonylmethylamino)acetic acid t-butyl ester

Aminoacetic acid *t*-butyl ester (1.26 g) and triethylamine (2. 09 mL) were dissolved in acetonitrile (10 mL), stirring was conducted for 5 minutes, bromoacetic acid benzyl ester (784 µL) was added thereto at room temperature, and stirring was conducted for 17 hours. The reactant was diluted with a saturated aqueous ammonium chloride solution and extracted with ethyl acetate. The extract was washed three times with a saturated aqueous ammonium chloride solution and dried over anhydrous sodium sulfate. Concentration was conducted under a reduced pressure, and the residue was purified by silica gel chromatography (ethyl acetate/hexane=1/3) to give the title compound (984 mg) as a colorless oil.
¹H-NMR(CDCl₃) δ(ppm): 0.87-0.89(6H,m), 1.45(9H,s), 1.64-1.75(1H ,m), 2.47(2H,d,*J*=7.3Hz), 3.42(2H,s), 3.58(2H,s), 5.14(2H,s), 7.3 0-7.36(5H,m).

### Reference Example 66

### (t-Butoxycarbonylmethylethylamino)acetic acid

(*t*-Butoxycarbonylmethylethylamino)acetic acid benzyl ester (167 g) was dissolved in ethanol (500 mL), 10%-Pd/C (16.7 g) was added thereto at room temperature under an argon atmosphere, the reaction container was subjected to replacement by hydrogen, and stirring was conducted at room temperature for 24 hours. The catalyst was filtered off with a Celite pad and washed with ethanol. The filtrate was concentrated under a reduced pressure to give the title compound (116 g) as a white solid. ¹H-NMR(CDCl₃) δ(ppm): 1.09-1.14(3H,m), 1.47(9H,s), 2.79-2.84(2H ,m), 3.36(2H,s), 3.41(2H,s), 9.35(1H,br.s).

Compounds were synthesized according to the following reaction formula with referring to the method of Reference Example 66. The synthesized compound are shown in Table 3, and the data are shown in Table 4.

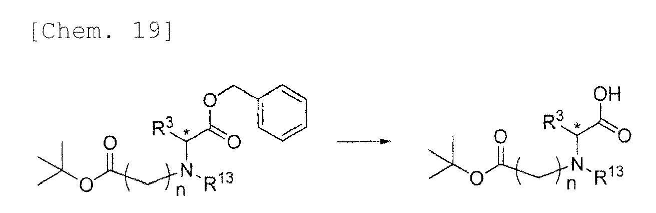

**[Table 3]**

| Reference Example | Starting raw material | R³ | R¹³ n | |
|---|---|---|---|---|
| Reference Example 67 | Reference Example 52 | H | Et | 2 |
| Reference Example 68 | Reference Example 53 | H | *i*-Pr | 2 |
| Reference Example 69 | Reference Example 55 | Et | Et | 1 |
| Reference Example 70 | Reference Example 56 | Et | Et | 1 |
| Reference Example 71 | Reference Example 57 | *i*-Pr | Et | 1 |
| Reference Example 72 | Reference Example 58 | *i*-Pr | Et | 1 |
| Reference Example 73 | Reference Example 59 | Me | Et | 1 |
| Reference Example 74 | Reference Example 60 | Me | Et | 1 |
| Reference Example 75 | Reference Example 61 | *n*-pr | Et | 1 |
| Reference Example 76 | Reference Example 62 | *n*-Pr | Et | 1 |
| Reference Example 77 | Reference Example 63 | Me | Et | 2 |
| Reference Example 78 | Reference Example 64 | Me | Et | 2 |

**[Table 4]**

| Reference Example | ¹H-NMR(CDCl₃)δ(ppm) |
|---|---|
| Reference Example 67 | 1.20(3H,t,*J*=7.1Hz);1.47(9H,s),2.57(2H,t,*J*=7.0Hz),2.86-2.94(2H,m),3.09(2H,t,*J*=7.0 Hz),3.29-3.40(2H,m). |
| Reference Example 68 | 1.21-1.25(6H,m),1.46(9H,s),2.64(2H,t,*J*=6.4Hz),3.12(2H,t,*J*=6.6Hz),3.31-3.36(2H,m) ,3.40-3.45(1H,m),6.20(1H,br.s). |
| Reference Example 69 | 1.04(3H,t,*J*=7.6Hz),1.08(3H,t,*J*=7.2Hz),1.49(9H,s),1.61-1.72(1H,m),1.9-2.02(1H,m) ,2.68-2.76(2H,m),3.21-3.24(1H,m),3.33-3.45(2H,m). |
| Reference Example 70 | 1.04(3H,t,*J*=7.6Hz),1.08(3H,t,*J*=7.2Hz),1.49(9H,s),1.61-1.72(1H,m),1.92-2.03(1H.m) ,2.68-2.76(2H,m),3.21-3.24(1H.m),3.33-3.45(2H,m). |
| Reference Example 71 | 1.01(3H,d,*J*=6.2Hz),1.02(3H,d,*J*=7.0Hz),1.05(3H,t,*J*=7.3Hz),1.50(9H,s),2.11(1H,dq,*J* =8.1,7.0Hz),2.74(2H,q,*J*=7.3Hz),3.05(1H,d,*J*=8.1Hz),3.44(2H,s). |
| Reference Example 72 | 1.00(3H,d,*J*=6.6Hz),1.03(3H,d,*J*=7.0Hz),1.06(3H,t,*J*=7.3Hz),1.48(9H,s),2.08(1H,dq,*J* =8.1,7.0Hz),2.75(2H,q,*J*=7.0Hz),3.04(1H,d,*J*=8.4Hz),3.34(1H,d,*J*=17.6Hz),3.46(1H,d ,*J*=17.6Hz). |
| Reference Example 73 | 0.96(3H,t,*J*=7.2Hz),1.16(3H,d,*J*=7.0Hz),1.40(9H,s),2.55-2.7(2H,m),3.20-3.38(2H,m), 3.4-3.55(1H,m). |
| Reference Example 74 | 0.96(3H,t,*J*=7.2Hz),1.15(3 H,d,*J*=7.3Hz),1.40(9H,s),2.55-2.7(2H,m),3.20-3.39(2H,m), 3.42-3.47(1H,m). |
| Reference Example 75 | 0.87(3H,t,*J*=7.3Hz),0.96(3H,t,*J*=7.1Hz),1.2-1.6(4H,m),1.40(9H,s),1.6-1.8(2H,m),2.55 -2.7(2H,m),3.14-3.34(2H,m),3.2-3.3(1H,m). |
| Reference Example 76 | 0.87(3H.t,*J*=7.3Hz),0.96(3H,t,*J*=7.1Hz),1.2-1.6(4H,m),1.40(9H,s),1.6-1.8(2H,m),2.55 -2.7(2H,m),3.13-3.39(2H,m),3.2-3.3(1H,m). |
| Reference Example 77 | 1.20(3H,t,*J*=7.1Hz),1.37(3H,d,*J*=7.3Hz),1.41-1.51(9H,m),2.39-2.93(5H,m),3.01-3.12( 1 H,m),3.56(1H,q,*J*=7.1Hz). |
| Reference Example 78 | 1.16(3H,t,*J*=7.1Hz),1.34(3H,d,*J*=7.0Hz),1.39-1.50(9H,m),2.37-2.90(5H,m),2.96-3.06(1H,m),3.44-3.58(1H,m). |

### Reference Example 79

### (t-Butoxycarbonylmethyl-i-butylamino)acetic acid

(Benzyloxycarbonylmethylamino)acetic acid *t*-butyl ester (140 mg) and isobutylaldehyde (137 µL) were dissolved in ethanol (2 mL), 10%-Pd/C (14 mg) was added thereto, and stirring was conducted under a hydrogen atmosphere at 50°C for 4 hours. The catalyst was filtered off with a Celite pad and washed with ethanol. The filtrate was concentrated under a reduced pressure to give the title compound (89 mg) as a white solid. ¹H-NMR(CDCl₃) δ(ppm): 0.91-0.96(6H,m), 1.48(9H,s), 1.67-1.79(1H ,m), 2.47(2H,d,*J*=7.3Hz), 3.35(4H,s).

### Reference Example 80

### (t-Butoxycarbonylpropylamino)acetic acid

Under an argon atmosphere, sodium hydrate (365 mg) was suspended in tetrahydrofuran (5 mL), and the suspension was cooled with ice. A solution of *t*-butoxycarbonylaminoacetic acid (400 mg) and 1-iodopropane (892 µL) in tetrahydrofuran (3 mL) was added dropwise thereto. The reaction liquid was returned to room temperature and stirred for 96 hours. Sodium hydrate (91.2 mg) and 1-iodopropane (223 µL) were further added to the reaction liquid, and stirring was conducted at 40°C for 67 hours. The reaction liquid was extracted by adding water and ethyl acetate, and the aqueous layer was adjusted to pH=2-3 with a 10% aqueous citric acid solution. The aqueous layer was extracted with ethyl acetate, and the organic layer was dried over anhydrous sodium sulfate. Concentration was conducted under a reduced pressure to give the title compound (210 mg) as an oily substance.
¹H-NMR(CDCl₃) δ(ppm): 0.89(3H,br.t,*J*=7.3Hz), 1.44-1.57(11H,m), 3.21-3.28(2H,m), 3.95(2H,d,*J*=21.6Hz).
ESI/MS (m/z): 216(M-H)⁻.

### Reference Example 81

### (t-Butoxycarbonylpentylamino)acetic acid

The title compound was obtained by conducting a reaction according to a similar method to that of Reference Example 81 by using 1-iodopentane instead of 1-iodopropane. ¹H-NMR(CDCl₃) δ(ppm): 0.90(3H,br.t,*J*=6.0Hz), 1.22-1.53(15H,m), 3.22-3.27(2H,m), 3.94(2H,d,*J*=32.6Hz), 9.89(1H,br.s). ESI/MS (m/z): 244(M-H)⁻.

### Reference Example 82

### (-)-(2-[(2S,4R)-4-Fluoro-2-[4-(5-fluorothiazol-2-ylcarbamoy 1)-5-methylthiophen-2-yl]pyrrolidin-1-yl]-2-oxoethyl)carbam ic acid t-butyl ester

5-((2*S*,4*R*)-4-Fluoropyrrolidin-2-yl)-2-methylthiophene-3-ca rboxylic acid (5-fluorothiazol-2-yl)amide (35.0 mg), 1-ethyl-3-(3-dimethylaminopropyl)carbodiimide hydrochloride (24.0 mg), 1-hydroxy-1H-benzotriazole monohydrate (20.0 mg) and *N*-*t*-BOC-glycine (23.0 mg) were dissolved in *N,N*-dimethylformamide (0.50 mL), diisopropylethylamine (13.0 µL) was added thereto, and stirring was conducted overnight at room temperature. A saturated aqueous sodium hydrogen carbonate solution was added to the reaction liquid, extraction was conducted by using chloroform, and the organic phase was dried over anhydrous sodium sulfate. Filtration was conducted, and the filtrate was then concentrated under a reduced pressure to give the title compound (54.0 mg) as a white powder.
ESI/MS (m/z): 487(M+H)⁺, 485(M-H)⁻.

Compounds were synthesized according to the following reaction formula with referring to the method of Reference Example 82. The synthesized compounds and data are shown in Tables 5 and 6.

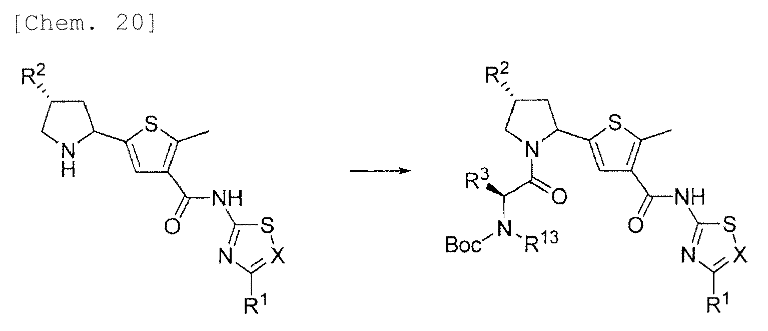

**[Table 5]**

| Reference Example | Starting raw material | R¹ | R² | R³ | R¹³ | X | ESI/MS (m/z): |
|---|---|---|---|---|---|---|---|
| 83 | Reference Example 27 | H | F | Me | H | C-F | 501 (M+H)⁺ 499 (M-H)⁻ |
| 84 | Reference Example 27 | H | F | *i*-Pr | H | C-F | 511 (M+H)⁺ 509 (M-H)⁻ |
| 85 | Reference Example 27 | H | F | H | Me | C-F | 501 (M+H)⁺ 499 (M-H)⁻ |
| 86 | Reference Example 27 | H | F | H | Et | C-F | 515 (M+H)⁺ 513 (M-H)⁻ |
| 87 | Reference Example 27 | H | F | H | *n*-Pr | C-F | 529 (M+H)⁺ 527 (M-H)⁻ |
| 88 | Reference Example 27 | H | F | H | *n*-Pen | C-F | 557 (M+H)⁺ 555 (M-H)⁻ |
| 89 | Reference Example 27 | H | F | Me | Me | C-F | 515 (M+H)⁺ 513 (M-H)⁻ |
| 90 | Reference Example 29 | Et | F | Me | H | N | 512 (M+H)⁺ 510 (M-H)⁻ |
| 91 | Reference Example 29 | Et | F | Et | H | N | 526 (M+H)⁺ 524 (M-H)⁻ |
| 92 | Reference Example 29 | Et | F | *i*-Pr | H | N | 540 (M+H)⁺ 538 (M-H)⁻ |
| 93 | Reference Example 29 | Et | F | H | Et | N | 526 (M+H)⁺ 524 (M-H)⁻ |
| 94 | Reference Example 23 | H | H | Me | H | C-F | 483 (M+H)⁺ 481 (M-H)⁻ |
| 95 | Reference Example 23 | H | H | Et | H | C-F | 497 (M+H)⁺ 495 (M-H)⁻ |
| 96 | Reference Example 23 | H | H | *i*-Pr | H | C-F | 511 (M+H)⁺ 509 (M-H)⁻ |
| 97 | Reference Example 23 | H | H | *n*-Pr | H | C-F | 511 (M+H)⁺ 509 (M-H)⁻ |
| 98 | Reference Example 23 | H | H | H | Me | C-F | 483 (M+H)⁺ 481 (M-H)⁻ |
| 99 | Reference Example 23 | H | H | H | Et | C-F | 483 (M+H)⁺ 481 (M-H)⁻ |
| 100 | Reference Example 25 | H | H | Me | H | N | 466 (M+H)⁺ 464 (M-H)⁻ |
| 101 | Reference Example 26 | Et | H | Me | H | N | 494 (M+H)⁺ 492 (M-H)⁻ |
| 102 | Reference Example 30 | H | H | H | H | C-F | 469 (M+H)⁺ 467 (M-H)⁻ |
| 103 | Reference Example 30 | H | H | H | Me | C-F | 483 (M+H)⁺ 481 (M-H)⁻ |
| 104 | Reference Example 30 | H | H | H | *n*-Pr | C-F | 511 (M+H)⁺ 509 (M-H)⁻ |

**[Table 6]**

| Reference Example | Starting raw material | R¹ | R² | R³ | R¹³ | X | ESI/MS (m/z): |
|---|---|---|---|---|---|---|---|
| 105 | Reference Example 30 | H | H | H | *n*-Pen | C-F | 539 (M+H)⁺ 537 (M-H)⁻ |
| 106 | Reference Example 31 | H | H | H | Me | C-Cl | 499 (M+H)⁺ 497 (M-H)⁻ |
| 107 | Reference Example 34 | Et | H | H | Me | N | 494 (M+H)⁺ 492 (M-H)⁻ |
| 108 | Reference Example 35 | SMe | H | H | Me | N | 512 (M+H)⁺ 510 (M-H)⁻ |

Compounds were synthesized according to the following reaction formula with referring to the method of Reference Example 82. The synthesized compounds are shown in Table 7, and the data are shown in Tables 8 and 9.

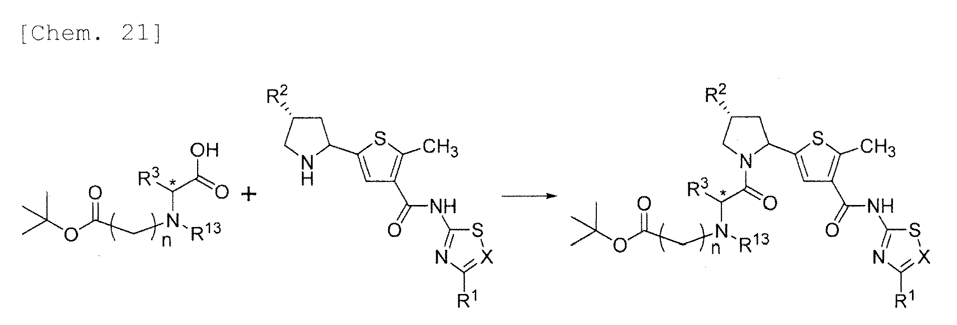

**[Table 7]**

| Reference Example | Starting raw material | Starting raw material | R¹ | R² | R³ | R¹³ | X | n |
|---|---|---|---|---|---|---|---|---|
| 109 | Reference Example 66 | Reference Example 28 | H | F | H | Et | C-Cl | 1 |
| 110 | Reference Example 66 | Reference Example 31 | H | H | H | Et | C-Cl | 1 |
| 111 | Reference Example 66 | Reference Example 32 | H | H | H | Et | C-H | 1 |
| 112 | Reference Example 66 | Reference Example 37 | H | F | H | Et | N | 1 |
| 113 | Reference Example 66 | Reference Example 23 | H | H | H | Et | C-F | 2 |
| 114 | Reference Example 67 | Reference Example 27 | H | F | H | Et | C-F | 2 |
| 115 | Reference Example 67 | Reference Example 31 | H | H | H | Et | C-Cl | 2 |
| 116 | Reference Example 68 | Reference Example 23 | H | H | H | *i*-Pr | C-F | 2 |
| 117 | Reference Example 67 | Reference Example 33 | H | H | H | Et | N | 1 |
| 118 | Reference Example 69 | Reference Example 23 | H | H | Et | Et | C-F | 1 |
| 119 | Reference Example 70 | Reference Example 23 | H | H | Et | Et | C-F | 1 |
| 120 | Reference Example 71 | Reference Example 23 | H | H | *i*-Pr | Et | C-F | 1 |
| 121 | Reference Example 72 | Reference Example 23 | H | H | *i*-Pr | Et | C-F | 1 |
| 122 | Reference Example 73 | Reference Example 23 | H | H | Me | Et | C-F | 1 |
| 123 | Reference Example 73 | Reference Example 27 | H | F | Me | Et | C-F | 1 |
| 124 | Reference Example 74 | Reference Example 23 | H | H | Me | Et | C-F | 1 |
| 125 | Reference Example 74 | Reference Example 27 | H | F | Me | Et | C-F | 1 |
| 126 | Reference Example 75 | Reference Example 23 | H | H | *n*-Pr | Et | C-F | 1 |
| 127 | Reference Example 75 | Reference Example 27 | H | F | *n*-Pr | Et | C-F | 1 |
| 128 | Reference Example 76 | Reference Example 23 | H | H | *n*-Pr | Et | C-F | 1 |
| 129 | Reference Example 76 | Reference Example 27 | H | F | *n*-Pr | Et | C-F | 1 |
| 130 | Reference Example 77 | Reference Example 23 | H | H | Me | Et | C-F | 2 |
| 131 | Reference Example 78 | Reference Example 23 | H | H | Me | Et | C-F | 2 |
| 132 | Reference Example 79 | Reference Example 23 | H | H | H | *i*-Pr | C-F | 1 |

**[Table 8]**

| Reference Example | ¹H-NMR(CDCl₃)δ(ppm) |
|---|---|
| 109 | 0.96,1.06(3H,eacht,*J*=7.2Hz),1.41,1.45(9H,eachs),2.17(1H,m),2.7-2.9(2H,m),2.72, 2.75(3H,eachs),3.2-3.4(3H,m),3.41(1H,d,*J*=14.4Hz),3.47(1H,d,*J*=14.4Hz),3.90(1H ,ddd,*J*=36,13.2,3.6Hz),4.30(1H,dd,*J*=119.6.13.2Hz),5.2-5.5(2H,m),7.19(1H,s),7.23( 1H,s). |
| 110 | 1.07-1.10(3H,m),1.45(9H,s),1.93-2.33(4H,m),2.63-2.78(5H,m),3.24-3.81(6H,m), 5.36(1H,d,*J*=5.9Hz),7.12(1H,s),7.20(1H,s). |
| 111 | 1.07-1,11(3H,m),1.45(9H,s),1.93-2.38(4H,m),2.66-2.78(5H,m),3.24-3.81(6H,m), 5.36(1H,d,*J*=6.6Hz),6.97(1H,d,*J*=3.7Hz),7.12(1H,s),7.37(1H,d,*J*=3.7Hz). |
| 112 | 0.96,1.07(3H,eacht,*J*=7.6Hz),1.42,1.46(9H,eachs),2.17(1H,m),2.7-2.9(3H,m),2.7 1,2.79(3H,eachs),3.2-3.6(4H,m),3.96(1H,ddd,*J*=36,13.2,3.6Hz),4.36(1H,dd,*J*=20. 4,13.6Hz),5.2-5.5(2H,m),7.41,7.44(1H,each s),8.31,8.33(1H,each s). |
| 113 | 1.07(3H,t,*J*=7.1Hz),1.45(9H,s),1.92-2.00(3H,m),2.06-2.21(2H,m),2.46-2.53(3H, m),2.62-2.71(2H,m),2.75(3H,s),3.04-3.12(1H,m),3.43-3.52(1H,m),4.03-4.08(1H, m),5.30-5.33(1H,m),6.95(1H,d,*J*=2.9Hz),7.39(1H,s),10.0(1H,br.s). |
| 114 | 1.06(3H,t,*J*=7.1Hz),1.46(9H,s),2.41-2.51(2H,m),2.60-2.76(3H,m),2,74(3H,s),3.4 3-3.47(1H,m),3.57-3.72(1H,m),3.91-4.12(2H,m),5.20-5.42(3H,m),5.80-5.86(1H, m),6.95(1H,d,*J*=2.9Hz),7.45(1H,s),9.97(1H,br.s). |
| 115 | 1.05-1.09(3H,m),1.45(9H,s),1.95-2.18(4H,m),2,28-2.32(1H,m),2.47-2.54(2H,m), 2.65-2.85(5H,m),2.93-3.10(2H,m),3.41-3.52(2H,m),4.02-4.06(1H,m),5.32(1H,d,*J* =6.6Hz),7.20(1H,s),7.41(1H,s). |
| 116 | 093-0.97(6H,m),1.46(9H,s),1.94-2.00(2H,m),2.02-2.30(3H,m),2.42-2.49(2H,m), 2.74(3H,s),2.85-2.96(1H,m),3.07-3.11(1H,m),3.12-3.22(1H,m),3.36-3.39(1H,m), 3.42-3.57(1H,m),4.07-4.13(1H,m),5.29-5.33(1H,m),6.96(1H,d,*J*=2.9Hz),7.42(1H, s),9.94(1H,br.s). |
| 117 | 0.95-0.99(3H,m),1.39(9H,s),1.86-2.24(4H,m),2.50-2.70(5H,m),3.15-3.75(6H,m), 5.20(1H,d,*J*=7.3Hz),7.58(1H,s),8.52(1H,s). |
| 118 | 0.87(3H,t,*J*=7.6Hz),1.07(3H,t,*J*=7,6Hz),1.44(9H,s),1.55-1.61(1H,m),1.76-1.83(1 H,m),2,00-2.28(4H,m),2.67-2.79(5H,m),3.25-3.65(4H,m),4.02-4.08(1H,m),5.33( 1H,d,*J*=7.6Hz),6.94(1H,d,*J*=3.2Hz),7.03(1H,s),9.74(1H, br.s). |
| 119 | 0.88(3H,t,*J*=7.6Hz),1.08(3H,t,*J*=7.2Hz),1.37(9H,s),1.55-1.61(1H,m),1.75-1.83(1 H,m),1.94-2.20(4H,m),2.68-1.79(5H,m),3.19-3.39(2H,m),3.42-3.46(1H,m),3.52-3.59(1H,m),4.08-4.15(1H,m),5.36(1H,d,*J*=6.4Hz),6.96(1H,s),7.19(1H,s),9.98(1H, br.s). |
| 120 | 0.81(3H,d,*J*=5.9Hz),1.00(3H,d,*J*=6.6Hz),1.05(3H,t,*J*=7.0Hz),1.44(9H,s),2.04-2.2 6(5H,m),2.70(3H,s),2.70-2.78(1H,m),2.83-2.91(1H,m),3.10(1H,d,*J*=10.3Hz),3.29 (1H,d,*J*=17.6Hz),3.50(1H,d,*J*=17.6Hz),3.60-3.65(1H,m),3.70-3.77(1H,m),5.35(1 H,d,*J*=7.7Hz),6.90(1H,m),7.09(1H,s). |
| 121 | 0.86(3H,d,*J*=6.6Hz),1.01(3H,d,*J*=7.0Hz),1.09(3H,t,*J*=7.3Hz),1.33(9H,s),1.98.-2.1 9(5H,m),2.73(3H,s),2.85-2.93(1H,m),2.99-3.08(1H,m),3.16(1H,d,*J*=10.3Hz),3.40 (1H,d,*J*=17.6Hz),3.51(1H,d,*J*=17.6Hz),3.53-3.60(1H,m),3.75-3.79(1H,m),5.37(1 H,d,*J*=7.0Hz),6.96(1H,m),7.24(1H,s). |

**[Table 9]**

| Reference Example | ¹H-NMR(CDCl₃)δ(ppm) |
|---|---|
| 122 | 1.08(3H,t,J=7.2Hz),1.16(2H,d,J=6.8Hz),1.44(9H,s),1.97-2.14(2H,m),2.20-2.29(1 H,m),2.36-2.41(1H,m),2.60-2.74(5H,m),3.17-3.39(2H,m),3.48-3.67(2H,m),3.75-3 .80(1H,m),4.21-4.28(1H,m),5.31(1H,d,J=7.2Hz),6.96(1 H,d,J=2.8Hz),7.02(1H,s),9 .58(1H,br.s). |
| 123 | 1.06(3H,t,J=7.1Hz),1.17(3H,d,J=6.6Hz),1.1-1.3(1H,m),1.46(9H,s),2.5-2.8(3H,m), 2.70(3H,s),3.22-3.33(2H,m),2.55-2.85(2H,m),5.01-5.09(1H,m).5.2-5.5(2H,m),6.8 6(1H,d,J=2.6Hz),7.18(1H,s),11.06(1H,br.s). |
| 124 | 1.10(3H,t,J=7.2Hz),1.14(3H,d,J=6.4Hz),1.40(9H,s),1.94-2.21(3H,m),2.58-2.71(5 H,m),3.10-3.31(2H,m),3.47-3.58(2H,m),3.70-3.77(1H,m),4.26-4.29(2H.m),6.94(1 H,d,J=2.4Hz),7.14(1H,s),9.85(1H,br.s). |
| 125 | 1.11(3H,t,J=6.4Hz),1.45(3H,d,J=7.3Hz),1.46(9H,s),2.0-2.25(1H,m),2.5-2.75(3H, m),2.64(3H,s),3.02-3.24(2H,m),3.71-3.76(1H,m),4.0-4.55(2H,m),5.2-5.4(2H,m),6 .88(1H,d,J=2.2Hz),7.30(1H,s),10.92(1H,br.s). |
| 126 | 0.88-0.98(3H,m),1.06(3H,t,J=7.11Hz),1.20-1.80(14H,m),1.95-2,44(5H,m),2.61-2.8 2(5H,m),3.58-3.78(2H,m),3.98-4.09(1H,m),5.25-5.34(1H,m),7.12-7.13(1H,m),7.2 3(1H,s). |
| 127 | 0.90(3H,t,J=7.3Hz),1.04(3H,t,J=7.2Hz),1.15-1.85(4H,m),1.45(9H,s),2.05-2.3(1H, m),2.6-2.8(4H,m),2.71(3H,s),3.25-3.4(2H,m),3.6-3.8(1H,m),4.67-4.76(1H,m),5.2-5,5(2H,m),6.89(1H,J=2.7Hz),7.14(1H,s),10.97(1H,br.s). |
| 128 | 0.70(3H,J=7.3Hz),1.01-1.78(15H,m),1.96-2.80(10H,m),3.10-3.27(1H,m)3.49-3.57(1H,m),3.63-3.76(2H,m),4.08-4.20(1H,m),5.94-6.0(1H,m),7.10-7.18(1H,m),7.2 9(1H, s). |
| 129 | 0.91(3H,t,J=7.0Hz),1.07(3H,t,J=7.1Hz),1.35-1.85(4H,m),1.44(9H,s),2.05-2.3(1H, m),2.45-2.75(4H,m),2.69(3H,s),3.06-3.25(2H,m),3.4-3.5(1H,m),4.2-4.4(1H,m),5. 15-5.45(2H,m),6.90(1H,J=2,6Hz),7.24(1H,s),10.95(1H,br.s). |
| 130 | 1.06(3H,t,J=7.1Hz),1.15(3H,d,J=6.6Hz),1.46(9Hs),1.95-2.45(5H,m),2.49-2.62(1 H,m),2.62-2.85(5H,m),2.85-3.01(1H,m),3.75-3.87(2H,m),4.01-4.11(1H,m),5.28-5 .37(1H,m),7.08-7.16(1H,m),7.23(1H,s). |
| 131 | 0.98-1.00(3H,m),1.09-1,18(3H,m),1.38-1.52(9H,m),1.94-2.94(13H,m),3.45-3.85(. 2H,m),4.06-4.16(1H,m),5.29-5.36(1H,m),7.09-7.16(1H,m),7.32(1H,s). |
| 132 | 0.82-0.93(6H,m),1.45(9H,s),1.68-1.74(1H,m),1.82-2.20(3H,m),2.45(2H,d,J=7.3H z),2.71(3H,s),3.21-3.38(2H,m),3.45-3.61(3H,m),3.65-3.82(2H,m),5.35(1H,d,J=7. 3Hz),6.92-7.02(1H,m),7.05-7.10(1H,m). |

### Reference Example 133

### 5-[1-(2-Chloroacetyl)pyrrolidin-2-yl]-2-methylthiophene-3-c arboxylic acid (5-fluorothiazol-2-yl)amide

2-Methyl-5-pyrrolidin-2-ylthiophene-3-carboxylic acid (5-fluorothiazol-2-yl)amide (467 mg) was dissolved in methylene chloride (5.0 mL), di-*t*-butylpyridine (663 µL) and chloroacetyl chloride (239 µL) were added thereto at 0°C, and stirring was conducted at room temperature for 4 hours. Water was added to the reaction liquid, extraction was conducted by using chloroform, and the organic phase was dried over anhydrous sodium sulfate. Filtration was conducted, the filtrate was then concentrated under a reduced pressure, and the obtained residue was purified by silica gel chromatography (hexane/ethyl acetate=1/2) to give the title compound (536 mg) as a white powder.
¹H-NMR(CDCl₃) δ(ppm): 1.97-2.32(4H,m), 2.71(3H,s), 3.60-3.70(1H ,m), 3.75-3.78(1H,m), 3.83-4.00(1H,m), 4.8(2H,s), 5.39(1H,d,*J*=5 .5Hz), 6.95(1H,s), 7.15(1H,s).
ESI/MS (m/z): 388(M+H)⁺, 386(M-H)⁻.

### Reference Example 1.34

### 5-[1-(2-Chloroacetyl)pyrrolidin-2-yl]-2-methylthiophene-3-c arboxylic acid (5-chlorothiazol-2-yl)amide

The title compound was obtained by conducting a reaction according to a similar method to that of Reference Example 57 by using 2-methyl-5-pyrrolidin-2-ylthiophene-3-carboxylic acid (5-chlorothiazol-2-yl)amide instead of 2-methyl-5-pyrrolidin-2-ylthiophene-3-carboxylic acid (5-fluorothiazol-2-yl)amide.
¹H-NMR(CDCl₃) δ(ppm): 1.97-2.42(4H,m), 2.71(3H,s), 3.60-3.68(1H ,m), 3.75-3.83(1H,m), 3.88-4.05(1H,m), 4.12-4.19(2H,m), 5.37-5. 41(1H,m), 7.20(1H,s), 7.32(1H,s).
ESI/MS (m/z): 404(M+H)⁺, 402(M-H)⁻.

### Reference Example 135

### (-)-5-[(2S,4R)-1-(2-Aminoacetyl)-4-fluoropyrrolidin-2-yl]-2 -methylthiophene-3-carboxylic acid

(5-fluorothiazol-2-yl)amide (-)-(2-[(2*S*,4*R*)-4-Fluoro-2-[4-(5-fluorothiazol-2-ylcarbamo yl)-5-methylthiophen-2-yl]pyrrolidin-1-yl]-2-oxoethyl)carba mic acid *t*-butyl ester (54.0 mg) was dissolved in methylene chloride (0.50 mL), trifluoroacetic acid (0.500 mL) was added thereto, and stirring was conducted at room temperature for 15 hours. The reaction liquid was concentrated under a reduced pressure, a saturated aqueous sodium hydrogen carbonate solution was added to the obtained residue, extraction was conducted by using chloroform, and the organic phase was dried over anhydrous sodium sulfate. Filtration was conducted, the filtrate was then concentrated under a reduced pressure, and the obtained residue was purified by thin layer silica gel chromatography (methylene chloride/methanol=15/1-5/1) to give the title compound (31.0 mg) as a white powder.
ESI/MS (m/z): 387(M+H)⁺, 385(M-H)⁻.

Compounds were synthesized according to the following reaction formula with referring to the method of Reference Example 135. The synthesized compounds and data are shown in Tables 10 and 11.

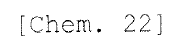

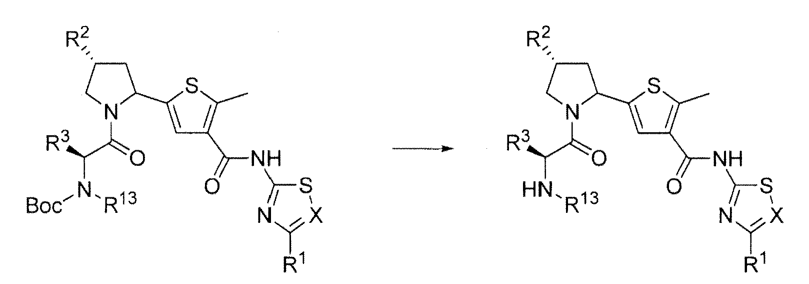

**[Table 10]**

| Reference Example | Starting raw material | R¹ | R² | R³ | R¹³ | X | ESI/MS (m/z): |
|---|---|---|---|---|---|---|---|
| 136 | Reference Example 83 | H | F | Me | H | C-F | 401(M+H)⁺ 399 (M-H)⁻ |
| 137 | Reference Example 84 | H | F | *i*-Pr | H | C-F | 383 (M+H)⁺ 381 (M-H)⁻ |
| 138 | Reference Example 85 | H | F | H | Me | C-F | 401 (M+H)⁺ 399 (M-H)⁻ |
| 139 | Reference Example 86 | H | F | H | Et | C-F | 415 (M+H)⁺ 413 (M-H)⁻ |
| 140 | Reference Example 87 | H | F | H | *n*-Pr | C-F | 429 (M+H)⁺ 427 (M-H)⁻ |
| 141 | Reference Example 88 | H | F | H | *n*-Pen | C-F | 457 (M+H)⁺ 455 (M-H)⁻ |
| 142 | Reference Example 89 | H | F | Me | Me | C-F | 415 (M+H)⁺ 413 (M-H)⁻ |
| 143 | Reference Example 90 | Et | F | Me | H | N | 412 (M+H)⁺ 410(M-H)⁻ |
| 144 | Reference Example 91 | Et | F | Et | H | N | 426(M+H)⁺ 424(M-H)⁻ |
| 145 | Reference Example 92 | Et | F | *i*-Pr | H | N | 440(M+H)⁺ 438(M-H)⁻ |
| 146 | Reference Example 93 | Et | F | H | Et | N | 426(M+H)⁺ 424(M-H)⁻ |
| 147 | Reference Example 94 | H | H | Me | H | C-F | 383(M+H)⁺ 381(M-H)⁻ |
| 148 | Reference Example 95 | H | H | Et | H | C-F | 397(M+H)⁺ 395(M-H)⁻ |
| 149 | Reference Example 96 | H | H | *i*-Pr | H | C-F | 411(M+H)⁺ 409(M-H)⁻ |
| 150 | Reference Example 97 | H | H | *n*-Pr | H | C-F | 411(M+H)⁺ 409(M-H)⁻ |
| 151 | Reference Example 98 | H | H | H | Me | C-F | 383(M+H)⁺ 381(M-H)⁻ |
| 152 | Reference Example 99 | H | H | H | Et | C-F | 397(M+H)⁺ 395(M-H)⁻ |
| 153 | Reference Example 100 | H | H | Me | H | N | 366(M+H)⁺ 364(M-H)⁻ |
| 154 | Reference Example 101 | Et | H | Me | H | N | 412(M+H)⁺ 410(M-H)⁻ |
| 155 | Reference Example 102 | H | H | H | H | C-F | C-F 369(M+H)⁺ 367(M-H)⁻ |
| 156 | Reference Example 103 | H | H | H | Me | C-F | 383(M+H)⁺ 381(M-H)⁻ |
| 157 | Reference Example 104 | H | H | H | *n*-Pr | C-F | 411(M+H)⁺ 409(M-H)⁻ |

**[Table 11]**

| Reference Example | Starting raw material | R¹ | R² | R³ | R¹³ | X | ESI/MS (m/z): |
|---|---|---|---|---|---|---|---|
| 158 | Reference Example 105 | H | H | H | *n*-Pen | C-F | 439(M+H)+ 437(M-H)- |
| 159 | Reference Example 106 | H | H | H | Me | C-Cl | 399(M+H)+ 397(M-H)- |
| 160 | Reference Example 107 | Et | H | H | Me | N | 394(M+H)+ 392(M-H)- |
| 161 | Reference Example 108 | SMe | H | H | Me | N | 412(M+H)+ 410(M-H)- |

### Reference Example 162

### (-)-(2-[(2S,4R)-4-Fluoro-2-[4-(5-fluorothiazol-2-ylcarbamoy l)-5-methylthiophen-2-yl]pyrrolidin-1-yl]-2-oxoethylamino)a cetic acid t-butyl ester

(-)-5-[(2*S*,4*R*)-1-(2-Aminoacetyl)-4-fluoropyrrolidin-2-yl]-2-methylthiophene-3-carboxylic acid (5-fluorothiazol-2-yl)amide (31.0 mg) was dissolved in tetrahydrofuran (1.0 mL), *t*-butyl bromoacetate (16.0 mg) and triethylamine (8.10 mg) were added thereto, and stirring was conducted overnight at room temperature. Water was added to the reaction liquid, extraction was conducted by using ethyl acetate, and the organic layer was washed with saturated brine and dried over anhydrous sodium sulfate. Filtration was conducted, the filtrate was then concentrated under a reduced pressure, and the obtained residue was purified by silica gel chromatography (methylene chloride/methanol=30/1) to give the title compound (19.0 mg) as a white powder.
ESI/MS(m/z): 501(M+H)⁺, 499(M-H)⁻.

Compounds were synthesized according to the following reaction formula with referring to the method of Reference Example 162. The synthesized compounds are shown in Table 12, and the data thereof are shown in Table 13.

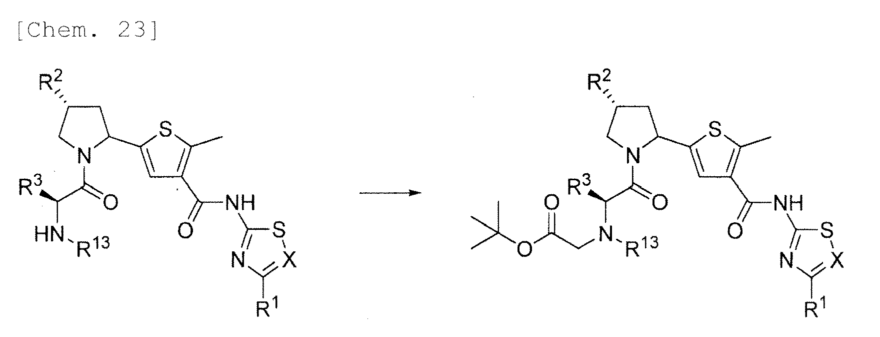

**[Table 12]**

| Reference Example | Starting raw material | R¹ | R² | R³ | R¹³ | X |
|---|---|---|---|---|---|---|
| 163 | Reference Example 137 | H | F | *i*-Pr | H | C-F |
| 164 | Reference Example 138 | H | F | H | Me | C-F |
| 165 | Reference Example 139 | H | F | H | Et | C-F |
| 166 | Reference Example 140 | H | F | H | *n*-Pr | C-F |
| 167 | Reference Example 141 | H | F | H | *n*-Pen | C-F |
| 168 | Reference Example 142 | H | F | Me | Me | C-F |
| 169 | Reference Example 146 | Et | F | H | Et | N |
| 170 | Reference Example 147 | H | H | Me | H | C-F |
| 171 | Reference Example 148 | H | H | Et | H | C-F |
| 172 | Reference Example 149 | H | H | *i*-Pr | H | C-F |
| 173 | Reference Example 152 | H | H | H | Et | C-F |
| 174 | Reference Example 157 | H | H | H | *n*-Pr | C-F |
| 175 | Reference Example 158 | H | H | H | *n*-Pen | C-F |

**[Table 13]**

| Reference Example | ¹H-NMR (CDCl₃)δ(ppm) | ESI/MS (m/z): |
|---|---|---|
| 163 | 0.93-0,97(6H,m),1,38-1.45(9H,m),1.86-1.89(1H,m),2.14-2.35(1H,m),2. 73(3H,s),2.92-3.31(3H,m),3.59-3.77(1H,m),4.02-4.10(1H,m),5.24-5.38( 1H,m),5.52(1H,t,*J*=7.2Hz),6.88-6.98(1H,m),7.21-7.26(1H,m). | 543(M+H)⁺ 541(M-H)⁻ |
| 164 | 1.42-1.47(9H,m),2.08-2.28(1H,m),2.41(3H,s),2.72(3H,s),3.11-3.46(3H, m),3.80-4.36(2H,m),5.19-5.70(2H,m),6.97-7.01(1H,m),7.18(1H,s),9.73( 1H,br.s). | 515(M+H)⁺ 513(M-H)⁻ |
| 165 | 1.07(3H,t,*J*=7.1Hz),1.42-1.46(9H,m),2.12-2.24(1H,m),2.65-2.72(7H,m), 3.21-3.32(3H,m),3.38-3.50(2H,m),3.84-4.31 (1H,m),5.19-5.37(1H,m),6. 96-6.98(1H,m),7.00-7.17(1H,m),7.24(1H,s),9.75(1H,br.s). | 529(M+H)⁺ 527(M-H)⁻ |
| 166 | 0.82-0.89(3H,m), 1.37-1.50(11H,m),1.76-1.84(1H,m),2.12-2.24(1H,m),2 .50-2.57(2H,m),2.65-2.74(4H,m),3.20-3.52(4H,m),4.26-4.35(1H,m),5.1 9-5.46(2H,m),6.94(1H,d,*J*=29.3Hz),7.21(1H,br.d,*J*=7.7Hz). | 543(M+H)⁺ 541(M-H)⁻ |
| 167 | 0.87(3H,t,*J*=7.0Hz),1.20-1.46(15H,m),2.10-2.30(1H,m),2.55-2.75(6H,m ),3.20-4.30(6N,m),5.16-5.45(2H,m),6.92(1H,br,d,*J*=33.5Hz),7.21(1H,s), 10.6(1H,br.s). | 553(M+H)⁺ 551(M-H)⁻ |
| 168 | 1.18-1.20(3H,m),1.46(9H,s),2.10-2.28(1H,m),2.37(3H,s),2.71(3H,s),3.2 3-3.26(2H,m),3.63-3.74(2H,m),4.28-4.90(1H,m),5.25-5.46(2H,m),6.96( 1H,s),7.14(1H-,s),9.93(1H,br.s). | 529(M+H)⁺ 527(M-H)⁻ |
| 169 | 1.45(9H,s),2.05-2.22(1H,m),2.65(3H,s),7.72-2.80(1H,m),3.28-3.60(4H, m),3.72-3.92(2H,m),5.20-5.48(2H,m),6.99-7.01(1H,m),7.42(1H,s). | 540 (M+H)⁺ 538(M-H)⁻ |
| 170 | 1.25-1.27(3H,m),1.40(9H,s),2.04-2.30(4H,m),2.71 (3H,s),3.00-3.37(2H, m),3.53-3.62(3H,m),5.22(1H,d,*J*=6.2Hz),6.92(1H,d,*J*=2.6Hz),7.13(1H,d ,*J*=5. 1Hz). | 497(M+H)⁺ 495(M-N)⁻ |
| 171 | 0.92-1.01 (3H,m),1.40(9H,s),1.56-1.78(3H,m),2.01-2.32(4H,m),2.68-2.7 9(3H,m),3.10-3.40(2H,m),3.48-3.76(2H,m),5.06-5.44(1H,m),6.79-7.09( 2H,m). | 511(M+H)⁺ 509(M-H)⁻ |
| 172 | 0.93-1.01(6H,m),1.37-1.45(9H,m),1.86-1.91(1H,m),2.05-2.27(5H,m),2. 71(3H,s),3.00-3.29(3H,m),3.56-3,74(2H,m),5.26(1H,d,*J*=6.4Hz),6.82-6.98(1H,m),7.11-7.13(1H,m). | 525(M+H)⁺ 523(M-H)⁻ |
| 173 | 1.03(3H,t,*J*=7.1Hz),1.40-1.45(9H,m),1.92-2.21(4H,m),2.70-2.76(5H,m), 3.22-3.49(4H,m),3.60-3.77(2H,m),5.48(1H,d,*J*=7.3Hz).6.85-6.94(1H,m) ,7.12-7.17(1H,m),10.60(1H,br.s). | 511(M+H)⁺ 509(M-H)⁻ |
| 174 | 0.82-0.89(3H,m),1.35-1.50(11H,m),1.93-2.32(4H,m),2.50-2.65(2H,m),2 .72(3H,d,*J*=11.4Hz),3.25-3.80(6H,m),5.35(1H,d,*J*=5.9Hz),6.96(1H,br.d, *J*=20.3Hz),7.11(1H,d,*J*=11.0Hz),9.87(1H,br.s). | 553(M+H)⁺ 551(M-H)⁻ |
| 175 | 0.82-0.88(3H,m),1.24-1.47(15H,m),1.82-2.35(4H,m),2.50-2.73(5H,m),3 .24-3.80(6H,m),5.35(1H,d,*J*=5.5Hz),6.94(1H,br.d,*J*=24.6Hz),7.12(1H,d, *J*=17,2Hz).10.1(1H,br.s). | 553(M+H)⁺ 551(M-H)⁻ |

### Reference Example 176

### (-)-3-[(2-[2-[4-(5-Fluorothiazol-2-ylcarbamoyl)-5-methylthi ophen-2-yl]pyrrolidin-1-yl]-2-oxoethyl)methylamino]propioni c acid t-butyl ester

(-)-2-Methyl-5-[1-(2-methylaminoacetyl)pyrrolidin-2-yl]thi ophene-3-carboxylic acid (5-fluorothiazol-2-yl)amide (425 mg) was dissolved in 1,4-dioxane (10 mL), butyl acrylate (486 µL) and Triton B (19.0 µL) were added thereto at room temperature, and stirring was conducted overnight at 60°C. The reaction liquid was concentrated under a reduced pressure, and the obtained residue was purified by silica gel chromatography (methylene chloride/methanol-20/1) to give the title compound (238 mg) as a white powder.
¹H-NMR(CDCl₃) δ(ppm): 1.43(9H,d,*J*=8.1Hz), 1.96-2.01(2H,m) 2.09 -2.38(4H,m), 2.45(2H,t,*J*=7.0Hz), 2.65-2.87(2H,m), 3.11-3.29(2H ,m), 3.46(3H,d,*J*=7.3Hz), 3.53-3.60(1H,m), 3.71-3.88(1H,m), 5.47 (1H,d,*J*=7.3Hz), 6.83-6.91(1H,m), 7.23-7.28(1H,m), 11.23(1H,br. s).
ESI/MS(m/z): 511(M+H)⁺, 509(M-H)⁻.

Compounds were synthesized according to the following reaction formula with referring to the method of Reference Example 176. The synthesized compounds are shown in Table 14, and the data thereof are shown in Table 15.

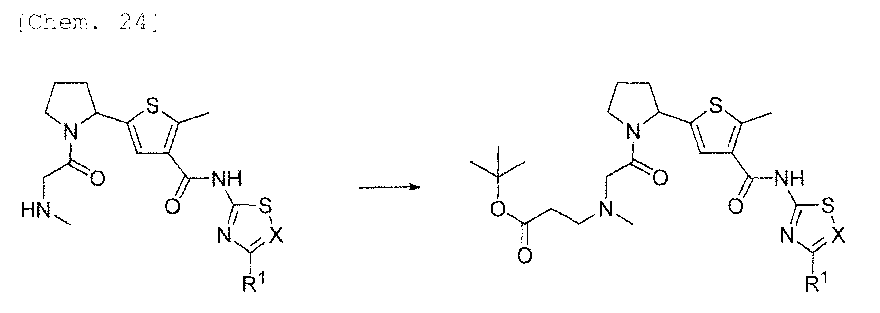

**[Table 14]**

| Reference Example | Starting raw material | R¹ | X |
|---|---|---|---|
| 177 | Reference Example 156 | H | C-F |
| 178 | Reference Example 159 | H | C-Cl |
| 179 | Reference Example 160 | Et | N |

**[Table 15]**

| Reference Example | ¹H-NMR(CDCl₃)δ(ppm) | ESI/MS (m/z): |
|---|---|---|
| 177 | 1.42(9H,s),2.00-2.14(4H,m),2.25-2.36(4H,m),2.43-2.46(2H,m),2.63 -2.79(4H,m),2.91-3.26(2H,m),3.53-3.86(2H,m),5.31-5,55(1H,m),7.1 1-7.13(1H,m),7.29-7.35(1H,m).(CD₃OD) | 511(M+H)⁺ 509(M-H)⁻ |
| 178 | 1.45(9H,s),1.98-2.19(4H,m),2.35(3H,s),2.47(2H,t,*J*=6.6Hz),2.59-2.7 1(2H,m),2.74(3H,s),2.85-3.30(2H,m),3.48-3.73(1H,m),3.91-3.97(1 H,m),5.29-5.34(1H,m),7.13(1H,s),7.24-7.27(1H,m),7.34(1H,s),10.7 3(1H,br.s). | 527(M+H)⁺ 525(M-H)⁻ |
| 179 | 1.32-1.37(3H,m),1.48(9H,s),1.93-2,02(1H,m),2.52-2.58(1H,m),2.78 (3H,s),2.81-2.90(2H,m),2.97-3.37(2H,m),5.31(1H,d,*J*=8.4Hz),7.24( 1H,s),7.53(1H,s). | 522(M+H)⁺ 520(M-H)⁻ |

### Reference Example 180

### (2-[2[4(5-Chlorothiazol-2-ylcarbamoyl)-5-methylthiophen-2 -yl]pyrrolidin-1-yl]-2-oxoethylamino)acetic acid t-butyl ester

Glycine *t*-butyl ester hydrochloride (210 mg) and diisopropylethylamine (257 µL) were dissolved in *N*,*N*-dimethylformamide (2 mL), and stirring was conducted at room temperature for 5 minutes.
5-[1-(2-Chloroacetyl)pyrrolidin-2-yl]-2-methylthiophene-3-c arboxylic acid (5-chlorothiazol-2-yl)amide (101 mg) was added slowly thereto at 50°C, and stirring was conducted at 50°C for 4 hours. Water was added to the reaction liquid, extraction was conducted by using ethyl acetate, and the organic layer was washed with a saturated aqueous sodium hydrogen carbonate solution and dried over anhydrous sodium sulfate. Filtration was conducted, the filtrate was then concentrated under a reduced pressure, and the obtained residue was purified by thin layer silica gel chromatography (chloroform/methanol=10/1) to give the title compound (106 mg) as a white powder.
ESI/MS (m/z):499(M+H)⁺, 497(M-H)⁻.

Compounds were synthesized according to the following reaction formula with referring to the method of Reference Example 180. The synthesized compounds are shown in Table 16, and the data thereof are shown in Table 17.

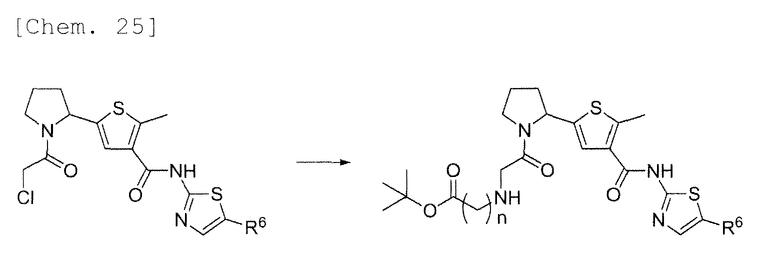

**[Table 16]**

| Reference Example | Starting raw material | R⁶ | n |
|---|---|---|---|
| 181 | Reference Example 133 | F | 3 |
| 182 | Reference Example 134 | Cl | 2 |

**[Table 17]**

| Reference Example | ¹H-NMR(CDCl₃)δ(ppm) | ESI/MS (m/z): |
|---|---|---|
| 181 | 1.42(9H,s),1.45-1.48(2H,m),1.68-1.85(2H,m),1.94-2.33(4H,m),2.53-2.64(2H,m),2.67(3H,s),3.24-3.76(5H,m),5.37(1H,d,*J*=6.2Hz),6.90(1 H,d,*J*=2.6Hz),7.25(1H,s). | 511(M+H)⁺ 509(M-H)⁻ |
| 182 | 1.43(9H,s),1.96-2.21(4H,m),2.40-2.47(2H,m),2.71(3H,s),2.79-2.96(2 H,m),3.42-3.66(4H,m),5.38(1H,d,*J*=6.6Hz),7.17(1H,s),7.24(1H,s). | 513(M+H)⁺ 511(M-H)⁻ |

### Reference Example 183

### 5-[(2-[2-[4-(5-Fluorothiazol-2-ylcarbamoyl)-5-methylthiophe n-2-yl]pyrrolidin-1-yl]-2-oxoethyl)methylamino]pentanoic acid methyl ester

2-Methyl-5-[1-(2-methylaminoacetyl)pyrrolidin-2-yl]thiophe ne-3-carboxylic acid (5-fluorothiazol-2-yl)amide (60.6 mg) was dissolved in *N*,*N*-dimethylformamide (1.0 mL), diisopropylethylamine (81.5 µL) and methyl 5-bromovalerate (27.0 µL) were added thereto at room temperature, and stirring was conducted overnight at 60°C. The reaction liquid was cooled to room temperature, water was added thereto, extraction was conducted by using ethyl acetate, and the organic layer was washed with saturated brine and dried over anhydrous sodium sulfate. Filtration was conducted, the filtrate was then concentrated under a reduced pressure, and the obtained residue was purified by thin layer silica gel chromatography (ethyl acetate/methanol=9/1) to give the title compound (63.0 mg) as a white powder.
¹H-NMR(CDCl₃) δ(ppm): 1.28-1.62(6H,m), 2.01-2.49(9H,m), 2.67-2. 71(3H,m), 2.76-2.99(1H,m), 3.56-3.85(5H,m), 4.25-4.57(1H,m), 5. 38(1H,d,*J*=6.9Hz), 7.12(1H,t,*J*=2.3Hz), 7.26-7.35(1H,m).
ESI/MS(m/z): 497(M+H)⁺, 495(M-H)⁻.

Compounds were synthesized according to the following reaction formula with referring to the method of Reference Example 183. The synthesized compounds and data are shown in Table 18.

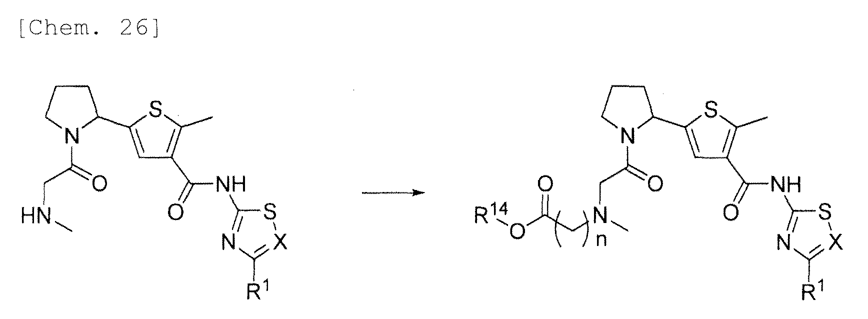

**[Table 18]**

| Reference Example | Starting raw material | R¹ | R¹⁴ | X | n | ESI/MS(m/z): |
|---|---|---|---|---|---|---|
| 184 | Reference Example 159 | H | *t*-Bu | C-Cl | 3 | 541(M+H)⁺ 539(M-H)⁻ |
| 185 | Reference Example 159 | H | Me | C-Cl | 4 | 513(M+H)⁺ 511(M-H)⁻ |
| 186 | Reference Example161 | SMe | *t*-Bu | N | 1 | 526(M+H)⁺ 524(M-H)⁻ |

### Example 10

### (-)-2-Methyl-5-[1-(2-ureidoacetyl)pyrrolidin-2-yl]thiophene -3-carboxylic acid (5-fluorothiazol-2-yl)amide

(-)-2-Methyl-5-pyrrolidin-2-ylthiophene-3-carboxylic acid (5-fluorothiazol-2-yl)amide (270 mg), 1-ethyl-3-(3-dimethylaminopropyl)carbodiimide hydrochloride (334 mg), 1-hydroxy-1H-benzotriazole monohydrate (264 mg) and hydantoin acid (206 mg) were dissolved in tetrahydrofuran (5. 40 mL), diisopropylethylamine (360 µL) was added thereto, and stirring was conducted at room temperature for 3 hours. A saturated aqueous sodium hydrogen carbonate solution was added to the reaction liquid, extraction was conducted by using chloroform, and the organic layer was dried over anhydrous sodium sulfate. Filtration was conducted, the filtrate was then concentrated under a reduced pressure, and the obtained residue was purified by thin layer silica gel chromatography (chloroform/methanol=9/1) to give the title compound (287 mg) as a white powder.
¹H-NMR(CD₃OD) δ(ppm): 1.94-2.48(4H,m) 2.68-2.70(3H,m), 3.55-3. 84(2H,m), 3.89-4.07(2H,m), 5.29-5.37(1H,m), 7.11-7.12(1H,m), 7. 32-7.36(1H,m).
ESI/MS(m/z): 412(M+H)⁺, 410(M-H)⁻.

### Example 11

### (-)-2-Methyl-5-[1-(2-ureidoacetyl)pyrrolidin-2-yl]thiophene -3-carboxylic acid (5-chlorothiazol-2-yl)amide

The title compound was obtained by conducting a reaction in a similar method to that of Example 10 by using (-)-2-methyl-5-pyrrolidin-2-ylthiophene-3-carboxylic acid (5-chlorothiazol-2-yl)amide instead of (-)-2-methyl-5-pyrrolidin-2-ylthiophene-3-carboxylic acid (5-fluorothiazol-2-yl)amide.
¹H-NMR(CD₃OD) δ(ppm): 1.94-2.46(4H,m), 2.70-2.72(3H,m), 3.54-3. 84(2H,m), 3.84-4.06(2H,m), 5.27-5.37(1H,m), 7.34-7.35(1H,m), 7. 39(1H,s).
ESI/MS(m/z): 428(M+H)⁺, 426(M-H)⁻.

### Example 12

### (-)-5-[(2S,4R)-4-Fluoro-1-(2-methanesulfonylaminoacetyl)pyr rolidin-2-yl]-2-methylthiophene-3-carboxylic acid (5-fluorothiazol-2-yl)amide

5-((2*S*,4*R*)-4-Fluoropyrrolidin-2-yl)-2-methylthiophene-3-ca rboxylic acid (5-fluorothiazol-2-yl)amide (297 mg), 1-ethyl-3-(3-dimethylaminopropyl)carbodiimide hydrochloride (345 mg), 1-hydroxy-1H-benzotriazole monohydrate (276 mg) and *N*-methanesulfonylglycine (276 mg) were dissolved in *N*,*N*-dimethylformamide (3.0mL), diisopropylethylamine (308 µL) was added thereto, and stirring was conducted at room temperature for 5.5 hours. Water was added to the reaction liquid, extraction was conducted by using ethyl acetate, and the organic layer was washed with a saturated aqueous sodium hydrogen carbonate solution and dried over anhydrous sodium sulfate. Filtration was conducted, and the filtrate was then concentrated under a reduced pressure and recrystallized from ethyl acetate to give the title compound (291 mg) as a white powder.
¹H-NMR(CDCl₃) δ(ppm): 2.10-2.18(1H,m), 2.59(3H,s), 3.00(3H,s), 3 .80-4.02(4H,m), 5.26-5.47(2H,m), 7.04-7.07(1H,m), 7.56(1H,s), 9 .70(1H,br.s).
ESI/MS(m/z): 465(M+H)⁺, 463(M-H)⁻.

Compounds were synthesized according to the following reaction formula with referring to the method of Example 12.
The synthesized compounds are shown in Table 19, and the data are shown in Table 20.

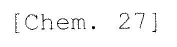

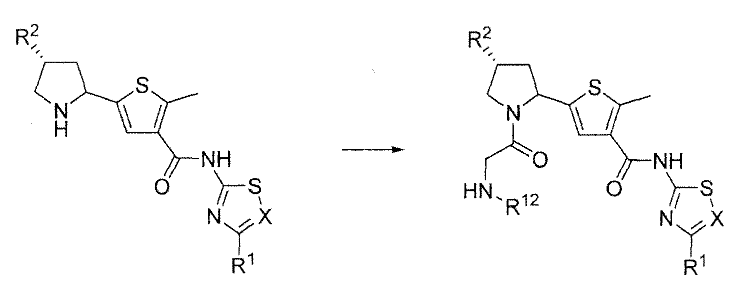

**[Table 19]**

| Example | Starting raw material | R¹ | R² | R¹² | X |
|---|---|---|---|---|---|
| 13 | Reference Example 27 | H | F | Es | C-F |
| 14 | Reference Example 28 | H | F | Ms | C-Cl |
| 15 | Reference Example 28 | H | F | Ac | C-Cl |
| 16 | Reference Example 30 | H | H | Ms | C-F |
| 17 | Reference Example 31 | H | H | Ms | C-Cl |
| 18 | Reference Example 31 | H | H | Ac | C-Cl |
| 19 | Reference Example 33 | H | H | Es | N |
| 20 | Reference Example 35 | SMe | H | Es | N |
| 21 | Reference Example 36 | OMe | H | Es | N |

**[Table 20]**

| Example | ¹H-NMR(CDCl₃)δ(ppm) | ESI/MS(m/z) |
|---|---|---|
| 13 | 1.32-1.45(4H,m),2.61(3H,s).3.05-3.12(2H,m),3.61-3,68(2H,m),3.80-4.23 (4H,m),5.27-5,48(1H,m),7,04-7.07(2H,m),7.56(1H,s).9,70(1H,br.s). | 479(M+H)⁺ 477(M-H)⁻ |
| 14 | 2.24-2.38(1H,m),2.67-2.95(7H,m),3.59-4.28(4H,m),5.26-5.45(2H,m),7.3 6-7.44(2H,m). (CD₃OD) | 481(M+H)⁺ 479(M-H)⁻ |
| 15 | 1.99(3H,s),2.25-2.38(1H,m),2.68-2.88(4H,m),3.59-4.27(4H,m),5.25-5.49 (2H,m),7.35-7.46(2H,m).(CD₃OD) | 445 (M+H)⁺ 443(M-H)⁻ |
| 16 | 1.98-2.35(5H,m),2.63(3H,s),3.02(3H,s),3.48-3.81(3H,m),5.35(1H,d,*J*=4. 8Hz),7.02-7.05(1H,m),7.33-7.36(1H,m). | 447 (M+H)⁺ 445(M-H)⁻ |
| 17 | 2.00-2.43(4H.m),2.67(3H,s),2.96(3H,s),3.55-3.75(2H,m),4.02-4.08(2H, m),5.29-5.35(1H,m),7.25-7.35(2H,m).(CD₃OD) | 463 (M+H)⁺ 461(M-H)⁻ |
| 18 | 2.07-2.18(7H,m),2.22-2.31(1H,m).2.70(3H,d,*J*=15.7Hz),3.50-3.70(2H,m ),3.91-3.96(1H,m),4.31-4.38(1H,m),5.33(1H,d,*J*=7.3Hz),7.17(1H,s),7.33 -7.57(1H,m),10.85(1H,br.s). | 427(M+H)⁺ 425(M-H)⁻ |
| 19 | 1.34-1.42(3H,m),1.90-2.01(2H,m),2.06-2.18(2H,m),2.24-2.34(1H,m),2.6 0(3H,s),3.04-3.12(2H,m),3.47-3.62(1H,m),3.74-3.80(1H,m),4.02-4.08(1 H,m),5.38-5.43(1H,m),6.61(1H,br.s),7.47(1H,s),8.32(1H,s),10.83(1H,br. s). | 444(M+H)⁺ 442(M-H)⁻ |
| 20 | 1.30-1.39(3H,m),1.88-1.95(1H,m).2.06-2.18(2H,m),2.24-2.35(1H,m),2.5 9(3H,s),2.67(3H,s),3.03-3.14(2H,m),3.42-3.56(1H,m),3.74-3.82(1H,m),4 .11-4.37(1H,m),5.39-5.44(1H,m),5.88-5.96(1H,m),7.51(1H,s),7.47(1H,s) 10.48(1H,br.s). | 490(M+H)⁺ 488(M-H)⁻ |
| 21 | 1.30-1.39(3H,m),1.87-1.95(1H,m),2.04-2.21(2H,m),2.26-2,36(1H,m),2.5 9(3H,s),3.06-3.14(2H,m),3.72-3.79(2H,m),4.04(3H,s),4.05-4.10(1H,m),4 .16-4.19(1H,m),5.36-5.40(1H,m),6.20-6.26(1H,m),7.49(1H,s),10.53(1H, br.s). | 474(M+H)⁺ 472(M-H)⁻ |

### Example 22

### (-)-2-Methyl-5-[1-((S)-2-ureidoropionyl)pyrrolidin-2-yl]th iophene-3-carboxylic acid (5-fluorothiazol-2-yl)amide

(-)-5-[1-((*S*)-2-Aminopropionyl)pyrrolidin-2-yl]-2-methylth iophene-3-carboxylic acid (5-fluorothiazol-2-yl)amide (60.0 mg) was dissolved in water (1.8 mL), potassium cyanate (62.3 mg) and acetic acid (79.9 µL) were added thereto, and stirring was conducted at room temperature for 3 hours. A saturated aqueous sodium hydrogen carbonate solution was added to the reaction liquid, extraction was conducted by using chloroform, and the organic phase was dried over anhydrous sodium sulfate. Filtration was conducted, and the filtrate was then concentrated under a reduced pressure to give the title compound (63.5 mg) as a white powder.
¹H-NMR(CD₃OD) δ:1.24-1.40(3H,m), 1.85-2.46(4H,m), 2.61-2.74(3H ,m), 3.64-3.92(2H,m) ,4.45-4.60(1H,m), 5.24-5.41(1H,m), 7.11-7. 12(1H,m), 7.24-7.27(1H,s).
ESI/MS(m/z): 426(M+H)⁺, 424(M-H)⁻.

### Example 23

### (-)-5-[(2S,4R)-1-((S)-2-Acetylaminopropionyl)-4-fluoropyrro lidin-2-yl]-2-methylthiophene-3-carboxylic acid (5-fluorothiazol-2-yl)amide

(-)-5-[(2*S*,4*R*)-1-((*S*)-2-Aminopropionyl)-4-fluoropyrrolidin -2-yl]-2-methylthiophene-3-carboxylic acid (5-fluorothiazol-2-yl)amide (31.8 mg) was dissolved in methylene chloride (1.0 mL), acetyl chloride (11.3 µL) and pyridine (19.3 µL) were added thereto, and stirring was conducted at room temperature for 45 minutes. A saturated aqueous sodium hydrogen carbonate solution was added to the reaction liquid, extraction was conducted by using ethyl acetate, and the organic layer was washed with saturated brine and dried over anhydrous sodium sulfate. Filtration was conducted, the filtrate was then concentrated under a reduced pressure, and the obtained residue was purified by thin layer silica gel chromatography (ethyl acetate) to give the title compound (27.8 mg) as a white powder.
¹H-NMR(CDCl₃) δ(ppm): 1.29-1.33(3H,m), 2.6-7(3H,d,*J*=6.2Hz), 2.74 -2.79(1H,m), 3.85-3.98(1H,m), 4.27-4.35(1H,m), 4.58-4.66(2H,m) ,5.31-5.49(2H,m), 7.13(1H,d,*J*=2.5Hz), 7.32(1H,s). ESI/MS(m/z): 443(M+H)⁺, 441(M-H)⁻.

Compounds were synthesized according to the following reaction formula with referring to the method of Example 23.
The synthesized compounds are shown in Table 21, and the data are shown in Table 22.

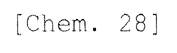

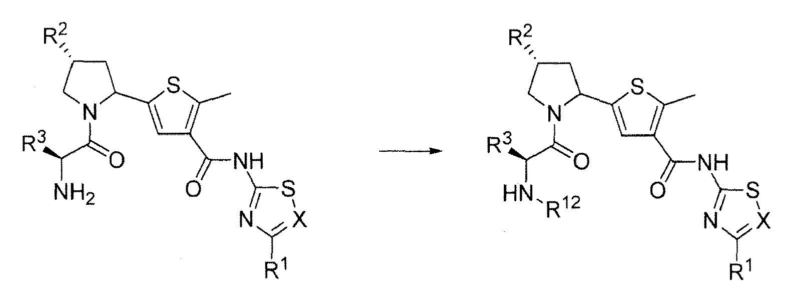

**[Table 21]**

| Example | Starting raw material | R¹ | R² | R³ | R¹² | X |
|---|---|---|---|---|---|---|
| 24 | Reference Example 136 | H | F | Me | Ms | C-F |
| 25 | Reference Example 143 | Et | F | Me | Ms | N |
| 26 | Reference Example 144 | Et | F | Et | Es | N |
| 27 | Reference Example 145 | Et | F | *i*-Pr | Ms | N |
| 28 | Reference Example 150 | H | H | *n*-Pr | Ms | C-F |
| 29 | Reference Example 153 | H | H | Me | Ms | N |
| 30 | Reference Example 154 | Et | H | Me | Es | N |
| 31 | Reference Example 154 | Et | H | Me | Ac | N |
| 32 | Reference Example 155 | H | H | H | EtCO | C-F |

**[Table 22]**

| Example | ¹H-NMR(CDCl₃)δ(ppm) | ESI/MS (m/z) |
|---|---|---|
| 24 | 1.38-1.46(3H,m),2.12-2.29(1H,m),2.69(3H,s),2.92(3H,s),3.57-3.85(1H,m), 4.05-4.16(1H,m),4.26-4.46(1H,m),5.18-5.47(2H,m),5.52-5.54(1H,m),6.81-6.88(1H,m),7.06(1H,s),7.38(1H,s),10.08(1H,br.s). | 479(M+H)⁺ 477(M-H)⁻ |
| 25 | 1.32-1.41(3H,m),1.55(3H,d,*J*=7.0Hz),2.10-2.26(1H,m),2.68(3H,s),2.84-2.9 8(4H,m),3.78-3.93(1H,m),4.18-4.26(1H,m),4.45-4.56(1H,m),5.30-5.63(2H, m),7.27(1H,s),7.75(1H,s),8.40(1H,d,*J*=9.2Hz), 11.35(1H,br.s). | 490(M+H)⁺ 488(M-H)⁻ |
| 26 | 1.05-1.38(11H,m),1.65-1.92(3H,m),2.08-2.27(1H,m)2.70(3Hs),2.75-3.05( 6Hm),3.78-3.92(1H,m),4.17-4.32(1H,m),5.31-5.34(1H,m),5.61-5.67(1N,m ),7.32-7.43(1H,m),7.83(1H,s),8.09(1H,d,*J*=9.9Hz),11.52(1H,br.s). | 518(M+H)⁺ 516(M-H)⁻ |
| 27 | 0.95-0.99(3H,m), 1.09-1,13(3H,m),1.33-1.39(3H,m),1.68-1.79(1H,m),2.08-2.34(2H,m),2.75(3H,s),2.81(3H,s),2.84-2.95(3H,m),3.78-3.92(1H,m),4,16-4.32(1H,m),5.31-5.35(1H,m),5.60-5.69(1H,m),7.82(1H,s),7.91(1H,d,*J*=9.9 Hz),11.31(1H,br.s). | 518(M+H)⁺ 516 (M-H)⁻ |
| 28 | 0.96-0.99(3H,m),1.50-1.69(2H,m),2.01-2.31(4H,m),2.64(3H,s),2.93(3H,s), 3.55-3.79(2H,m),4.16-4.32(2H,m),5.42-5.47(1H,m),7.06-7.12(1H,m),7.28-7.32(1H,m),10.59(1H,br.s). | 489(M+H)⁺ 487(M-H)⁻ |
| 29 | 1.51(3H,d,*J*=7.0Hz),1.58-1.76(3H,m),2,06-2.22(3H,m),2.66(3H,s),2.99(3H, s),3.52-3.82(1H,m),4.41-4.48(1H,m),5.50(1H,d,*J*=7.7Hz),7.53(1H,s),7.97(1 H,s),11.77(1H,br.s). | 444(M+H)⁺ 442(M-H)⁻ |
| 30 | 1.32-1.43(6H,m),1.51(3H,d,*J*=7.3Hz),2.14(3H,s),2.65(3H,s),2.85-3.08(5H, m),3.65-3.78(1H,m),4,29-4.40(1H,m),5.51(1H,d,*J*=7.3Hz),7.55(1H,s),8.05( 1H,s),11.63(1H,br.s). | 486(M+H)⁺ 484(M-H)⁻ |
| 31 | 1.39(4H,t,*J*=7.5Hz),1.48(3H,d,*J*=7.3Hz),1.95(3H,s),2.05-2.35(5H,m),2.90( 3H,s),3.68-3.90(2H,m),4.95-5.04(1H,m),5.47(1H,d,*J*=17.0Hz),7.74(1H,s),9. 06(1H,d,*J*=8.8Hz),12.02(1H,br.s). | 436(M+H)⁺ 434(M-H)⁻ |
| 32 | 1.95-2.31(4H,m),2.72(3H,br.s),3.41-3.77(4H,m),5.20(1H,d,*J*=7.2Hz),6.97(1H,d,*J*=2.7Hz),7.20(1H,br.s). | 425(M+H)⁺ 423 (M-H)⁻ |

### Example 33

### (-)-(2-{(2S,4R)-4-Fluoro-2-[4-(5-fluorothiazol-2-ylcarbamoy l)-5-methylthiophen-2-yl]pyrrolidin-1-yl)-2-oxoethylamino)a cetic acid trifluoroacetate

(-)-(2-{(2*S*,4*R*)-4-Fluoro-2-[4-(5-fluorothiazol-2-ylcarbamo yl)-5-methylthiophen-2-yl]pyrrolidin-1-yl}-2-oxoethylamino) acetic acid *t*-butyl ester (19.0 mg) was dissolved in methylene chloride (0.25 mL), trifluoroacetic acid (0.25 mL) was added thereto, and stirring was conducted overnight at room temperature. The reaction liquid was concentrated under a reduced pressure, and the obtained residue was dissolved in water (1.0 mL), and the solution was lyophilized to give the title compound (13.0 mg) as a white powder.
¹H-NMR(DMSO-d₆) δ(ppm): 2.18-2.42(2H,m), 2.64-2.83(4H,m), 3.75-3.97(4H,m), 4.13-4.23(1H,m), 5.30-5.61(2H,m), 7.34-7.39(1H,m), 7.63(1H,s), 9.25(1H,br.s), 12.21(1H,br.s).
ESI/MS(m/z): 445(M+H)⁺, 443(M-H)⁻.

Compounds were synthesized according to the following reaction formula with referring to the method of Example 33.
The synthesized compounds are shown in Table 23, and the data are shown in Table 24.

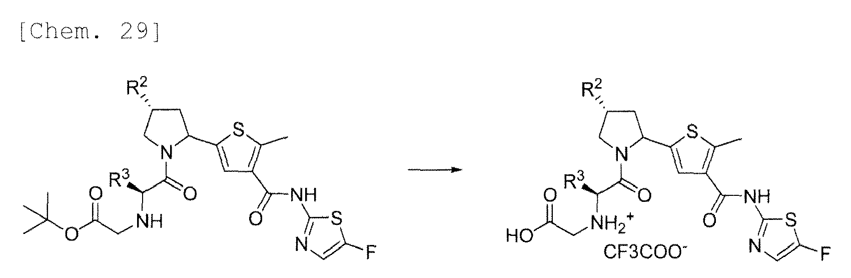

**[Table 23]**

| Example | Starting raw material | R² | R³ |
|---|---|---|---|
| 34 | Reference Example 163 | F | *i*-Pr |
| 35 | Reference Example 170 | H | Me |
| 36 | Reference Example 171 | H | Et |
| 37 | Reference Example 172 | H | *i*-Pr |

**[Table 24]**

| Example | ¹H-NMR(CD₃OD)δ(ppm) | ESI/MS(m/z) |
|---|---|---|
| 34 | 1.03-1.22(6H,m),2.26-2.48(2H,m),2.71(3H,s),2.75-2.85(1H,m),3.78-3. 93(3H,m),4.09-4.21(2H,m),4.38-4.40(1H,m),5.30-5.59(2H,m),7.15(1H ,d,*J*=4.7Hz),7.44-7.51(1H,m). | 487(M+H)⁺ 485(M-H)⁻ |
| 35 | 1.58(3H,t,*J*=6.6Hz),2.01-2.35(4H,m),2.69(3H,s),3.51-3.93(4H,m),4.09 -4.37(1H,m),5.35(1H,br.s),7.14(1H,d,*J*=2.6Hz),7.32(1H,br.s). | 441(M+H)⁺ 439(M-H)⁻ |
| 36 | 0.87-0.99(3H,m),1.83-2.27(6H,m),2.63(3H,s),3.45-3.92(4H,m),4.22(1 H,s),5.29-5.32(1H,m),7.37-7.38(1H,m),7.57(1H,s),9.21(1H,br.s),12.23 (1H,br.s). (DMSO-d₆) | 455(M+H)⁺ 453(M-H)⁻ |
| 37 | 1.04-1.24(6H,m),2.01-2,41(5H,m),2.70(3H,s),3.33-3.40(1H,m),3.70-3. 96(4H,m),4.04-4.28(1H,m),5.37(1H,d,*J*=16.0Hz),7.14(1H,d,*J*=2.7Hz), 7.36(1H,d,*J*=16,1Hz). | 469(M+H)⁺ 467(M-H)⁻ |

### Example 38

### [(2-{(2S,4R)-4-Fluoro-2-[4-(5-fluorothiazol-2-ylcarbamoyl)-5-methylthiophen-2-yl]pyrrolidin-1-yl}-2-oxoethyl)methylami no]acetic acid hydrochloride

[(2-{(2*S*,4*R*)-4-Fluoro-2-[4-(5-fluorothiazol-2-ylcarbamoyl) -5-methylthiophen-2-yl]pyrrolidin-1-yl}-2-oxoethyl)methylam ino]acetic acid *t*-butyl ester (59.0 mg) was dissolved in methylene chloride (3.0 mL), a 4 N-hydrochloric acid/1,4-dioxane solution (3.42 mL) was added thereto, and stirring was conducted at room temperature for 18 hours. Diethyl ether was added to the reaction liquid, and the precipitated crystal was collected by filtration and washed with diethyl ether to give the title compound (52.0 mg) as a white powder.
¹H-NMR(CD₃OD) δ(ppm): 2.27-2.43(1H,m), 2.68(3H,s), 2.75-2.84(1H ,m), 2.87-3.12(4H,m), 3.43-3.52(1H,m), 3.73-3.94(2H,m), 4.06-4. 2.1(2H,m), 5.28-5.51(2H,m), 7.16(1H,s), 7.39(1H,s).
ESI/MS(m/z): 459(M+H)⁺, 457(M-H)⁻.

Compounds were synthesized according to the following reaction formula with referring to the method of Example 38. The synthesized compounds are shown in Table 25, and the data are shown in Tables 26 to 28.

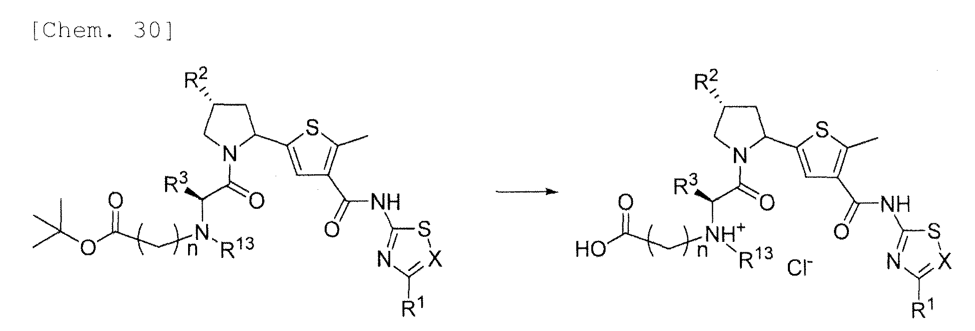

**[Table 25]**

| Example | Starting raw material | R¹ | R² | R³ | R¹³ | X | n |
|---|---|---|---|---|---|---|---|
| 39 | Reference Example 109 | H | F | H | Et | C-Cl | 1 |
| 40 | Reference Example 110 | H | H | H | Et | C-Cl | 1 |
| 41 | Reference Example 111 | H | H | H | Et | C-H | 1 |
| 42 | Reference Example 112 | H | F | H | Et | N | 1 |
| 43 | Reference Example 113 | H | H | H | Et | C-F | 2 |
| 44 | Reference Example 114 | H | F | H | Et | C-H | 2 |
| 45 | Reference Example 115 | H | H | H | Et | C-Cl | 2 |
| 46 | Reference Example 116 | H | H | H | *i*-Pr | C-F | 2 |
| 47 | Reference Example 117 | H | H | H | Et | N | 1 |
| 48 | Reference Example 118 | H | H | Et | Et | C-F | 1 |
| 49 | Reference Example 119 | H | H | Et | Et | C-F | 1 |
| 50 | Reference Example 120 | H | H | *i*-Pr | Et | C-F | 1 |
| 51 | Reference Example 121 | H | H | *i*-Pr | Et | C-F | 1 |
| 52 | Reference Example 122 | H | H | Me | Et | C-F | 1 |
| 53 | Reference Example 123 | H | F | Me | Et | C-F | 1 |
| 54 | Reference Example 124 | H | H | Me | Et | C-F | 1 |
| 55 | Reference Example 125 | H | F | Me | Et | C-F | 1 |
| 56 | Reference Example 126 | H | H | n-Pr | Et | C-F | 1 |
| 57 | Reference Example 127 | H | F | *n*-Pr | Et | C-F | 1 |
| 58 | Reference Example 128 | H | H | *n*-Pr | Et | C-F | 1 |
| 59 | Reference Example 129 | H | F | *n*-Pr | Et | C-F | 1 |
| 60 | Reference Example 130 | H | H | Me | Et | C-F | 2 |
| 61 | Reference Example 131 | H | H | Me | Et | C-F | 2 |
| 62 | Reference Example 132 | H | H | H | *i*-Bu | C-F | 1 |
| 63 | Reference Example 165 | H | F | H | Et | C-F | 1 |
| 64 | Reference Example 166 | H | F | H | *n*-Pr | C-F | 1 |
| 65 | Reference Example 168 | H | F | Me | Me | C-F | 1 |
| 66 | Reference Example 139 | Et | F | H | Et | N | 1 |
| 67 | Reference Example 173 | H | H | H | Et | C-F | 1 |
| 68 | Reference Example 174 | H | H | H | *n*-Pr | C-F | 1 |
| 69 | Reference Example 176 | H | H | H | Me | C-F | 2 |
| 70 | Reference Example 177 | H | H | H | Me | C-F | 2 |
| 71 | Reference Example 178 | H | H | H | Me | C-Cl | 2 |
| 72 | Reference Example 179 | Et | H | H | Me | N | 2 |
| 73 | Reference Example 180 | H | H | H | H | C-Cl | 1 |
| 74 | Reference Example 181 | H | H | H | H | C-F | 3 |
| 75 | Reference Example 182 | H | H | H | H | C-Cl | 2 |
| 76 | Reference Example 184 | H | H | H | Me | C-Cl | 3 |
| 77 | Reference Example 186 | SMe | H | H | Me | N | 1 |

**[Table 26]**

| Example | ¹H-NMR (CD₃OD)δ(ppm) | ESI/MS(m/z) |
|---|---|---|
| 39 | 0.90-1.22(3H,m),2.28(1H,m),2.64-2.75(3H,m),3.2-4.5(9H,m),5.30-5.62(2H, m),7.57-7.59(2H,m),12.4(1H,br.s). (DMSO-*d*₆) | 489[M+H]⁺ 487[M-H]⁻ |
| 40 | 1.20-1.23(3H,m),1.91-2.33(4H,m),2.64(3H,s),3.28-3.70(4H,m),4,09(2H,m). 4.28(2H,m),5.26(1H,d,76.6Hz),7.57-7.59(2H,m),12.4(1H,br.s). (DMSO-*d*₆) | 471[M+H]⁺ 469[M-H]⁻ |
| 41 | 1.20-1.23(3H,m),1.99-2.33(4H,m),2.65(3H,s),3.05-3.85(4H,m),4.02-4.10(2 H,m),4.29-4.44(2H,m),5.27(1H,d,*J*=6.6Hz),7.26-7.27(1H,m),7.55-7.57(2H, m). (DMSO-*d*₆) | 437[M+H]⁺ 435[M-H]⁻ |
| 42 | 1.0-1.22(3H,m),2.28(1 H,m),2.60-2,75(3H,m),3.2-4.5(9H,m),5.30-5.66(2H, m),7.77(1H,s),8.53(1H,s),13.0(1H,br.s).(DMSO-*d*₆) | 456[M+H]⁺ 454[M-H]⁻ |
| 43 | 1.32-1.40(3H,m),2.02-2.24(3H,m),2.32-2.41(1H,m),2.70(3H,s),2.82-2.91(2 H,m),3.46-3.68(3H,m),3.71-3.82(2H,m),4.28-4.42(2H,m),5.28-5.42(1H,m), 7.17-7.21(1H,m),7.34-7.39(11H,m). | 469[M+H]⁺ 467[M-H]⁻ |
| 44 | 1.27-1.39(3H,m),2.28-2.46(2H,m),2.68(3H,s),2,75-2.89(3H,m),3.42-3.56(2 H,m),3.82-4.11(2H,m),4,32-4.45(4H,m),5.29-5.54(3H,m),7.14-7.18(2H,m). | 487 [M+H]⁺ 485[M-H]⁻ |
| 45 | 1.16-1.22(3H,m),1.90-1.99(2H,m),2.09-2.33(4H,m),2.50-2.78(7H,m),3.19-3 .63(2H,m),4.24(2H,m),5.26(1H,d,*J*=6.6Hz),7.59(2H,s),9.47(1H,br,s), 12.4(1H,br.s).(DMSO-*d*₆) | 485[M+H]⁺ 483 [M-H]⁻ |
| 46 | 1.32-1.44(6H,m),2.05-2.28(3H,m),2.30-2.50(1H,m),2.66(3H,s),2.72-2.86(2 H,m),3.41-3.82(6H,m),4.12-4.31(2H,m),5.34-5.40(1H,m),7.14-7.18(1H,m), 7.32-7.36(1H,M). | 483 [M+H]⁺ 481 [M-H]⁻ |
| 47 | 1.20-1.23(3H,m),1.90-2.33(4H,m),2.68(3H,s),3.28-3.72(4H,m),4.04-4.09(2 H,m),4.29-4.45(2H,m),5.27(1H,d,*J*=6.6Hz),7.68(1H,s),8.53(1H,s),13.1(1H, br.s).(DMSO-*d*₆) | 438 [M+H]⁺ 436 [M-H]⁻ |
| 48 | 0.99(3H,t,*J*=7.6Hz),1.37(3H,t,*J*=7.2Hz),1.99-2.18(4H,m),2.25-2.41(2H,m), 2.68(3H,s),3.36-3.41(2H,m),3.48-3.66(1H,m),3.78-3.88(2H,m),4.06-4.13(1 H,m),4.53(1H,s),5.45(1H,d,*J*=7.2Hz),7.19(1H,d,*J*=2.4Hz),7.38(1H,s). | 483 [M+H]⁺ 481 [M-H]⁻ |
| 49 | 1.07(3H,t,*J*=7.2Hz),1.33(3H,t,*J*=7.6Hz),1.99-2.16(4H,m),2.24-2.39(2H,m), 2.68(3H,s),3.30-3.41(3H,m),3.46-3.57(1H,m),3.68-3.77(1H,m),3.94-3.98(1 H,m),4.19(1H,d,*J*=7.6Hz),5.40(1H,d,*J*=7.2Hz),7.21(1H,d,*J*=2.8Hz),7.40(1H ,s). | 483 [M+H]⁺ 481 [M-F]⁻ |
| 50 | 0.96(3H,d,*J*=6.6Hz),1.15(3H,d,*J*=7.0Hz),1.36(3H,t,*J*=7.3Hz),2.11-2.20(2H, m),2.28-2.34(1H,m),2.35-2.45(2H,m),2.68(3H,s),3.28-3.39(3H,m),3.46-3.5 1(2H,m),3.79-3.84(2H,m),5.45-5.48(1H,m),7.15(1H,d,*J*=2.6Hz),7.40(1H,s). | 497 [M+H]⁺ 495 [M-H]⁻ |
| 51 | 1.11(3H,d,*J*=6.6Hz),1.17(3H,d,*J*=7.0Hz),1.31(3H,t*,J*=7.3Hz),2.13-2.14(2H, m),2.29-2.38(2H,m),2.39-2.47(1H,m),2.67(3H,s),3.23-3.31(2H,m),3.43-3.4 9(2H,m),3.75-3.79(1H,m),3.88-3.98(1H,m),4.00(1H,d,*J*=17.9Hz),5.40-5.41 (1H,m),7.15(1H,d,*J*=2.6Hz),7.36(1H,s). | 497 [M+H]⁺ 495 [M-H]⁻ |
| 52 | 1.37(3H,t,*J*=7.2Hz),1.62(3H,d,*J*=6.8Hz),2.08-2.44(5H,m),2.68(3H,s),3.35-3 .62(2H,m),3.75-3.88(2H,m),4.07(1H,d,*J*=17.2Hz),4.61(1H,q,*J*=6.8Hz),5.42 (1H,d,*J*=7.6Hz),7.21(1H,d,*J*=2.8Hz),7.32(1H,s). | 469 [M+H]⁺ 467 [M-H]⁻ |

**[Table 27]**

| Example | ¹H-NMR (CD₃OD)δ(ppm) | ESI/MS (m/z) |
|---|---|---|
| 53 | 1.22(3H,t,*J*=7.0Hz),1.43(3H,d,*J*=7.0Hz),2.2-2.8(3H,m),2.64(3H,s),3.15-3.4(2 H,m),3.7-4.3(3H,m),4.5-4.6(2H,m),5.3-5.6(2H,m),7.34(1H,*J*=2.6Hz),7.57(1 H,s),12.11(1H,br.s). | 487 [M+H]⁺ 485 [M+H]⁻ |
| 54 | 1.35(3H,t,*J*=7.2Hz),1.62(3H,d,*J*=7.2Hz),2.08-2.47(5H,m),2.68(3H,s),3.46-3. 74(3H,m),3.91-3.95(1H,m),4.07-4.12(1H,m),4.64-4.69(1H,m),5.34(1H,d,*J*=6 .4Hz),7.22(1H,d,*J*=2.0Hz),7.39(1H,s). | 469 [M+H]⁺ 467 [M-H]⁻ |
| 55 | 1.1-1.2(3H,m),1.35-1.45(3H,m),2.3-2.8(3H,m),2.63(3H,s),3.01-3.08(2H,m),3 .7-4.3(5H,m),5.3-5.8(2H,m),7.34(1H,*J*=2.2Hz),7.62(1H,s). | 487 [M+H]⁺ 485 [M+H]⁻ |
| 56 | 0.97(3H,t,*J*=7.3Hz),1.39(3H,t,*J*=7.1Hz),1.82-2.56(7H,m),2.66-2.79(3H,m),2. 89-3.02(1H,m),3.4*8*-3.72(2H,m),3.72-3.94(2H,m),4.08-4.48(2H,m),4.52-4.61 (1H,m),5.43-5.52(1H,m),7.14-7.23(1H,m),7.36(1H,s). | 497 [M+H]⁺ 495 [M-H]⁻ |
| 57 | 0.84(3H,t,*J*=7.1Hz),1.18(3H,t,*J*=7.4Hz),1.15-1.85(4H,m),2.64(3H,s),3.1-3.7( 6H,m),3.7-4.0(1H,m),4.0-4.3(1H,m),5.3-5.6(2H,m),7.34(1H,d,*J*=2.6Hz),7.62 (1H,s),12.11(1H,br.s). | 515 [M+H]⁺ 513 [M+H]⁻ |
| 58 | 1.03(3H,t,*J*=7.3Hz),1.28-1.59(5H,m),1.88-2.54(7H,m),2.66-2.76(3H,m),3.64 -3.83(1H,m),3.88-3,96(2H,m),4.12-4.33(2H,m),4.56-4.68(1H,m),5.34-5.44(1 H,m),7.12-7.19(1H,m),7.35(H,s). | 497 [M+H]⁺ 495 [M-H]⁻ |
| 59 | 0.89(3H,t,*J*=7.3Hz),1.05-1.15(3H,m),1.2-1.4(2H,m),1.7-1.8(2H,m),2.63(3H,s ),3.6-4.0(6H,m),4.05-4.2(2H,m),5.3-5,85(2H,m),7.34(1H,*J*=2.6Hz),7.62(1H,s ). | 515 [M+H]⁺ 513 [M+H]⁻ |
| 60 | 1.30-1.41(3H,m),1.51-1.64(3H,m),1.95-2.58(7H,m),2.63-2.74(3H,m),2.78-2.92(1H,m),3.36-3.90(4H,m),4.36-4.46(1H,m),5.37-5.46(1H,m),7.12-7.18(1H, m),7.29(1H,s). | 483 [M+H]⁺ 481 [M-H]⁻ |
| 61 | 1.08-1.41(3H,m),1.51-1.64(3H,m),1.94-2.54(6H,m),2.62-2.94(5H,m),3.36-3. 79(3H,m),3.87-4.01(1H,m),4.32-4.46(1H,m),5.32-5.41(1H,m),7.16(1H,m),7. 36(1H,s). | 483 [M+H]⁺ 481 [M-H]⁻ |
| 62 | 0.92-1.06(6H,m),2,02-2.18(3H,m),2.25-2.37(1H,m),2.70(3H,s),3.13-3.24(3H ,m),3.52,3.61(1H,m),3.74-3.79(1H,m),4.12-4.56(4H,m),5.40(1H,d,*J*=7.3Hz), 7.19(1H,s),7.35(H,s). | 483 [M+H]⁺ 481 [M-H]⁻ |
| 63 | 1.32-1.38(3H,m),2.31-2.34(1H,m),2.70(3H,s),2.75-2.91(1H,m),3.38-3.42(2H ,m),3.71-3.95(2H,m),4.01-4.53(4H,m),5.29-5*.*49(2H,m),5.47(2H,t,*J*=8.1Hz), 7.16-7.22(1H,m),7.45(1H,br.s). | 473 (M+H)⁺ 471 (M-H)⁻ |
| 64 | 0.98(3H,t,*J*=7.0Hz),1.52-1.80(2H,m),2.26-2.45(1H,m),2.71(3H,d,*J=*24.2Hz), 2.75-2.90(1H,m),3.18-3.27(1H,m),3.79-4.54(7H,m),5.31-5.50(2H,m),7.17(1 H.br.d,*J*=4.8Hz),7.37(1H,s). | 487 [M+H]⁺ 485 [M-H]⁻ |
| 65 | 1.56(3H,t,*J*=13.5Hz),2.27-2.43(H,m),2.68(3H,s),2.72-2.84(2H,m),3.03-3.06 (3H,m),3.87-4.14(3H,m),4.62-4.73(1H,m),5.28-5.54(2H,m),7.17(1H,s),7.39( 1H,s). | 473 (M+H)⁺ 471 (M-H)⁻ |
| 66 | 1.30-1.38(6H,m),2.28-2.41(1H,m),2.72(3H,s),2.81-2.88(3H,m),3.74-4.00(2H ,m),4.06-4.52(4H,m),5.30-5.51(2H,m),5.47(2H,t,*J*=8.1Hz),7.46(1H,s),7.55(1 H,s). | 484 (M+H)⁺ 483 (M-H)⁻ |

**[Table 28]**

| Example | ¹H-NMR(CD₃OD)δ(ppm) | ESI/MS(m/z) |
|---|---|---|
| 67 | 1.37(1H,br.t,*J=*6.6Hz),2.06-2.09(3H,m),2.31-2.34(1H,m),2.70(3H,s),3.42-3.50( 2H,m),3.76-3.80(2H,m),4.10-4.58(4H,m),5.40(1H,d,*J=*7.7Hz),7.27(1H,br.s),7.4 2(1H,br.s). | 455 (M+H)⁺ 453 (M-H)⁻ |
| 68 | 0.90-1.04(3H,m),1.60-1.82(2H,m),2.02-2.16(3H,m),2.28-2.48(1H,m),2.71(3H,d ,*J=*7.7Hz),3.26-3.30(2H,m),3.54-3.84(2H,m),4.09-4.56(4H,m),5.40(1H,d,*J=*7.0 Hz),7.18(1H,d,*J=*11.3Hz),7.33(1H,d,*J=*16.8Hz). | 469 [M+H]⁺ 467 [M-H]⁻ |
| 69 | 2.07-2.11(2H,m),2.70(3H,s),2.85-2.95(3H,m),2.99(2H,d,*J=*2.9Hz),3.46-3.75(7H ,m),4.23-4.43(2H,m),5.36(1H.d,*J=*7.4Hz),7.20-7.22(1H,m),7.37(1H,d,*J=*6.6Hz). | 455 (M+H)⁺ 453 (M-H)⁺ |
| 70 | 1.99-2.33(4H,m),2.63(3H,s),2.68-2.48(3H,m),3.32-3.75(7H,m),4.21-4.35(2H,m) ,5.29(1H,d,*J=*7.3Hz),7.36-7.37(1H,m),7.52(1H,d,*J=*8.8Hz). | 455 (M+H)⁺ 453 (M-H)⁻ |
| 71 | 2.07-2.11(2H,m),2.70(3H,s),2,85-2.95(3H,m),2.99(2H,d,*J=*2.9Hz),3.46-3.75(7H ,m),4.23-4.43(2H,m),5.36(1H,d,*J=*7.4Hz),7.20-7.22(1H,m),7.37(1H,d,*J=*6.6Hz). | 471 (M+H)⁺ 469 (M-H)⁻ |
| 72 | 1.34(3H,t,*J=*7.5Hz),2.00-2.33(5H,m),2.72(3H,s),2.82-2.95(5H,m),2.95-3.06(2H, m),3.52-3.83(3H,m),4.18-4.40(2H,m),5.41(1H,d,*J=*6.6Hz),7.43(1H,s). | 466 (M+H)⁺ 464 (M-H)⁻ |
| 73 | 2.00-2.37(4H,m),2.69(3H,s),3.54-3.81(2H,m),3.98(2H,s),4,15(2H,s),5.39(1H,d, *J=*6.2Hz),7.34-7.40(2H,m). | 443 (M+H)⁺ 441 (M-H)⁻ |
| 74 | 1.98-2.32(5H,m),2.62(3H,s),2.76-2.90(2H,m),2.94-3.00(2H,m),3.57-3.80(3H,m) ,4.00-4.14(2H,m),5.07-5.34(1H,m),7.36-7.49(1H,m),8.96(1H,br.s). | 455 (M+H)⁺ 453 (M-H)⁻ |
| 75 | 2.06-2.33(4H,m),2.45-2.57(1H,s),2.69(3H,s),2.82(1H,t,*J=*5.5Hz),3.26-3.34(2H, m),3.36-3.73(2H,m),4.10-4.25(2H,m),5.35(1H,d,*J=*6.6Hz),7.37-7,45(2H,m). | 457 (M+H)⁺ 455 (M-H)⁻ |
| 76 | 1.98-2.31(3H,m),2.26-2.52(2H,m),2.71(3H,s),2.94-3.00(2H,m),2.84-2.99(6H,m) ,3.57-3.80(3H,m),4.23-4.50(2H,m),5.39(1H,d,*J=*7.0Hz),7.39-7.45(2H,m). | 485 (M+H)⁺ 483 (M-H)⁻ |
| 77 | 1.26-1.30(2H,m),1.35-1.40(1H,m),2.03-2.22(2H,m),2.62(3H,s),2.71(3H,s),3.02-3.09(2H,m),3.45-3.60(2H,m),3.64-3.75(2H,m),3.95-4.37(2H,m),5.41(1H,d,*J=*6.6Hz),7.41(1H,s). | 470 (M+H)⁺ 468 (M-H)⁻ |

### Example 78

### 5-[(2-{2-[4-(5-Fluorothiazol-2-ylcarbamoyl)-5-methylthiophe n-2-yl]pyrrolidin-1-yl}-2-oxoethyl)methylamino]pentanoic acid hydrochloride

5-[(2-{2-[4-(5-Fluorothiazol-2-ylcarbamoyl)-5-methylthioph en-2-yl]pyrrolidin-1-yl}-2-oxcethyl)methylamino]pentanoic acid methyl ester (63.0 mg) was dissolved in methanol (1.0 mL), a 1 N sodium hydroxide aqueous solution (500 µL) was added thereto, and stirring was conducted at room temperature for 5 hours. A 4 N hydrochloric acid/1,4-dioxane solution (500 µL) was added to the reaction liquid, and stirring was conducted at room temperature for 1 hours. The reaction liquid was concentrated under a reduced pressure, methylene chloride was added to the obtained residue, the insoluble substances were filtered off, and the filtrate was concentrated under a reduced pressure to give the title compound (57.6 mg) as a pale yellow white powder.
¹H-NMR(DMSO-d6)δ:1.20-1.22(2H,m), 1.49-1.69(4H,m), 1.91-2.36( 6H,m), 2.63-2.83(6H,m), 3.02-3.18(1H,m), 3.56-3.70(1H,m), 4.17-4.36(2H,m), 5.28(1H,d,*J=*6.6Hz), 7.37(1H,br.s), 7.54(1H,br.s), 9 .62(1H,br.s), 12.21(1H,br.s).
ESI/MS(m/z): 483(M+H)⁺, 481(M-H)⁻.

### Example 79

### 5-[(2-{2-[4-(5-Chlorothiazol-2-ylcarbamoyl)-5-methylthiophe n-2-yl]pyrrolidin-1-yl}-2-oxoethyl)methylamino]pentanoic acid hydrochloride

The title compound was obtained by conducting a reaction in a similar methods to that of Example 52 by using 5-[(2-{2-[4-(5-chlorothiazol-2-ylcarbamoyl)-5-methylthiophe n-2-yl]pyrrolidin-1-yl}-2-oxoethyl)methylamino]pentanoic acid methyl ester instead of 5-[(2-{2-[4-(5-fluorothiazol-2-ylcarbamoyl)-5-methylthiophe n-2-yl]pyrrolidin-1-yl}-2-oxoethyl)methylamino]pentanoic acid methyl ester.
¹H-NMR(CD₃OD)δ(ppm): 1.60-1.81(4H,m), 2.02-2.45(6H,m), 2.70(3H ,d,*J=*2.9Hz), 2.78(3H,d,*J=*2.9Hz), 3.11-3.25(1H,m), 3.58-3.82(2H ,m), 4.20-4.35(1H,m), 5.39(1H,d,*J=*7.0Hz), 7.40-7.48(2H,m).
ESI/MS(m/z): 499(M+H)⁺, 497(M-H)⁻ .

### Example 80

### [(2-[(2S,4R)-4-Fluoro-2-[4-(5-fluorothiazol-2-ylcarbamoyl)-5-methylthiophen-2-yl]pyrrolidin-1-yl]-2-oxoethyl)pentylami no]acetic acid

While (2-[(2S,4R)-4-Fluoro-2-[4-(5-fluorothiazol-2-ylcarbamoyl)-5 -methylthiophen-2-yl]pyrrolidin-1-yl]-2-oxoethyl)pentylamin o]acetic acid t-butyl ester (44.6 mg) was stirred under ice-cooling, a 4 N hydrochloric acid/ethyl acetate solution (391 µL) was added, and stirring was conducted overnight at room temperature. Furthermore, a 4 N hydrochloric acid/ethyl acetate solution (391 µL) was added thereto, and stirring was conducted at room temperature for 27 hours. Diethyl ether (10 mL) was added to the reaction liquid, stirring was conducted for 30 minutes, and the precipitated crystal was collected by filtration, washed with diethyl ether and dried under a reduced pressure. Water and a 1 N aqueous sodium hydroxide solution were added to the solid to adjust the pH 10, and extraction was conducted by using ethyl acetate. 1 N hydrochloric acid was added to the aqueous layer to adjust the pH 5, and concentration was conducted under a reduced pressure. Chloroform was added to the residue, and the residue was dried over anhydrous sodium sulfate to give the title compound (9.2 mg) as a white powder.
¹H-NMR(CD₃OD)δ(ppm): 0.85-0.89(3H,m), 1.22-1.30(5H,m), 1.49-1. 62(2H,m), 2.28-2.38(1H,m), 2.66-2.79(4H,m), 2.97-3.12(2H,m), 3. 48-3.66(2H,m), 3.82-4.27(3H,m), 5.26-5.52(2H,m), 7.12(1H,br.s) , 7.41(1H,d,*J=*16.8Hz).
ESI/MS(m/z): 515(M+H)⁺, 513(M-H)⁻.

### Example 81

### [(2-[2-[4-(5-Fluorothiazol-2-ylcarbamoyl)-5-methylthiophen-2-yl]pyrrolidin-1-yl]-2-oxoethyl)pentylamino]acetzc acid

The title compound was obtained by conducting a reaction in a similar method to that of Example 80 by using [(2-[2-[4-(5-fluorothiazol-2-ylcarbamoyl)-5-methylthiophen-2-yl]pyrrolidin-1-yl]-2-oxoethyl)pentylamino]acetic acid t-butyl ester instead of (2-[(2S,4R)-4-fluoro-2-[4-(5-fluorothiazol-2-ylcarbamoyl)-5 -methylthiophen-2-yl]pyrrolidin-1-yl]-2-oxoethyl)pentylamin o]acetic acid t-butyl ester.
¹H-NMR(CD₃OD)δ(ppm): 0.86-0.90(3H,m), 1.29-1.33(4H,m), 1.52-1. 70(2H,m), 2.02-2.44(4H,m), 2.69(3H,br.d,*J=*16.8Hz), 3.04-3.18(2 H,m), 3.57-3.78(4H,m), 4.15-4.21(2H,m), 5.37(1H,d,*J=*7.7Hz), 7.1 2(1H,br.s), 7.31(1H,br.d,*J=*7.3Hz).
ESI/MS(m/z): 497(M+H)⁺, 495(M-H)⁻.

### Pharmacological Test Example 1

### (1) Expression and purification of human glucokinase

To the EcoRI, HindIII site of a pQE-80 expression vector were introduced a DNA fragment in which the same restriction enzyme site had been added to the N-terminal deficiency (1-15 amino acids deficiency)-encoding region of a human GK. E. coli was transformed by using this plasmid to express a human glucokinase, and purification was conducted as follows.
A fungus body that had been collected from 200 ml of an E.coli culture solution was collected and suspended in 20 ml of Extraction buffer A (20 mM HEPES, pH 8.0, 1 mM MgCl₂, 150 mM NaCl, 2 mM mercaptoethanol, 0.25 mg/ml lysozyme, 50 mg/ml sodium azide), and the suspension was stood still at room temperature for 5 minutes. 20 ml of Extraction buffer B (1.5 M NaCl, 100 mM CaCl₂, 100 mM MgC1₂, 0.02 mg/ml DNA catabolic enzyme 1, a protease inhibitor tablet (Complete (registered trademark) 1697498): one tablet per 20 ml of the buffer) was added therero, followed by standing still at room temperature for 5 minutes. The extract liquid was then centrifuged by 15,000 g for 30 minutes at 4°C, and the supernatant was used as an E. coli extract liquid. The E. coli extract liquid was filtered by a 0.45 µm filter and applied to an Ni-NTA agarose 2 mL bed that had been equilibrated with a buffer (20 mM HEPES pH 8.0, 0.5 M NaCl) comprising 20 mM of imidazole. The bed was washed with about 10 ml of a washing buffer and eluted stepwise with buffers (20 mM HEPES pH 8.0, 0.5 M NaCl) each comprising imidazole by from 40 to 500 mM. Each of the column fractions was analyzed by using SDS gel electrophoresis, and the fraction comprising hGK (MW: 52 KDa) was concentrated. The concentrated sample was then passed through a Sephacryl S-200HR (11/60) gel filtration column and eluted with buffer B (20 mM HEPES, pH 8.0, 1 mM MgCl₂, 150 mM NaCl, 1 mM DTT). The eluted fraction was analyzed by SDS gel electrophoresis, and the fraction comprising hGK was concentrated. Finally, 50% glycerol was added to the fraction, and the fraction was stored at -20°C.

### (2) Measurement of glucokinase-activity

178 µl of GK assay mix (25 mM Hepes buffer (pH 7.1), 25 mM KCl, 2 mM MgCl₂, 1 mM NADP, 1 mM dithiotheitol, 2 unit/mL G6PDH, 5 mM D-glucose, a suitable amount of GK) was added to a UV permeable 96-well plate. 2 µl of the test compound dissolved in DMSO was added thereto, followed by standing still at room temperature for 10 minutes, and 20 µl of 20 mM ATP was then added thereto to start a reaction. An absorbance at 340 nm was measured by Spectra Max Plus for 5 minutes at intervals of 30 seconds at room temperature, and the compound was evaluated in the reaction at the first 3 minutes. The activity of the test compound at the final concentration of 10 µM was calculated in comparison with a well that did not comprise the compound, and was shown in Table 29. As a result, it was found that the compound of the present invention that was subjected to the present test had 600% or more of a human GK activation effect at 10 µM as compared to the well that did not comprise the test compound.

**[Table 29]**

| Example | GK activation effect (%) | Example | GK activation effect (%) | Example | GK activation effect (%) |
|---|---|---|---|---|---|
| 10 | 600 | 35 | 767 | 65 | 851 |
| 12 | 630 | 38 | 801 | 66 | 910 |
| 13 | 603 | 40 | 604 | 67 | 860 |
| 17 | 621 | 42 | 701 | 68 | 813 |
| 22 | 613 | 47 | 673 | 69 | 726 |
| 23 | 641 | 52 | 701 | 71 | 728 |
| 24 | 873 | 55 | 721 | 76 | 717 |
| 25 | 937 | 56 | 622 | 77 | 789 |
| 26 | 947 | 59 | 670 | 78 | 695 |
| 27 | 882 | 62 | 789 | 79 | 717 |
| 28 | 710 | 63 | 722 | 80 | 752 |
| 29 | 685 | 64 | 765 | 81 | 787 |
| 30 | 766 | | | | |

### Industrial Applicability

The compound of the present invention has a glucokinase-activating effect, and thus is useful as an agent for the prevention or treatment of diabetes or diabetic retinopathy, diabetic nephropathy, diabetic neuropathy, ischemic heart diseases, or chronic complications of diabetes such as arteriosclerosis.

## Claims

1. A compound represented by the following general formula (I) or a pharmaceutically acceptable salt thereof: 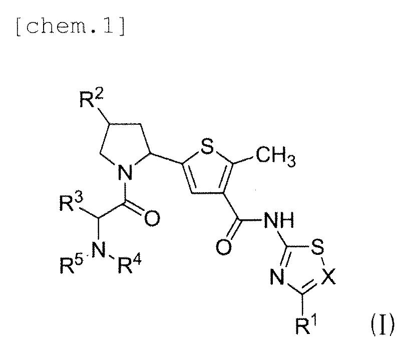 wherein
X means a nitrogen atom or CR⁶, wherein R⁶ means a hydrogen atom or a halogen atom;
R¹ means a hydrogen atom, a C1-C6 alkyl group, a C1-C6 alkoxy group, or a C1-C6 alkylthio group;
R² means a hydrogen atom or a fluorine atom;
R³ means a hydrogen atom or a C1-C6 alkyl group; and
one of R⁴ and R⁵ means a hydrogen atom or a C1-C6 alkyl group, and the other means a C1-C6 alkylenecarboxylic acid, a C1-C6 alkylsulfonyl group, a C1-C6 alkylcarbonyl group, or CONH₂.

2. The compound according to Claim 1, wherein, in the above-mentioned general formula (I), X is a nitrogen atom, C-F, or C-C1 and R¹ is a hydrogen atom, a C1-C3 alkyl group, a C1-C3 alkoxy group, or a C1-C3 alkylthio group.

3. The compound according to Claim 2, wherein X is C-F or C-Cl, and R¹ is a hydrogen atom.

4. The compound according to Claim 2, wherein X is a nitrogen atom, and R¹ is a hydrogen atom or a C1-C3 alkyl group.

5. The compound according to Claim 1, wherein, in the above-mentioned general formula (I), R³ is a hydrogen atom or a C1-C3 alkyl group, and one of R⁴ and R⁵ is a hydrogen atom or a C1-C6 alkyl group, and the other is a C1-C3 alkylenecarboxylic acid, a C1-C3 alkylsulfonyl group, a C1-C3 alkylcarbonyl group, or CONH₂.

6. The compound according to Claim 5, wherein one of R⁴ and R⁵ is a hydrogen atom or a C1-C3 alkyl group, and the other is a C1-C3 alkylenecarboxylic acid or a C1-C3 alkylsulfonyl group.

7. The compound according to Claim 1, wherein, in the above-mentioned general formula (I), X is C-F, R¹ is a hydrogen atom, R³ is a hydrogen atom or a C1-C3 alkyl group, and one of R⁴ and R⁵ is a hydrogen atom or a C1-C6 alkyl group, and the other is a C1-C3 alkylenecarboxylic acid.

8. The compound according to Claim 1, wherein, in the above-mentioned general formula (I), X is a nitrogen atom or C-F, R¹ is a hydrogen atom or a C1-C3 alkyl group, R³ is a hydrogen atom or a C1-C3 alkyl group, and one of R⁴ and R⁵ is a hydrogen atom, and the other is a C1-C3 alkylsulfonyl group.

9. A compound represented by the following general formula (II): 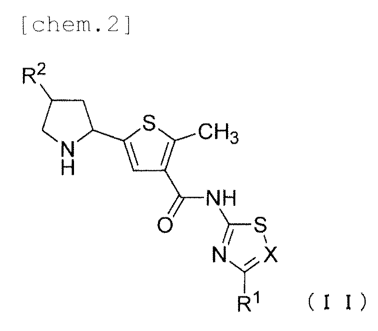 wherein
X means a nitrogen atom or CR⁶, wherein R⁶ is a hydrogen atom or a halogen atom;
R¹ means a hydrogen atom, a C1-C6 alkyl group, a C1-C6 alkoxy group, or a C1-C6 alkylthio group; and
means a hydrogen atom or a fluorine atom.

10. A compound represented by the following general formula (III): 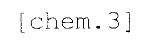 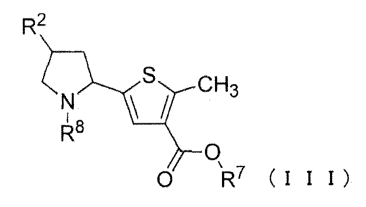 wherein
means a hydrogen atom or a fluorine atom;
R⁷ means a hydrogen atom, or a protective group for a carboxyl group; and
R⁸ means a hydrogen atom, or a protective group for an amino group.

11. A compound represented by the following general formula (IV): 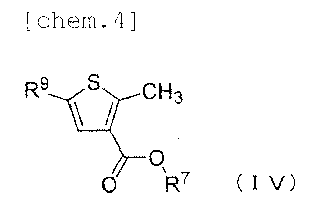 wherein
R⁷ means a hydrogen atom, or a protective group for a carboxyl group; and
R⁹ means a bromine atom or an iodine atom.

12. A pharmaceutical composition comprising a compound represented by the following general formula (I) or a pharmaceutically acceptable salt thereof as an active ingredient: 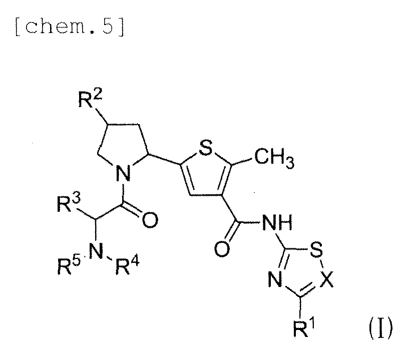 wherein
X means a nitrogen atom or CR⁶, wherein R⁶ means a hydrogen atom or a halogen atom;
means a hydrogen atom, a C1-C6 alkyl group, a C1-C6 alkoxy group, or a C1-C6 alkylthio group;
R² means a hydrogen atom or a fluorine atom;
R³ means a hydrogen atom or a C1-C6 alkyl group; and one of R⁴ and R⁵ means a hydrogen atom or a C1-C6 alkyl group, and the other means a C1-C6 alkylenecarboxylic acid, a C1-C6 alkylsulfonyl group, a C1-C6 alkylcarbonyl group, or CONH₂.

13. The pharmaceutical composition according to Claim 12,
wherein, in the above-mentioned general formula (I) of the compound as an active ingredient, X is C-F, R¹ is a hydrogen atom, R³ is a hydrogen atom or a C1-C3 alkyl group, and one of R⁴ and R⁵ is a hydrogen atom or a C1-C6 alkyl group and the other is a C1-C3 alkylenecarboxylic acid.

14. The pharmaceutical composition according to Claim 12, wherein, in the above-mentioned general formula (I) of the compound as an active ingredient, X is a nitrogen atom or C-F, R¹ is a hydrogen atom or a C1-C3 alkyl group, R³ is a hydrogen atom or a C1-C3 alkyl group, and one of R⁴ and R⁵ is a hydrogen atom and the other is a C1-C3 alkylsulfonyl group.

15. The pharmaceutical composition according to Claim 12, which is for the prevention or treatment of diabetes.
